# EUROPEAN PATENT SPECIFICATION

(11) **EP 1 718 624 B1**
(45) Date of publication and mention of the grant of the patent: **25.03.2009**
(21) Application number: 05708360.2
(22) Date of filing: 15.02.2005
(51) Int. Cl.: C07D 277/46, C07D 417/12, C07D 231/40, C07D 403/12, C07D 487/08, A61K 31/415, A61K 31/426, A61K 31/427, A61P 3/10

(54) **BENZAMIDE DERIVATIVES AND THEIR USE AS GLUCOKINASE ACTIVATING AGENTS**
BENZAMIDDERIVATE UND DEREN VERWENDUNG ALS GLUCOKINASEAKTIVIERENDE MITTEL
DERIVES DE BENZAMIDE ET LEUR UTILISATION EN TANT QU'ACTIVATEURS DE LA GLUCOKINASE

(30) Priority: 18.02.2004 GB 0403593; 16.06.2004 GB 0413386; 16.10.2004 GB 0423039
(43) Date of publication of application: 08.11.2006
(60) Divisional application: 08158356.9
(73) Proprietor: AstraZeneca AB, 151 85 Södertälje (SE)
(72) Inventor: JOHNSTONE, Craig, AstraZeneca R & D Alderley, Cheshire SK10 4TG (GB); MCKERRECHER, Darren, AstraZeneca R & D Alderley, Cheshire SK10 4TG (GB); PIKE, Kurt, Gordon, AstraZeneca R & D Alderley, Cheshire SK10 4TG (GB)
(86) International application number: PCT/GB2005/000545
(87) International publication number: WO 2005/080359

(56) References cited:
- WO-A-03/015774
- WO-A-20/03000267
- WO-A-20/04076420

## Description

The present invention relates to a group of benzoyl amino heterocyclyl compounds which are useful in the treatment or prevention of a disease or medical condition mediated through glucokinase (GLK or GK), leading to a decreased glucose threshold for insulin secretion. In addition the compounds are predicted to lower blood glucose by increasing hepatic glucose uptake. Such compounds may have utility in the treatment of Type 2 diabetes and obesity. The invention also relates to pharmaceutical compositions comprising said compounds and to methods of treatment of diseases mediated by GLK using said compounds.

In the pancreatic β-cell and liver parenchymal cells the main plasma membrane glucose transporter is GLUT2. Under physiological glucose concentrations the rate at which GLUT2 transports glucose across the membrane is not rate limiting to the overall rate of glucose uptake in these cells. The rate of glucose uptake is limited by the rate of phosphorylation of glucose to glucose-6-phosphate (G-6-P) which is catalysed by glucokinase (GLK) [1]. GLK has a high (6-10mM) Km for glucose and is not inhibited by physiological concentrations of G-6-P [1]. GLK expression is limited to a few tissues and cell types, most notably pancreatic β-cells and liver cells (hepatocytes) [1]. In these cells GLK activity is rate limiting for glucose utilisation and therefore regulates the extent of glucose induced insulin secretion and hepatic glycogen synthesis. These processes are critical in the maintenance of whole body glucose homeostasis and both are dysfunctional in diabetes [2].

In one sub-type of diabetes, Maturity-Onset Diabetes of the Young Type 2 (MODY-2), the diabetes is caused by GLK loss of function mutations [3, 4]. Hyperglycaemia in MODY-2 patients results from defective glucose utilisation in both the pancreas and liver [5]. Defective glucose utilisation in the pancreas of MODY-2 patients results in a raised threshold for glucose stimulated insulin secretion. Conversely, rare activating mutations of GLK reduce this threshold resulting in familial hyperinsulinism [6, 6a, 7]. In addition to the reduced GLK activity observed in MODY-2 diabetics, hepatic glucokinase activity is also decreased in Type 2 diabetics [8]. Importantly, global or liver selective overexpression of GLK prevents or reverses the development of the diabetic phenotype in both dietary and genetic models of the disease [9-12]. Moreover, acute treatment of Type 2 diabetics with fructose improves glucose tolerance through stimulation of hepatic glucose utilisation [13]. This effect is believed to be mediated through a fructose induced increase in cytosolic GLK activity in the hepatocyte by the mechanism described below [13].

Hepatic GLK activity is inhibited through association with GLK regulatory protein (GLKRP). The GLK/GLKRP complex is stabilised by fructose-6-phosphate (F6P) binding to the GLKRP and destabilised by displacement of this sugar phosphate by fructose-1-phosphate (F1P). F1P is generated by fructokinase mediated phosphorylation of dietary fructose. Consequently, GLK/GLKRP complex integrity and hepatic GLK activity is regulated in a nutritionally dependent manner as F6P is dominant in the post-absorptive state whereas F1P predominates in the post-prandial state. In contrast to the hepatocyte, the pancreatic β-cell expresses GLK in the absence of GLKRP. Therefore, β-cell GLK activity is regulated extensively by the availability of its substrate, glucose. Small molecules may activate GLK either directly or through destabilising the GLK/GLKRP complex. The former class of compounds are predicted to stimulate glucose utilisation in both the liver and the pancreas whereas the latter are predicted to act exclusively in the liver. However, compounds with either profile are predicted to be of therapeutic benefit in treating Type 2 diabetes as this disease is characterised by defective glucose utilisation in both tissues.

GLK, GLKRP and the K_{ATP} channel are expressed in neurones of the hypothalamus, a region of the brain that is important in the regulation of energy balance and the control of food intake [14-18]. These neurones have been shown to express orectic and anorectic neuropeptides [15, 19, 20] and have been assumed to be the glucose-sensing neurones within the hypothalamus that are either inhibited or excited by changes in ambient glucose concentrations [17, 19, 21, 22]. The ability of these neurones to sense changes in glucose levels is defective in a variety of genetic and experimentally induced models of obesity [23-28]. Intracerebroventricular (icv) infusion of glucose analogues, that are competitive inhibitors of glucokinase, stimulate food intake in lean rats [29, 30]. In contrast, icv infusion of glucose suppresses feeding [31]. Thus, small molecule activators of GLK may decrease food intake and weight gain through central effects on GLK. Therefore, GLK activators may be of therapeutic use in treating eating disorders, including obesity, in addition to diabetes. The hypothalamic effects will be additive or synergistic to the effects of the same compounds acting in the liver and/or pancreas in normalising glucose homeostasis, for the treatment of Type 2 diabetes. Thus the GLK/GLKRP system can be described as a potential "Diabesity" target (of benefit in both Diabetes and Obesity).

GLK is also expressed in specific entero-endocrine cells where it is believed to control the glucose sensitive secretion of the incretin peptides GIP (glucose-dependent insulinotropic polypeptide) and GLP-1 (Glucagon-Like Peptide-1) from gut K-cells and L-cells respectively (32, 33, 34). Therefore, small molecule activators of GLK may have additional beneficial effects on insulin secretion, β-cell function and survival and body weight as a consequence of stimulating GIP and GLP-1 secretion from these entero-endocrine cells.

In WO00/58293 and WO01/44216 (Roche), a series of benzylcarbamoyl compounds are described as glucokinase activators. The mechanism by which such compounds activate GLK is assessed by measuring the direct effect of such compounds in an assay in which GLK activity is linked to NADH production, which in turn is measured optically - see details of the *in vitro* assay described hereinafter. Compounds of the present invention may activate GLK directly or may activate GLK by inhibiting the interaction of GLKRP with GLK.

Further GLK activators have been described in WO03/095438 (substituted phenylacetamides, Roche), WO03/055482 (carboxamide and sulphonamide derivatives, Novo Nordisk), W02004/002481 (arylcarbonyl derivatives, Novo Nordisk), and in WO03/080585 (amino-substituted benzoylaminoheterocycles, Banyu).

Our International application Number: WO03/000267 describes a group of benzoyl amino pyridyl carboxylic acids which are activators of the enzyme glucokinase (GLK).

Our International application Number: WO03/015774 describes compounds of the Formula (A): 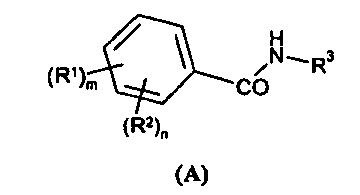 wherein R³ is a substituted heterocycle other than a carboxylic acid substituted pyridyl.

International application WO2004/076420 (Banyu) describes compounds which are generally a subset of those described in WO03/015774, wherein for example R¹ is an (substituted) alkyl ether and R² is (substituted) phenoxy. Compounds disclosed by WO2004/076420 have been disclaimed from the score of the present invention.

We have surprisingly found a small group of compounds, generally a selected subgroup of those described in WO 03/015774, which have generally superior potency for the GLK enzyme, and more advantageous physical properties, including, for example, one or more of higher aqueous solubility, higher permeability, and/or lower plasma protein binding. Consequently, such compounds having a balance of these properties would be expected to display higher plasma free drug levels and superior in vivo efficacy after oral dosing as determined, for example, by activity in Oral Glucose Tolerance Tests (OGTTs). Therefore this group of compounds would be expected to provide superior oral exposure at a lower dose and thereby be particularly suitable for use in the treatment or prevention of a disease or medical condition mediated through GLK.

Thus, according to the first aspect of the invention there is provided a compound of Formula (I), or a salt or solvate thereof: 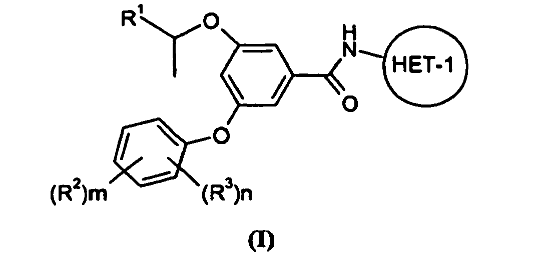 wherein:
R¹ is methoxymethyl;
R² is selected from -C(O)NR⁴R⁵, -SO₂NR⁴R⁵, -S(O)ₚR⁴ and HET-2;
HET-1 is a 5- or 6-membered, C-linked heteroaryl ring containing a nitrogen atom in the 2-position and optionally 1 or 2 further ring heteroatoms independently selected from O, N and
S; which ring is optionally substituted on an available carbon atom, or on a ring nitrogen atom provided it is not thereby quaternised, with 1 or 2 substituents independently selected from R⁶;
HET-2 is a 4-, 5- or 6-membered, C- or N-linked heterocyclyl ring containing 1, 2, 3 or 4 heteroatoms independently selected from O, N and S, wherein a -CH₂- group can optionally be replaced by a -C(O)- , and wherein a sulphur atom in the heterocyclic ring may optionally be oxidised to a S(O) or S(O)₂ group, which ring is optionally substituted on an available carbon or nitrogen atom by 1 or 2 substituents independently selected from R⁷;
R³ is selected from halo, fluoromethyl, difluoromethyl, trifluoromethyl, methyl, methoxy and cyano;
R⁴ is selected from hydrogen, (1-4C)alkyl [optionally substituted by 1 or 2 substituents independently selected from HET-2, -OR⁵, -SO₂R⁵, (3-6C)cycloalkyl (optionally substituted with 1 group selected from R⁷) and -C(O)NR⁵R⁵], (3-6C)cycloalkyl (optionally substituted with I group selected from R⁷) and HET-2;
R⁵ is hydrogen or (1-4C)alkyl;
   or R⁴ and R⁵ together with the nitrogen atom to which they are attached may form a heterocyclyl ring system as defined by HET-3;
R⁶ is independently selected from (1-4C)alkyl, halo, hydroxy(1-4C)alkyl, (1-4C)alkoxy(1-4C)alkyl, (1-4C)alkylS(O)p(1-4C)alkyl, amino(1-4C)alkyl, (1-4C)alkylamino(1-4C)alkyl, di(1-4C)alkylamino(1-4C)alkyl and HET-4;
R⁷ is selected from -OR⁵, (1-4C)alkyl, -C(O)(1-4C)alkyl, -C(O)NR⁴R⁵, (1-4C)alkoxy(1-4C)alkyl, hydroxy(1-4C)alkyl and -S(O)pR⁵;
HET-3 is an N-linked, 4, 5 or 6 membered, saturated or partially unsaturated heterocyclyl ring, optionally containing 1 or 2 further heteroatoms (in addition to the linking N atom) independently selected from O, N and S, wherein a -CH₂- group can optionally be replaced by a -C(O)- and wherein a sulphur atom in the ring may optionally be oxidised to a S(O) or S(O)₂ group; which ring is optionally substituted on an available carbon or nitrogen atom by 1 or 2 substituents independently selected from R⁸; or
HET-3 is an N-linked, 7 membered, saturated or partially unsaturated heterocyclyl ring, optionally containing 1 further heteroatom (in addition to the linking N atom) independently selected from O, S and N, wherein a -CH₂- group can optionally be replaced by a -C(O)-group and wherein a sulphur atom in the ring may optionally be oxidised to a S(O) or S(O)₂ group; which ring is optionally substituted on an available carbon or nitrogen atom by 1 or 2 substituents independently selected from R⁸; or
HET-3 is an 6-10 membered bicyclic saturated or partially unsaturated heterocyclyl ring, optionally containing 1 further nitrogen atom (in addition to the linking N atom) wherein a -CH₂- group can optionally be replaced by a -C(O)-; which ring is optionally substituted on an available carbon or nitrogen atom by 1 substituent selected from hydroxy and R³;
R⁸ is selected from -OR⁵, (1-4C)alkyl, -C(O)(1-4C)alkyl, -C(O)NR⁴R⁵, (1-4C)alkylamino, di(1-4C)alkylamino, HET-3 (wherein said ring is unsubstituted), (1-4C)alkoxy(1-4C)alkyl, hydroxy( 1-4C)alkyl and -S(O)pR⁵;
HET-4 is a 5- or 6-membered, C-or N- linked unsubstituted heteroaryl ring containing 1, 2 or 3 ring heteroatoms independently selected from O, N and S;
p is (independently at each occurrence) 0, 1 or 2;
m is 0 or 1;
n is 0, 1 or 2;
provided that when m is 0, then n is 1 or 2;
and that the compound of Formula (I) is other than:
3-(4-methanesulfonylphenoxy)-5-(2-methoxy-1-methyl-ethoxy)-N-thiazol-2-yl-benzamide: N-(4-hydroxymethyl-thiazol-2-yl)-3-(4-methanesulfonylphenoxy)-5-(2-methoxy-1-methylethoxy)-benzamide;
N-[4-(1-hydroxy-ethyl)-thiazol-2-yl]-3-(4-methanesulfonylphenoxy)-5-(2-methoxy-1-methylethoxy)-benzamide;
N-[3-hydroxymethyl-1,2,4-thiadiazol-5-yl]-3-(4-methanesulfonyphenoxy)-5-2-methoxy-1-methyl-ethoxy)benzamide, or
3-(4-methanesulfonylphenoxy)-5-(2-methoxy-1-methyl-ethoxy)-(pyrazol-3-yl)benzamide.

In another aspect of the invention, there is provided a compound of the formula (I) as hereinbefore defined, wherein
R¹ is methoxymethyl;
R² is selected from -C(O)-HET-3 and -SO₂-HET-3;
HET-1 is a 5- or 6-membered, C-linked heteroaryl ring containing a nitrogen atom in the 2-position and optionally 1 or 2 further ring heteroatoms independently selected from O, N and S; which ring is optionally substituted on an available carbon atom, or on a ring nitrogen atom provided it is not thereby quaternised, with 1 or 2 substituents independently selected from R⁶;
HET-2 is a 4-, 5- or 6-membered, C- or N-linked heterocyclyl ring containing 1, 2, 3 or 4 heteroatoms independently selected from O, N and S, wherein a -CH₂- group can optionally be replaced by a -C(O)- , and wherein a sulphur atom in the heterocyclic ring may optionally be oxidised to a S(O) or S(O)₂ group, which ring is optionally substituted on an available carbon or nitrogen atom by 1 or 2 substituents independently selected from R⁷;
R³ is selected from halo, fluoromethyl, difluoromethyl, trifluoromethyl, methyl, methoxy and cyano;
R⁴ is selected from hydrogen, (1-4C)alkyl [optionally substituted by I or 2 substituents independently selected from HET-2, -OR⁵, -SO₂R⁵, (3-6C)cycloalkyl (optionally substituted with 1 group selected from R⁷) and -C(O)NR⁵R⁵], (3-6C)cycloalkyl (optionally substituted with 1 group selected from R⁷) and HET-2;
R⁵ is hydrogen or (1-4C)alkyl; or
R⁴ and R⁵ together with the nitrogen atom to which they are attached may form a heterocyclyl ring system as defined by HET-3;
R⁶ is independently selected from (1-4C)alkyl, halo, hydroxy(1-4C)alkyl, (1-4C)alkoxy(1-4C)alkyl, (1-4C)alkylS(O)p(1-4C)alkyl, amino(1-4C)alkyl, (1-4C)alkylamino(1-4C)alkyl, di(1-4C)alkylamino(1-4C)alkyl and HET-4;
R⁷ is selected from -OR⁵, (1-4C)alkyl, -C(O)(1-4C)alkyl, -C(O)NR⁴R⁵, (1-4C)alkoxy(1-4C)alkyl, hydroxy(1-4C)alkyl and -S(O)pR⁵;
HET-3 is an N-linked, 4, 5 or 6 membered, saturated or partially unsaturated heterocyclyl ring, optionally containing 1 or 2 further heteroatoms (in addition to the linking N atom) independently selected from O, N and S, wherein a -CH₂- group can optionally be replaced by a -C(O)- and wherein a sulphur atom in the ring may optionally be oxidised to a S(O) or S(O)₂ group; which ring is optionally substituted on an available carbon or nitrogen atom by 1 or 2 substituents independently selected from R⁸; or
HET-3 is an N-linked, 7 membered, saturated or partially unsaturated heterocyclyl ring, optionally containing 1 further heteroatom (in addition to the linking N atom) independently selected from O, S and N, wherein a -CH₂- group can optionally be replaced by a -C(O)-group and wherein a sulphur atom in the ring may optionally be oxidised to a S(O) or S(O)₂ group; which ring is optionally substituted on an available carbon or nitrogen atom by 1 or 2 substituents independently selected from R⁸; or
HET-3 is an 6-10 membered bicyclic saturated or partially unsaturated heterocyclyl ring, optionally containing 1 further nitrogen atom (in addition to the linking N atom) wherein a -CH₂- group can optionally be replaced by a -C(O)-; which ring is optionally substituted on an available carbon or nitrogen atom by 1 substituent selected from hydroxy and R³;
R⁸ is selected from -OR⁵, (1-4C)alkyl, -C(O)(1-4C)alkyl, -C(O)NR⁴R⁵, (1-4C)alkylamino, di(1-4C)alkylamino, HET-3 (wherein said ring is unsubstituted), (1-4C)alkoxy(1-4C)alkyl, hydroxy(1-4C)alkyl and -S(O)pR⁵;
HET-4 is a 5- or 6-membered, C-or N- linked unsubstituted heteroaryl ring containing 1, 2 or 3 ring heteroatoms independently selected from O, N and S;
p is (independently at each occurrence) 0, 1 or 2;
m is 0 or 1;
n is 0, 1 or 2;
provided that when m is 0, then n is 1 or 2;
or a salt or solvate thereof;

In a further aspect of the invention there is provided a compound of the formula (I), as hereinbefore defined or a salt or solvate thereof, wherein:
HET-3 is an N-linked, 4 to 6 membered, saturated or partially unsaturated heterocyclyl ring, optionally containing 1 or 2 further heteroatoms (in addition to the linking N atom) independently selected from O, N and S, wherein a -CH₂- group can optionally be replaced by a -C(O)- and wherein a sulphur atom in the ring may optionally be oxidised to a S(O) or S(O)₂ group; which ring is optionally substituted on an available carbon or nitrogen atom by 1 or 2 substituents independently selected from R⁸.

In another aspect of the invention, there is provided a compounds of the formula (I) as hereinbefore defined, wherein
R¹ is methoxymethyl;
R² is selected from -(O)NR⁴¹R⁵¹, -SO₂NR⁴¹R⁵¹ and -S(O)ₚR⁴¹;
HET-1 is a 5- or 6-membered, C-linked heteroaryl ring containing a nitrogen atom in the 2-position and optionally 1 or 2 further ring heteroatoms independently selected from O, N and S; which ring is optionally substituted on an available carbon atom, or on a ring nitrogen atom provided it is not thereby quaternised, with 1 or 2 substituents independently selected from R⁶;
HET-2 is a 4-, 5- or 6-membered, C- or N-linked heterocyclyl ring containing 1, 2, 3 or 4 heteroatoms independently selected from O, N and S, wherein a -CH₂- group can optionally be replaced by a -C(O)- , and wherein a sulphur atom in the heterocyclic ring may optionally be oxidised to a S(O) or S(O)₂ group, which ring is optionally substituted on an available carbon or nitrogen atom by 1 or 2 substituents independently selected from R⁷;
R³ is selected from halo, fluoromethyl, difluoromethyl, trifluoromethyl, methyl, methoxy and cyano;
R⁴¹ is selected from (1-4C)alkyl [substituted by 1 or 2 substituents independently selected from HET-2, -OR⁵, -SO₂R⁵, (3-6C)cycloalkyl (optionally substituted with 1 group selected from R⁷) and -C(O)NR⁵R⁵], (3-6C)cycloalkyl (optionally substituted with I group selected from R⁷) and HET-2;
R⁵¹ is hydrogen or (1-4C)alkyl;
R⁴ is selected from (1-4C)alkyl [optionally substituted by 1 or 2 substituents independently selected from HET-2, -OR⁵, -SO₂R⁵, (3-6C)cycloalkyl (optionally substituted with 1 group selected from R⁷) and -C(O)NR⁵R⁵], (3-6C)cycloalkyl (optionally substituted with 1 group selected from R⁷) and HET-2;
R⁵ is hydrogen or (1-4C)alkyl;
   or R⁴ and R⁵ together with the nitrogen atom to which they are attached may form a heterocyclyl ring system as defined by HET-3;
R⁶ is independently selected from (1-4C)alkyl, halo, hydroxy(1-4C)alkyl, (1-4C)alkoxy(1-4C)alkyl, (1-4C)alkyls(O)p(1-4C)alkyl, amino(1-4C)alkyl, (1-4C)alkylamino(1-4C)alkyl, di(1-4C)alkylamino(1-4C)alkyl and HET-4;
R⁷ is selected from -OR⁵, (1-4C)alkyl, -C(O)(1-4C)alkyl, -C(O)NR⁴R⁵, (1-4C)alkoxy(1-4C)alkyl, hydroxy(1-4C)alkyl and-S(O)pR⁵;
HET-3 is an N-linked, 4, 5 or 6 membered, saturated or partially unsaturated heterocyclyl ring, optionally containing 1 or 2 further heteroatoms (in addition to the linking N atom) independently selected from O, N and S, wherein a -CH₂- group can optionally be replaced by a -C(O)- and wherein a sulphur atom in the ring may optionally be oxidised to a S(O) or S(O)₂ group; which ring is optionally substituted on an available carbon or nitrogen atom by 1 or 2 substituents independently selected from R⁸; or
HET-3 is an N-linked, 7 membered, saturated or partially unsaturated heterocyclyl ring, optionally containing 1 further heteroatom (in addition to the linking N atom) independently selected from O, S and N, wherein a -CH₂- group can optionally be replaced by a -C(O)-group and wherein a sulphur atom in the ring may optionally be oxidised to a S(O) or S(O)₂ group; which ring is optionally substituted on an available carbon or nitrogen atom by 1 or 2 substituents independently selected from R⁸; or
HET-3 is an 6-10 membered bicyclic saturated or partially unsaturated heterocyclyl ring, optionally containing 1 further nitrogen atom (in addition to the linking N atom) wherein a -CH₂- group can optionally be replaced by a -C(O)-; which ring is optionally substituted on an available carbon or nitrogen atom by 1 substituent selected from hydroxy and R³;
R⁸ is selected from -OR⁵, (1-4C)alkyl, -C(O)(1-4C)alkyl, -C(O)NR⁴R⁵, (1-4C)alkylamino, di(1-4C)alkylamino, HET-3 (wherein said ring is unsubstituted), (1-4C)alkoxy(1-4C)alkyl, hydroxy(1-4C)alkyl and -S(O)pR⁵;
HET-4 is a 5- or 6-membered, C-or N- linked unsubstituted heteroaryl ring containing 1, 2 or
3 ring heteroatoms independently selected from O, N and S;
p is (independently at each occurrence) O, 1 or 2;
m is 0 or 1;
n is 0, 1 or 2;
provided that when m is 0, then n is 1 or 2;
or a salt or solvate thereof.

In a further aspect of the invention there is provided a compound of the formula (I) as hereinbefore defined, or a salt or solvate thereof, wherein:
R⁴ is selected from hydrogen, (1-4C)alkyl [optionally substituted by 1 or 2 substituents independently selected from HET-2, -OR⁵, -SO₂R⁵, (3-6C)cycloalkyl (optionally substituted with 1 group selected from R⁷) and -C(O)NR⁵R⁵], and HET-2;
HET-3 as an 6-10 membered bicyclic saturated or partially unsaturated heterocyclyl ring, optionally containing 1 further nitrogen atom (in addition to the linking N atom) wherein a -CH₂- group can optionally be replaced by a -C(O)-, is optionally substituted on an available carbon or nitrogen atom by 1 substituent selected from R³.

In another aspect of the invention, there is provided a compounds of the formula (I) as hereinbefore defined, wherein
R¹ is methoxymethyl;
R² is HET-2;
HET-1 is a 5- or 6-membered, C-linked heteroaryl ring containing a nitrogen atom in the 2-position and optionally 1 or 2 further ring heteroatoms independently selected from O, N and S; which ring is optionally substituted on an available carbon atom, or on a ring nitrogen atom provided it is not thereby quaternised, with 1 or 2 substituents independently selected from R⁶;
HET-2 is a 4-, 5- or 6-membered, C- or N-linked heterocyclyl ring containing 1, 2, 3 or 4 heteroatoms independently selected from O, N and S, wherein a -CH₂- group can optionally be replaced by a -C(O)- , and wherein a sulphur atom in the heterocyclic ring may optionally be oxidised to a S(O) or S(O)₂ group, which ring is optionally substituted on an available carbon or nitrogen atom by 1 or 2 substituents independently selected from R⁷;
R³ is selected from halo, fluoromethyl, difluoromethyl, trifluoromethyl, methyl, methoxy and cyano;
R⁴ is selected from hydrogen, (1-4C)alkyl [optionally substituted by 1 or 2 substituents independently selected from HET-2, -OR⁵, -SO₂R⁵, (3-6C)cycloalkyl (optionally substituted with 1 group selected from R⁷) and -C(O)NR⁵R⁵], (3-6C)cycloalkyl (optionally substituted with 1 group selected from R⁷) and HET-2;
R⁵ is hydrogen or (1-4C)alkyl;
   or R⁴ and R⁵ together with the nitrogen atom to which they are attached may form a heterocyclyl ring system as defined by HET-3;
R⁶ is independently selected from (1-4C)alkyl, halo, hydroxy(1-4C)alkyl, (1-4C)alkoxy(1-4C)alkyl, (1-4C)alkylS(O)p(1-4C)alkyl, amino(1-4C)alkyl, (1-4C)alkylamino(1-4C)alkyl, di(1-4C)alkylamino(1-4C)alkyl and HET-4;
R⁷ is selected from -OR⁵, (1-4C)alkyl, -C(O)(1-4C)alkyl, -C(O)NR⁴R⁵, (1-4C)alkoxy(1-4C)alkyl, hydroxy(1-4C)alkyl and -S(O)pR⁵;
HET-3 is an N-linked, 4, 5 or 6 membered, saturated or partially unsaturated heterocyclyl ring, optionally containing 1 or 2 further heteroatoms (in addition to the linking N atom) independently selected from O, N and S, wherein a -CH₂- group can optionally be replaced by a -C(O)- and wherein a sulphur atom in the ring may optionally be oxidised to a S(O) or S(O)₂ group; which ring is optionally substituted on an available carbon or nitrogen atom by 1 or 2 substituents independently selected from R⁸; or
HET-3 is an N-linked, 7 membered, saturated or partially unsaturated heterocyclyl ring, optionally containing 1 further heteroatom (in addition to the linking N atom) independently selected from O, S and N, wherein a -CH₂- group can optionally be replaced by a -C(O)-group and wherein a sulphur atom in the ring may optionally be oxidised to a S(O) or S(O)₂ group; which ring is optionally substituted on an available carbon or nitrogen atom by 1 or 2 substituents independently selected from R⁸; or
HET-3 is an 6-10 membered bicyclic saturated or partially unsaturated heterocyclyl ring, optionally containing 1 further nitrogen atom (in addition to the linking N atom) wherein a -CH₂- group can optionally be replaced by a -C(O)-; which ring is optionally substituted on an available carbon or nitrogen atom by 1 substituent selected from hydroxy and R³;
R⁸ is selected from -OR⁵, (1-4C)alkyl, -C(O)(1-4C)alkyl, -C(O)NR⁴R⁵, (1-4C)alkylamino, di(1-4C)alkylamino, HET-3 (wherein said ring is unsubstituted), (1-4C)alkoxy(1-4C)alkyl, hydroxy(1-4C)alkyl and -S(O)pR⁵;
HET-4 is a 5- or 6-membered, C-or N- linked unsubstituted heteroaryl ring containing 1, 2 or 3 ring heteroatoms independently selected from O, N and S;
p is (independently at each occurrence) 0, 1 or 2;
m is 0 or 1;
n is 0, 1 or 2;
provided that when m is 0, then n is 1 or 2;
or a salt or solvate thereof;

It will be understood that when R⁴ is -C(O)NR⁵R⁵, each R⁵ is independently selected from hydrogen and (1-4C)alkyl, and therefore this definition of R⁴ includes (but is not limited to) -CONH₂, -CONHMe, -CONMe₂ and -CONMeEt.

It will be understood that where a compound of the formula (I) contains more than one HET-2 ring, they may be the same or different.

It will be understood that where a compound of the formula (I) contains more than one group R⁴, they may be the same or different.

It will be understood that where a compound of the formula (I) contains more than one group R⁵, they may be the same or different.

It will be understood that where a compound of the formula (I) contains more than one group R⁸, they may be the same or different.

A similar convention applies for all other groups and substituents on a compound of formula (I) as hereinbefore defined.

Compounds of Formula (I) may form salts which are within the ambit of the invention. Pharmaceutically acceptable salts are preferred although other salts may be useful in, for example, isolating or purifying compounds.

In another aspect, the invention relates to compounds of formula (I) as hereinabove defined or to a pharmaceutically acceptable salt.

In another aspect, pro-drugs of compounds of formula (I) as hereinabove defined are provided. Suitable examples of pro-drugs of compounds of formula (I) are in-vivo hydrolysable esters of compounds of formula (I).

In this specification the generic term "alkyl" includes both straight-chain and branched-chain alkyl groups. However references to individual alkyl groups such as "propyl" are specific for the straight chain version only and references to individual branched-chain alkyl groups such as *t*-butyl are specific for the branched chain version only. For example, "(1-4C)alkyl" includes methyl, ethyl, propyl, isopropyl and t-butyl. An analogous convention applies to other generic terms.

For the avoidance of doubt, reference to the group HET-1 containing a nitrogen in the 2-position, is intended to refer to the 2-position relative to the amide nitrogen atom to which the group is attached. For example, the following structures are encompassed (but not limited to):

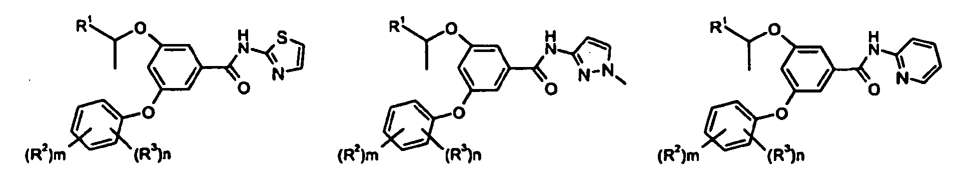

Suitable examples of HET-1 as a 5- or 6-membered, C-linked heteroaryl ring as hereinbefore defined, include thiazolyl, isothiazolyl, thiadiazolyl, pyridyl, pyrazinyl, pyridazinyl, pyrazolyl, imidazolyl, pyrimidinyl, oxazolyl, isoxazolyl, oxadiazolyl and triazolyl.

It will be understood that HET-2 can be a saturated, or partially or fully unsaturated ring.

Suitable examples of HET-2 include azetidinyl, furyl, thienyl, thiazolyl, isothiazolyl, thiadiazolyl, pyridyl, pyrazinyl, pyridazinyl, pyrazolyl, imidazolyl, pyrimidinyl, oxazolyl, isoxazolyl, oxadiazolyl, morpholino, morpholinyl, piperidinyl, piperazinyl, morpholinyl, thiomorpholinyl, pyrrolyl, pyrrolidinyl, pyrrolidonyl, 2,5-dioxopyrrolidinyl, 1,1-dioxotetrahydrothienyl, 2-oxoimidazolidinyl, 2,4-dioxoimidazolidinyl, 2-oxo-1,3,4-(4-triazolinyl), 2-oxazolidinonyl, 2-oxotetrahydrofuranyl, tetrahydrofuranyl, tetrahydropyranyl, 1,1-dioxothiomorpholino, 1,3-dioxolanyl, 1,2,4-triazolyl, 1,2,3-triazolyl, pyranyl, and 4-pyridonyl.

It will be understood that HET-2 may be linked by any appropriate available C or N atom, therefore for example, for HET-2 as "imidazolyl" includes 1- , 2-, 4- and 5- imidazolyl.

Suitable examples of HET-3 as a 4-6 membered saturated or partially unsaturated heterocyclic ring are morpholino, piperidinyl, piperazinyl, pyrrolidinyl and azetidinyl.

Suitable examples of HET-3 as a 7-membered saturated or partially unsaturated heterocyclic ring are homopiperazinyl, homo-morpholino, homo-thiomorpholino (and versions thereof wherein the sulfur is oxidised to an SO or S(O)₂ group) and homo- piperidinyl.

Suitable examples of HET-3 as an 6-10 membered bicyclic heterocyclic ring are bicyclic saturated or partially unsaturated heterocyclyl ring such as those illustrated by the structures shown below (wherein the dotted line indicates the point of attachment to the rest of the molecule):

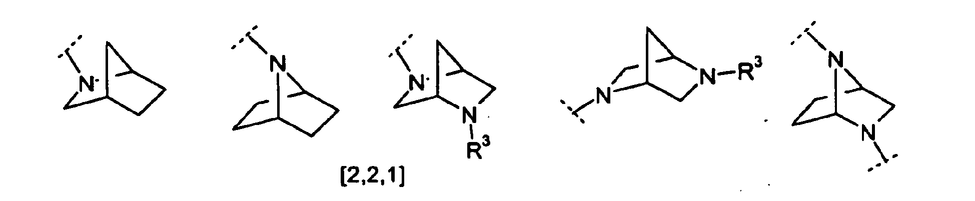

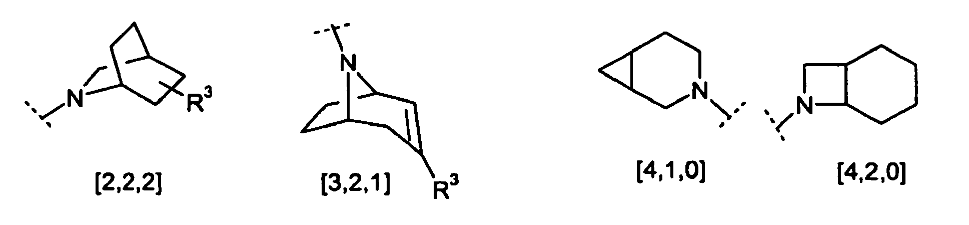

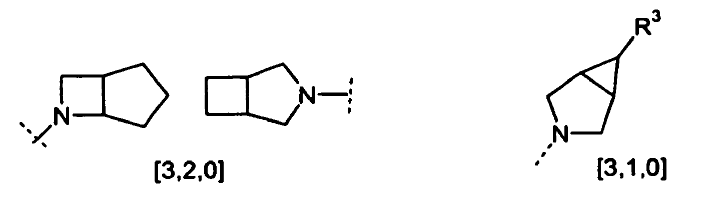

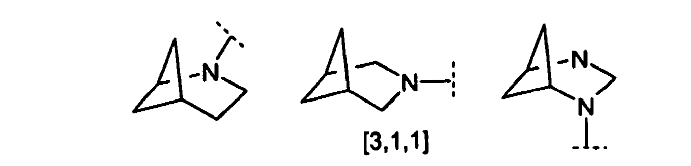

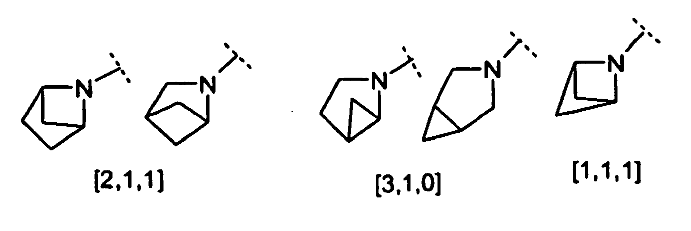

In particular HET-3 is a [2,2,1] system such as 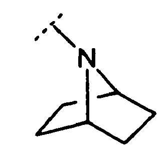 (7-azabicyclo[2.2.1]hept-7-yl).

Suitable examples of HET-4 are furyl, pyrrolyl, thienyl, thiazolyl, isothiazolyl, thiadiazolyl, pyridyl, pyrazinyl, pyridazinyl, pyrazolyl, imidazolyl, pyrimidinyl, oxazolyl, isoxazolyl and triazolyl.

It will be appreciated that, where definitions of heterocylyl groups HET-1 to HET-4 encompass heteroaryl rings which may be substituted on nitrogen, such substitution may not result in charged quaternary nitrogen atoms. It will be appreciated that the definitions of HET-1 to HET-4 are not intended to include any O-O, O-S or S-S bonds. It will be appreciated that the definitions of HET-1 to HET-4 are not intended to include unstable structures.

Examples of **(1-4C)alkyl** include methyl, ethyl, propyl, isopropyl, butyl and tert-butyl; examples of **(3-6C)cycloalkyl** include cyclopropyl, cyclobutyl, cyclopentyl and cyclohexyl; examples of **halo** include fluoro, chloro, bromo and iodo; examples **of hydroxy(1-4C)alkyl** include hydroxymethyl, 1-hydroxyethyl, 2-hydroxyethyl, 2-hydroxypropyl, 3-hydroxypropyl, 1-hydroxyisopropyl and 4-hydroxybutyl; examples of **(1-4C)alkoxy(1-4C)alkyl** include methoxymethyl, ethoxymethyl, tert-butoxymethyl, 2-methoxyethyl, 2-ethoxyethyl, methoxypropyl, 2-methoxypropyl and methoxybutyl; examples of **(1-4C)alkylS(O)p(1-4C)alkyl** include methylsulfinylmethyl, ethylsulfinylmethyl, ethylsulfinylethyl, methylsulfinylpropyl, methylsulfinylbutyl, methylsulfonylmethyl, ethylsulfonylmethyl, ethylsulfonylethyl, methylsulfonylpropyl, methylsulfonylbutyl, methylthiomethyl, ethylthiomethyl, ethylthioethyl, methylthiopropyl, and methylthiobutyl; examples of **amino(1-4C)alkyl** include aminomethyl, aminoethyl, 2-aminopropyl, 3-aminopropyl, 1-aminoisopropyl and 4-aminobutyl; examples of **(1-4C)alkylamino(1-4C)alkyl** include (N-methyl)aminomethyl, (N-ethyl)aminomethyl, 1-((N-methyl)amino)ethyl, 2-((N-methyl)amino)ethyl, (N-ethyl)aminoethyl, (N-methyl)aminopropyl, and 4-((N-methyl)amino)butyl; examples of **di(1-4C)alkylamino(1-4C)alkyl** include dimethylaminomethyl, methyl(ethyl)aminomethyl, methyl(ethyl)aminoethyl, (N,N-diethyl)aminoethyl, (N,N-dimethyl)aminopropyl and (N,N-dimethyl)aminobutyl; examples of **(1-4C)alkylamino** include methylamino, ethylamino, propylamino, isopropylamino, butylamino and tert-butylamino; examples of **di(1-4C)alkylamino** include dimethylamino, methyl(ethyl)amino, diethylamino, dipropylamino, di-isopropylamino and dibutylamino; examples of **-C(O)(1-4C)alkyl** include methylcarbonyl, ethylcarbonyl, propylcarbonyl and tert-butyl carbonyl.

It is to be understood that, insofar as certain of the compounds of Formula (I) defined above may exist in optically active or racemic forms by virtue of one or more asymmetric carbon atoms, the invention includes in its definition any such optically active or racemic form which possesses the property of stimulating GLK directly or inhibiting the GLK/GLKRP interaction. The synthesis of optically active forms may be carried out by standard techniques of organic chemistry well known in the art, for example by synthesis from optically active starting materials or by resolution of a racemic form. It is also to be understood that certain compounds may exist in tautomeric forms and that the invention also relates to any and all tautomeric forms of the compounds of the invention which activate GLK.

In one embodiment of the invention are provided compounds of formula (I), in an alternative embodiment are provided pharmaceutically-acceptable salts of compounds of formula (I), in a further alternative embodiment are provided in-vivo hydrolysable esters of compounds of formula (I), and in a further alternative embodiment are provided pharmaceutically-acceptable salts of in-vivo hydrolysable esters of compounds of formula (I).

Preferred values of each variable group are as follows. Such values may be used where appropriate with any of the values, definitions, claims, aspects or embodiments defined hereinbefore or hereinafter. In particular, each may be used as an individual limitation on the broadest definition of formula (I). Further, each of the following values may be used in combination with one or more of the other following values to limit the broadest defintion of formula (I).
(1) R¹ is methoxymethyl and the configuration is preferably (S), that is: 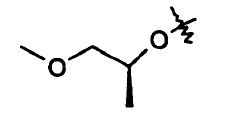
(2) R² is -C(O)NR⁴R⁵
(3) R² is -SO₂NR⁴R⁵
(4) R² is -S(O)ₚR⁴
(5) R² is HET-2
(6) m is 1 and R² is in the para position relative to the ether linkage
(7) m is 1 and n is 0 or 1
(8) m is 1 and n is 0
(9) m is 1, n is 0 and R² is in the para position relative to the ether linkage
(10) m is 1, n is 1, R² is in the para position relative to the ether linkage, R³ is in the ortho position relative to the ether linkage
(11) m is 1, n is 1, R² is in the para position relative to the ether linkage, R³ is in the meta position relative to the ether linkage
(12)nis0
(13) n is 1
(14) n is 2
(15) n is 2 and both R³ are halo
(16) n is 2 and each R³ is independently halo or methoxy
(17) m is 1, n is 2 and R² is in the para position relative to the ether linkage
(18) m is 1, n is 2, R² is in the para position relative to the ether linkage and each R³ is in an ortho position relative to the ether linkage
(19) m is 1, n is 2, both R³ are halo, R² is in the para position relative to the ether linkage and each R³ is in an ortho position relative to the ether linkage
(20) R³ is fluoromethyl or difluoromethyl
(21) R³ is halo or trifluoromethyl
(22) R³ is halo
(23) R³ is chloro or fluoro
(24) R³ is fluoro
(25) R³ is methoxy
(26) n is 2 and both R³ are fluoro,
(27) n is 2, both R³ are fluoro and are in the 3- and 5-positions (meta-positions) relative to the ether linkage
(28) m is 1, n is 2, R² is in the para position relative to the ether linkage, both R³ are fluoro and are in the 3- and 5-positions relative to the ether linkage
(29) p is 0
(30) p is 1
(31) p is 2
(32) HET-1 is a 5-membered heteroaryl ring
(33) HET-1 is a 6-membered heteroaryl ring
(34) HET-1 is substituted with 1 or 2 substituents independently selected from R⁶
(35) HET-1 is substituted with 1 substituent selected from R⁶
(36) HET-1 is unsubstituted
(37) HET-1 is selected from thiazolyl, isothiazolyl, thiadiazolyl, pyridyl, pyrazinyl, pyridazinyl, pyrazolyl, imidazolyl, pyrimidinyl, oxazolyl, isoxazolyl, oxadiazolyl, and triazolyl
(38) HET-1 is selected from thiazolyl, isothiazolyl, thiadiazolyl, pyrazolyl, imidazolyl, oxazolyl, isoxazolyl and oxadiazolyl
(39) HET-1 is selected from pyridyl, pyrazinyl, pyridazinyl and pyrimidinyl
(40) HET-1 is selected from thiazolyl, pyrazolyl and oxazolyl
(41) HET-1 is selected from thiadiazolyl and oxadiazolyl
(42) HET-1 is selected from 1,3,4-thiadiazolyl and 1,3,4-oxadiazolyl
(43) HET-1 is selected from 1,2,4-oxadiazolyl and 1,2,4-oxadiazolyl
(44) HET-1 is pyrazolyl
(45) HET-1 is pyridyl or pyrazinyl
(46) HET-1 is selected from thiazolyl, pyrazolyl, thiadiazolyl and pyridyl;
(47) R⁶ is selected from (1-4C)alkyl, halo, hydroxy(1-4C)alkyl, di(1-4C)alkylamino(1-4C)alkyl and HET-4
(48) R⁶ is selected from methyl, ethyl, bromo, chloro, fluoro, hydroxymethyl, methoxymethyl, aminomethyl, N-methylaminomethyl, dimethylaminomethyl
(49) R⁶ is selected from (1-4C)alkyl, halo, hydroxy(1-4C)alkyl, (1-4C)alkoxy(1-4C)alkyl, (1-4C)alkylS(O)p(1-4C)alkyl, amino(1-4C)alkyl, (1-4C)alkylamino(1-4C)alkyl, and di(1-4C)alkylamino(1 -4C)alkyl
(50) R⁶ is selected from methyl, ethyl, bromo, chloro, fluoro, aminomethyl, N-methylaminomethyl, and dimethylaminomethyl
(51) R⁶ is selected from methyl, ethyl, bromo, chloro, fluoro, hydroxymethyl and methoxymethyl
(52) R⁶ is selected from methyl, ethyl, bromo, chloro and fluoro
(53) R⁶ is methyl
(54) R⁶ is selected from methyl, ethyl, bromo, chloro, fluoro, aminomethyl, N-methylaminomethyl, dimethylaminomethyl, hydroxymethyl and methoxymethyl
(55) R⁶ is selected from methyl, ethyl, aminomethyl, N-methylaminomethyl, dimethylaminomethyl, hydroxymethyl and methoxymethyl
(56) R⁶ is selected from methyl, ethyl, isopropyl and methoxymethyl
(57) when 2 substituents R⁶ are present, both are selected from methyl, ethyl, bromo, chloro and fluoro; preferably both are methyl
(58) R⁶ is selected from (1-4C)alkylS(O)p(1-4C)alkyl, (1-4C)alkylamino(1-4C)alkyl, di(1-4C)alkylamino(1-4C)alkyl and HET-4
(59) R⁶ is HET-4
(60) HET-4 is selected from furyl, pyrrolyl and thienyl
(61) HET-4 is furyl
(62) R⁴ is hydrogen
(63) R⁴ is (1-4C)alkyl [substituted by 1 or 2 substituents independently selected from HET-2, -OR⁵, -SO₂R⁵, (3-6C)cycloalkyl (optionally substituted with 1 group selected from R⁷) and -C(O)NR⁵R⁵]
(64) R⁴ is (1-4C)alkyl [substituted by 1 substituent selected from HET-2, -OR⁵, -SO₂R⁵, (3-6C)cycloalkyl and -C(O)NR⁵R⁵]
(65) R⁴ is (1-4C)alkyl
(66) R⁴ is (1-4C)alkyl substituted by -OR⁵
(67) R⁴ is (1-4C)alkyl substituted by HET-2
(68) R⁴ is (3-6C)cycloalkyl, particularly cyclopropyl
(69) R⁴ is (3-6C)cycloalkyl substituted by a group selected from R⁷
(70) R⁴ is (3-6C)cycloalkyl substituted by a group selected from -OR⁵ and (1-4C)alkyl
(71) R⁴ is HET-2
(72) R⁴ is selected from hydrogen, (1-4C)alkyl, and (1-4C)alkyl substituted with -OR⁵
(73) HET-2 is unsubstituted
(74) HET-2 is substituted with 1 or 2 substituents independently selected from (1-4C)alkyl, hydroxy and (1-4C)alkoxy
(75) HET-2 is a fully saturated ring system
(76) HET-2 is a fully unsaturated ring system
(77) HET-2 is selected from azetidinyl, morpholino, morpholinyl, piperidinyl, piperazinyl, 3-oxopiperazinyl, thiomorpholinyl, pyrrolidinyl, pyrrolidonyl, 2,5-dioxopyrrolidinyl, 1,1-dioxotetrahydrothienyl, 2-oxazolidinonyl, 2-oxotetrahydrofuranyl, tetrahydrofuranyl, tetrahydropyranyl, 1,1-dioxothiomorpholino, 1,3-dioxolanyl, 2-oxoimidazolidinyl, 2,4-dioxoimidazolidinyl, pyranyl and 4-pyridonyl
(78) HET-2 is selected from azetidinyl, morpholino, morpholinyl, piperidinyl, piperazinyl, pyrrolidinyl, thiomorpholinyl, tetrahydrofuranyl, and tetrahydropyranyl
(79) HET-2 is selected from furyl, thienyl, thiazolyl, isothiazolyl, thiadiazolyl, pyridyl, pyrazinyl, pyridazinyl, pyrazolyl, imidazolyl, pyrimidinyl, oxazolyl, isoxazolyl, oxadiazolyl, pyrrolyl, 1,2,4-triazolyl and 1,2,3-triazolyl
(80) HET-2 is selected from furyl, thienyl, thiazolyl, isothiazolyl, thiadiazolyl, pyridyl, imidazolyl, pyrimidinyl, oxazolyl, isoxazolyl, oxadiazolyl, piperidinyl, piperazinyl, 3-oxopiperazinyl, pyrrolidinyl, pyrrolidonyl, 2-oxazolidinonyl, tetrahydrofuranyl, tetrahydropyranyl, 1,1-dioxotetrahydrothienyl, and 2-oxoimidazolidinyl
(81) HET-2 is selected from morpholino, furyl, imidazolyl, oxazolyl, isoxazolyl, oxadiazolyl, piperidinyl, piperazinyl, 3-oxopiperazinyl, pyrrolidinyl, 2-pyrrolidonyl, 2-oxazolidinonyl, tetrahydrofuranyl, tetrahydropyranyl, 1,1-dioxotetrahydrothienyl, and 2-oxoimidazolidinyl
(82) HET-2 is selected from morpholino, furyl, imidazolyl, isoxazolyl, oxadiazolyl, piperidinyl, piperazinyl, 3-oxopiperazinyl, pyrrolidinyl, 2-pyrrolidonyl, tetrahydropyranyl, 1,1-dioxotetrahydrothienyl, and 2-oxoimidazolidinyl
(83) R⁵ is hydrogen
(84) R⁵ is (1-4)alkyl, preferably methyl
(85) R⁵ is hydrogen or methyl
(86) R⁷ is selected from -OR⁵, (1-4C)alkyl, -C(O)(1-4C)alkyl, -C(O)NR⁴R⁵, (1-4C)alkoxy(1-4C)alkyl, and hydroxy(1-4C)alkyl
(87) R⁷ is selected from -OR⁵, (1-4C)alkyl, -C(O)(1-4C)alkyl, -C(O)NR⁴R⁵, and hydroxy(1-4C)alkyl
(88) R⁷ is selected from hydroxy, methoxy, -COMe, -CONH₂, -CONHMe, -CONMe₂, and hydroxymethyl
(89) R⁷ is selected from (1-4C)alkyl, hydroxy and (1-4C)alkoxy
(90) R⁷ is selected from methyl, ethyl, methoxy and hydroxy
(91) R⁷ is methyl
(92) R⁸ is selected from methyl, hydroxy, methoxy, -COMe, -CONH₂, -CONHMe, -CONMe₂, hydroxymethyl, hydroxyethyl, -NHMe and -NMe₂
(93) R⁸ is selected from morpholino, piperidinyl, piperazinyl, pyrrolidinyl and azetidinyl
(94) R⁸ is selected from methyl, -COMe, -CONH₂, hydroxyethyl and hydroxy
(95) R⁸ is methyl
(96) HET-3 is a fully saturated ring
(97) HET-3 is selected from morpholino, piperidinyl, piperazinyl, pyrrolidinyl and azetidinyl
(98) R⁴ and R⁵ together with the nitrogen to which they are attached form a ring as defined by HET-3
(99) HET-3 is selected from pyrrolidinyl and azetidinyl
(100) HET-3 is azetidinyl
(101) HET-3 is a 4, 5 or 6-membered saturated or partially unsaturated heterocyclic ring as hereinbefore defined
(102) HET-3 is a 7-membered saturated or partially unsaturated heterocyclic ring as hereinbefore defined
(103) HET-3 is an 6 to 10-membered bicyclic saturated or partially unsaturated heterocyclic ring as hereinbefore defined
(104) HET-3 is 7-azabicyclo[2.2.1]hept-7-yl
(105) HET-3 is selected from morpholino, piperidinyl, piperazinyl, pyrrolidinyl, azetidinyl and 7-azabicyclo[2.2.1]hept-7-yl
(106) HET-3 is selected from piperidinyl, pyrrolidinyl, azetidinyl and 7-azabicyclo[2.2.1]hept-7-yl

According to a further feature of the invention there is provided the following preferred groups of compounds of the invention:

In a further aspect of the invention there is provided a compound of Formula (I) wherein:
R¹ is methoxymethyl;
R² is selected from -C(O)NR⁴R⁵, -SO₂NR⁴R⁵, -S(O)ₚR⁴ and HET-2;
HET-1 is a 5- or 6-membered, C-linked heteroaryl ring containing a nitrogen atom in the 2-position and optionally 1, 2 or 3 further ring heteroatoms independently selected from O, N and S; which ring is optionally substituted on an available carbon atom, or on a ring nitrogen atom provided it is not thereby quaternised, with 1 or 2 substituents independently selected from R⁶;
HET-2 is a 5- or 6-membered, C- or N-linked heterocyclyl ring containing 1, 2, 3 or 4 heteroatoms independently selected from O, N and S, wherein a -CH₂- group can optionally be replaced by a -C(O)- , and wherein a sulphur atom in the heterocyclic ring may optionally be oxidised to an S(O) or S(O)₂ group, which ring is optionally substituted on an available carbon or nitrogen atom by 1 or 2 substituents independently selected from R⁷;
R³ is selected from halo, fluoromethyl, difluoromethyl, trifluoromethyl, methyl, methoxy and cyano;
R⁴ is selected from hydrogen, (1-4C)alkyl, [optionally substituted by -OR⁵] and HET-2;
R⁵ is hydrogen or (1-4C)alkyl;
   or R⁴ and R⁵ together with the nitrogen atom to which they are attached may form a 4-6 membered heterocyclyl ring system as defined by HET-3;
R⁶ is independently selected from (1-4C)alkyl, halo, hydroxy(1-4C)alkyl, (1-4C)alkoxy(1-4C)alkyl, (1-4C)alkylS(O)p(1-4C)alkyl, amino(1-4C)alkyl, (1-4C)alkylamino(1-4C)alkyl, di(1-4C)alkylamino(1-4C)alkyl and HET-4;
R⁷ is selected from -OR⁵ and (1-4C)alkyl;
HET-3 is an N-linked, 4 to 6 membered, saturated or partially unsaturated heterocyclyl ring, optionally containing 1 or 2 further heteroatoms (in addition to the linking N atom) independently selected from O, N and S, wherein a -CH₂- group can optionally be replaced by a -C(O)- and wherein a sulphur atom in the ring may optionally be oxidised to an S(O) or S(O)₂ group; which ring is optionally substituted on an available carbon or nitrogen atom by 1 or 2 substituents independently selected from R⁸;
R⁸ is selected from -OR⁵ and (1-4C)alkyl;
HET-4 is a 5- or 6-membered, C-or N- linked unsubstituted heteroaryl ring containing 1, 2 or 3 ring heteroatoms independently selected from O, N and S;
p is (independently at each occurrence) 0, 1 or 2;
m is 0 or 1;
n is 0, 1 or 2;
provided that when m is 0, then n is I or 2;
or a salt or solvate thereof.

In a further aspect of the invention there is provided a compound of Formula (I) wherein:
R¹ is methoxymethyl;
R² is selected from -C(O)NR⁴R⁵, -SO₂NR⁴R⁵, -S(O)ₚR⁴ and HET-2;
HET-1 is a 5- or 6-membered, C-linked heteroaryl ring containing a nitrogen atom in the 2-position and optionally 1, 2 or 3 further ring heteroatoms independently selected from O, N and S; which ring is optionally substituted on an available carbon atom, or on a ring nitrogen atom provided it is not thereby quaternised, with 1 or 2 substituents independently selected from R⁶;
HET-2 is a 5- or 6-membered, C- or N-linked heterocyclyl ring containing 1, 2, 3 or 4 heteroatoms independently selected from O, N and S, wherein a -CH₂- group can optionally be replaced by a -C(O)- , and wherein a sulphur atom in the heterocyclic ring may optionally be oxidised to an S(O) or S(O)₂ group, which ring is optionally substituted on an available carbon or nitrogen atom by 1 or 2 substituents independently selected from R⁷;
R³ is selected from halo, fluoromethyl, difluoromethyl, trifluoromethyl, methyl, methoxy and cyano;
R⁴ is selected from hydrogen, (1-4C)alkyl, [optionally substituted by -OR⁵] and HET-2;
R⁵ is hydrogen or (1-4C)alkyl;
   or R⁴ and R⁵ together with the nitrogen atom to which they are attached may form a heterocyclyl ring system as defined by HET-3;
R⁶ is independently selected from (1-4C)alkyl, halo, hydroxy(1-4C)alkyl, (1-4C)alkoxy(1-4C)alkyl, (1-4C)alkylS(O)p( 1-4C)alkyl, amino(1-4C)alkyl, (1-4C)alkylamino(1-4C)alkyl, di(1-4C)alkylamino(1-4C)alkyl and HET-4;
R⁷ is selected from -OR⁵ and (1-4C)alkyl;
HET-3 is an N-linked, 4 to 6 membered, saturated or partially unsaturated heterocyclyl ring, optionally containing 1 or 2 further heteroatoms (in addition to the linking N atom) independently selected from O, N and S, wherein a -CH₂- group can optionally be replaced by a -C(O)- and wherein a sulphur atom in the ring may optionally be oxidised to an S(O) or S(O)₂ group; which ring is optionally substituted on an available carbon or nitrogen atom by 1 or 2 substituents independently selected from R⁸; or
HET-3 is an N-linked, 7 membered, saturated or partially unsaturated heterocyclyl ring, optionally containing 1 further heteroatom (in addition to the linking N atom) independently selected from O, S and N, wherein a -CH₂- group can optionally be replaced by a -C(O)-group and wherein a sulphur atom in the ring may optionally be oxidised to an S(O) or S(O)₂ group; which ring is optionally substituted on an available carbon or nitrogen atom by 1 or 2 substituents independently selected from R⁸; or
HET-3 is an 6-10 membered bicyclic saturated or partially unsaturated heterocyclyl ring, optionally containing 1 further nitrogen atom (in addition to the linking N atom) wherein a -CH₂- group can optionally be replaced by a -C(O)-; which ring is optionally substituted on an available carbon or nitrogen atom by I substituent selected from R³;
R⁸ is selected from -OR⁵ and (1-4C)alkyl;
HET-4 is a 5- or 6-membered, C-or N- linked unsubstituted heteroaryl ring containing 1, 2 or 3 ring heteroatoms independently selected from O, N and S;
p is (independently at each occurrence) 0, 1 or 2;
m is 0 or 1;
n is 0, 1 or 2;
provided that when m is 0, then n is 1 or 2;
or a salt or solvate thereof.

In a further aspect of the invention is provided a compound of the formula (I) as hereinbefore defined wherein:
R¹ is methoxymethyl;
R² is selected from -C(O)NR⁴R⁵, -SO₂NR⁴R⁵, -S(O)ₚR⁴ and HET-2;
HET-1 is a 5- or 6-membered, C-linked heteroaryl ring containing a nitrogen atom in the 2-position and optionally 1 or 2 further ring heteroatoms independently selected from O, N and
S; which ring is optionally substituted on an available carbon atom, or on a ring nitrogen atom provided it is not thereby quaternised, with 1 or 2 substituents independently selected from R⁶;
HET-2 is a 4-, 5- or 6-membered, C- or N-linked heterocyclyl ring containing 1, 2, 3 or 4 heteroatoms independently selected from O, N and S, wherein a -CH₂- group can optionally be replaced by a -C(O)- , and wherein a sulphur atom in the heterocyclic ring may optionally be oxidised to an S(O) or S(O)₂ group, which ring is optionally substituted on an available carbon or nitrogen atom by 1 or 2 substituents independently selected from R⁷;
R³ is selected from halo, fluoromethyl, difluoromethyl, trifluoromethyl, methyl, methoxy and cyano;
R⁴ is selected from (1-4C)alkyl [substituted by 1 or 2 substituents independently selected from HET-2, -SO₂R⁵, (3-6C)cycloalkyl (optionally substituted with 1 group selected from R⁷) and -C(O)NR⁵R⁵];
R⁵ is hydrogen or (1-4C)alkyl;
   or R⁴ and R⁵ together with the nitrogen atom to which they are attached may form a 4-6 membered heterocyclyl ring system as defined by HET-3;
R⁶ is independently selected from (1-4C)alkyl, halo, hydroxy(1-4C)alkyl, (1-4C)alkoxy(1-4C)alkyl, (1-4C)alkylS(O)p(1-4C)alkyl, amino(1-4C)alkyl, (1-4C)alkylamino(1-4C)alkyl, di(1-4C)alkylamino(1-4C)alkyl and HET-4;
R⁷ is selected from -C(O)(1-4C)alkyl, -C(O)NR⁴R⁵, (1-4C)alkoxy(1-4C)alkyl, hydroxy(1-4C)alkyl and -S(O)pR⁵;
HET-3 is an N-linked, 4 to 6 membered, saturated or partially unsaturated heterocyclyl ring, optionally containing 1 or 2 further heteroatoms (in addition to the linking N atom) independently selected from O, N and S, wherein a -CH₂- group can optionally be replaced by a -C(O)- and wherein a sulphur atom in the ring may optionally be oxidised to an S(O) or S(O)₂ group; which ring is optionally substituted on an available carbon or nitrogen atom by 1 or 2 substituents independently selected from R⁸;
R⁸ is selected from -C(O)(1-4C)alkyl, -C(O)NR⁴R⁵, (1-4C)alkylamino, di(1-4C)alkylamino, HET-3 (wherein said ring is unsubstituted), (1-4C)alkoxy(1-4C)alkyl, hydroxy(1-4C)alkyl and -S(O)pR⁵;
HET-4 is a 5- or 6-membered, C-or N- linked unsubstituted heteroaryl ring containing 1, 2 or 3 ring heteroatoms independently selected from O, N and S;
p is (independently at each occurrence) 0, 1 or 2;
m is 0 or 1;
n is 0, 1 or 2;
provided that when m is 0, then n is 1 or 2;
or a salt or solvate thereof.

In a further aspect of the invention is provided a compound of the formula (I) as hereinbefore defined wherein:
R¹ is methoxymethyl;
R² is selected from -C(O)NR⁴R⁵, -SO₂NR⁴R⁵, -S(O)ₚR⁴ and HET-2;
HET-1 is a 5- or 6-membered, C-linked heteroaryl ring containing a nitrogen atom in the 2-position and optionally 1 or 2 further ring heteroatoms independently selected from O, N and
S; which ring is optionally substituted on an available carbon atom, or on a ring nitrogen atom provided it is not thereby quaternised, with 1 or 2 substituents independently selected from R⁶ ;
HET-2 is a 4-, 5- or 6-membered, C- or N-linked heterocyclyl ring containing 1, 2, 3 or 4 heteroatoms independently selected from O, N and S, wherein a -CH₂- group can optionally be replaced by a -C(O)-, and wherein a sulphur atom in the heterocyclic ring may optionally be oxidised to an S(O) or S(O)₂ group, which ring is optionally substituted on an available carbon or nitrogen atom by 1 or 2 substituents independently selected from R⁷;
R³ is selected from halo, fluoromethyl, difluoromethyl, trifluoromethyl, methyl, methoxy and cyano;
R⁴ is selected from (1-4C)alkyl [substituted by 1 or 2 substituents independently selected from HET-2, -SO₂R⁵, (3-6C)cycloalkyl (optionally substituted with 1 group selected from R⁷) and -C(O)NR⁵R⁵];
R⁵ is hydrogen or (1-4C)alkyl;
   or R⁴ and R⁵ together with the nitrogen atom to which they are attached may form a heterocyclyl ring system as defined by HET-3;
R⁶ is independently selected from (1-4C)alkyl, halo, hydroxy(1-4C)alkyl, (1-4C)alkoxy(1-4C)alkyl, (1-4C)alkylS(O)p(1-4C)alkyl, amino(1-4C)alkyl, (1-4C)alkylamino(1-4C)alkyl, di(1-4C)alkylamino(1-4C)alkyl and HET-4;
R⁷ is selected from -C(O)(1-4C)alkyl, -C(O)NR⁴R⁵, (1-4C)alkoxy(1-4C)alkyl, hydroxy(1-4C)alkyl and -S(O)pR⁵;
HET-3 is an N-linked, 4 to 6 membered, saturated or partially unsaturated heterocyclyl ring, optionally containing 1 or 2 further heteroatoms (in addition to the linking N atom) independently selected from O, N and S, wherein a -CH₂- group can optionally be replaced by a -C(O)- and wherein a sulphur atom in the ring may optionally be oxidised to an S(O) or S(O)₂ group; which ring is optionally substituted on an available carbon or nitrogen atom by 1 or 2 substituents independently selected from R⁸; or
HET-3 is an N-linked, 7 membered, saturated or partially unsaturated heterocyclyl ring, optionally containing 1 further heteroatom (in addition to the linking N atom) independently selected from O, S and N, wherein a -CH₂- group can optionally be replaced by a -C(O)-group and wherein a sulphur atom in the ring may optionally be oxidised to an S(O) or S(O)₂ group; which ring is optionally substituted on an available carbon or nitrogen atom by 1 or 2 substituents independently selected from R⁸; or
HET-3 is an 6-10 membered bicyclic saturated or partially unsaturated heterocyclyl ring, optionally containing 1 further nitrogen atom (in addition to the linking N atom), wherein a -CH₂- group can optionally be replaced by a -C(O)-; which ring is optionally substituted on an available carbon or nitrogen atom by I substituent selected from R³;
R⁸ is selected from -C(O)(1-4C)alkyl, -C(O)NR⁴R⁵, (1-4C)alkylamino, di(1-4C)alkylamino, HET-3 (wherein said ring is unsubstituted), (1-4C)alkoxy(1-4C)alkyl, hydroxy(1-4C)alkyl and -S(O)pR⁵;
HET-4 is a 5- or 6-membered, C-or N- linked unsubstituted heteroaryl ring containing 1, 2 or 3 ring heteroatoms independently selected from O, N and S;
p is (independently at each occurrence) 0, 1 or 2;
m is 0 or 1;
n is 0, 1 or 2;
provided that when m is 0, then n is 1 or 2;
   or a salt or solvate thereof.

In a further aspect of the invention is provided a compound of the formula (I) as hereinbefore defined wherein
R¹ is methoxymethyl;
m is 1 and n is 0 or 1;
HET-1 is a 5- or 6-membered heteroaryl ring;
R² is -CONR⁴R⁵ or -SO₂NR⁴R⁵;
R³ is halo or trifluoromethyl;
R⁴ is (1-4C)alkyl [optionally substituted by 1 or 2 substituents independently selected from HET-2, -OR⁵, -SO₂R⁵, (3-6C)cycloalkyl (optionally substituted with 1 group selected from R⁷) and -C(O)NR⁵R⁵];
R⁵ is hydrogen or methyl;
HET-2 is a 5- or 6- membered heterocyclyl ring as hereinbefore defined, containing 1 or 2 heteroatoms independently selected from O, N and S; and
R⁷ is selected from -OR⁵ and (-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is a 5- or 6-membered heteroaryl ring;
R² is -CONR⁴R⁵ or -SO₂NR⁴R⁵;
R³ is halo or trifluoromethyl;
R⁴ is (1-4C)alkyl [optionally substituted by 1 or 2 substituents independently selected from HET-2, -OR⁵, -SO₂R⁵, (3-6C)cycloalkyl (optionally substituted with 1 group selected from R⁷) and -C(O)NR⁵R⁵];
R⁵ is hydrogen or methyl;
R⁶ is selected from methyl, ethyl, bromo, chloro, fluoro, aminomethyl, N-methylaminomethyl, and dimethylaminomethyl;
HET-2 is a 5- or 6- membered heterocyclyl ring as hereinbefore defined, containing 1 or 2 heteroatoms independently selected from O, N and S; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from thiazolyl, isothiazolyl, thiadiazolyl, pyrazolyl, imidazolyl, oxazolyl, isoxazolyl and oxadiazolyl;
R² is -CONR⁴R⁵ or -SO₂NR⁴R⁵;
R³ is halo or trifluoromethyl;
R⁴ is (1-4C)alkyl [optionally substituted by 1 or 2 substituents independently selected from HET-2, -OR⁵, -SO₂R⁵, (3-6C)cycloalkyl and -C(O)NR⁵R⁵];
R⁵ is hydrogen or methyl;
R⁶ is selected from methyl, ethyl, bromo, chloro, fluoro, aminomethyl, N-methylaminomethyl, and dimethylaminomethyl;
HET-2 is selected from azetidinyl, morpholino, morpholinyl, piperidinyl, piperazinyl, 3-oxopiperazinyl, thiomorpholinyl, pyrrolidinyl, pyrrolidonyl, 2,5-dioxopyrrolidinyl, 1,1-dioxotetrahydrothienyl, 2-oxazolidinonyl, 2-oxotetrahydrofuranyl, tetrahydrofuranyl, tetrahydropyranyl, 1,1-dioxothiomorpholino, 1,3-dioxolanyl, 2-oxoimidazolidinyl, 2,4-dioxoimidazolidinyl, pyranyl and 4-pyridonyl; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from pyridyl, pyrazinyl, pyridazinyl and pyrimidinyl;
R² is -CONR⁴R⁵ or -SO₂NR⁴R⁵;
R³ is halo or trifluoromethyl;
R⁴ is (1-4C)alkyl [optionally substituted by 1 or 2 substituents independently selected from HET-2, -OR⁵, -SO₂R⁵, (3-6C)cycloalkyl and -C(O)NR⁵R⁵];
R⁵ is hydrogen or methyl;
R⁶ is selected from methyl, ethyl, bromo, chloro, fluoro, aminomethyl, N-methylaminomethyl, and dimethylaminomethyl;
HET-2 is selected from azetidinyl, morpholino, morpholinyl, piperidinyl, piperazinyl, 3-oxopiperazinyl, thiomorpholinyl, pyrrolidinyl, pyrrolidonyl, 2,5-dioxopyrrolidinyl, 1,1-dioxotetrahydrothienyl, 2-oxazolidinonyl, 2-oxotetrahydrofuranyl, tetrahydrofuranyl, tetrahydropyranyl, 1,1-dioxothiomorpholino, 1,3-dioxolanyl, 2-oxoimidazolidinyl, 2,4-dioxoimidazolidinyl, pyranyl and 4-pyridonyl; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from thiazolyl, isothiazolyl, thiadiazolyl, pyrazolyl, imidazolyl, oxazolyl, isoxazolyl and oxadiazolyl;
R² is -CONR⁴R⁵ or -SO₂NR⁴R⁵;
R³ is halo or trifluoromethyl;
R⁴ is (1-4C)alkyl [optionally substituted by 1 or 2 substituents independently selected from HET-2, -OR⁵, -SO₂R⁵, (3-6C)cycloalkyl and -C(O)NR⁵R⁵];
R⁵ is hydrogen or methyl;
R⁶ is selected from methyl, ethyl, bromo, chloro, fluoro, aminomethyl, N-methylaminomethyl, and dimethylaminomethyl;
HET-2 is selected from furyl, thienyl, thiazolyl, isothiazolyl, thiadiazolyl, pyridyl, pyrazinyl, pyridazinyl, pyrazolyl, imidazolyl, pyrimidinyl, oxazolyl, isoxazolyl, oxadiazolyl, pyrrolyl, 1,2,4-triazolyl and 1,2,3-triazolyl; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from pyridyl, pyrazinyl, pyridazinyl and pyrimidinyl;
R² is -CONR⁴R⁵ or -SO₂NR⁴R⁵;
R³ is halo or trifluoromethyl;
R⁴ is (1-4C)alkyl [optionally substituted by 1 or 2 substituents independently selected from HET-2, -OR⁵, -SO₂R⁵, (3-6C)cycloalkyl and -C(O)NR⁵R⁵];
R⁵ is hydrogen or methyl;
R⁶ is selected from methyl, ethyl, bromo, chloro, fluoro, aminomethyl, N-methylaminomethyl, and dimethylaminomethyl;
HET-2 is selected from furyl, thienyl, thiazolyl, isothiazolyl, thiadiazolyl, pyridyl, pyrazinyl, pyridazinyl, pyrazolyl, imidazolyl, pyrimidinyl, oxazolyl, isoxazolyl, oxadiazolyl, pyrrolyl, 1,2,4-triazolyl and 1,2,3-triazolyl; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from thiazolyl, isothiazolyl, thiadiazolyl, pyrazolyl, oxazolyl, isoxazolyl and oxadiazolyl;
R² is -CONR⁴R⁵ or -SO₂NR⁴R⁵;
R³ is halo or trifluoromethyl;
R⁴ is selected from hydrogen, (1-4C)alkyl [optionally substituted by -OR⁵], (3-6C)cycloalkyl (optionally substituted with 1 group selected from R⁷) and HET-2;
R⁵ is hydrogen or methyl;
R⁶ is selected from methyl, ethyl, bromo, chloro, fluoro, aminomethyl, N-methylaminomethyl, and dimethylaminomethyl;
HET-2 is selected from morpholino, furyl, imidazolyl, isoxazolyl, oxadiazolyl, piperidinyl, piperazinyl, 3-oxopiperazinyl, pyrrolidinyl, 2-pyrrolidonyl, tetrahydropyranyl, 1,1-dioxotetrahydrothienyl, and 2-oxoimidazolidinyl; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from pyridyl and pyridazinyl;
R² is -CONR⁴R⁵ or -SO₂NR⁴R⁵;
R³ is halo or trifluoromethyl;
R⁴ is selected from hydrogen, (1-4C)alkyl, [optionally substituted by -OR⁵], (3-6C)cycloalkyl (optionally substituted with 1 group selected from R⁷) and HET-2;
R⁵ is hydrogen or methyl;
R⁶ is selected from methyl, ethyl, bromo, chloro, fluoro, aminomethyl, N-methylaminomethyl, and dimethylaminomethyl;
HET-2 is selected from morpholino, furyl, imidazolyl, isoxazolyl, oxadiazolyl, piperidinyl, piperazinyl, 3-oxopiperazinyl, pyrrolidinyl, 2-pyrrolidonyl, tetrahydropyranyl, 1,1-dioxotetrahydrothienyl, and 2-oxoimidazolidinyl; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from thiazolyl, isothiazolyl, thiadiazolyl, pyrazolyl, oxazolyl, isoxazolyl and oxadiazolyl;
R² is -CONR⁴R⁵ or -SO₂NR⁴R⁵;
R³ is halo or trifluoromethyl;
R⁴ is selected from (1-4C)alkyl, [optionally substituted by -OR⁵], (3-6C)cycloalkyl (optionally substituted with 1 group selected from R⁷) and HET-2;
R⁵ is hydrogen or methyl;
R⁶ is selected from methyl, ethyl, bromo, chloro, fluoro, aminomethyl, N-methylaminomethyl, and dimethylaminomethyl;
HET-2 is selected from piperidinyl, piperazinyl, 3-oxopiperazinyl, 2-pyrrolidonyl, 2,5-dioxopyrrolidinyl, 2-oxotetrahydrofuranyl, tetrahydrofuranyl, tetrahydropyranyl, 2-oxoimidazolidinyl, and 2,4-dioxoimidazolidinyl; and
R⁷ is (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from thiazolyl, isothiazolyl, thiadiazolyl, pyrazolyl, oxazolyl, isoxazolyl and oxadiazolyl;
R² is -CONR⁴R⁵ or -SO₂NR⁴R⁵;
R³ is halo or trifluoromethyl;
R⁴ is selected from (1-4C)alkyl, [optionally substituted by -OR⁵], (3-6C)cycloalkyl (optionally substituted with 1 group selected from R⁷) and HET-2;
R⁵ is hydrogen or methyl;
R⁶ is selected from methyl, ethyl, bromo, chloro, fluoro, aminomethyl, N-methylaminomethyl, and dimethylaminomethyl;
HET-2 is piperidinyl or piperazinyl; and
R⁷ is (1-4C)alkyl;
or a salt or solvate thereof.

In a further aspect of the invention is provided a compound of the formula (I) as hereinbefore defined wherein
R¹ is methoxymethyl;
m is 1 and n is 0 ;
HET-1 is selected from thiazolyl, thiadiazolyl and pyrazolyl;
R² is -CONR⁴R⁵;
R⁴ is piperidinyl, optionally substituted with methyl;
R⁵ is hydrogen or methyl;
R⁶ is methyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from pyridyl and pyridazinyl;
R² is -CONR⁴R⁵ or -SO₂NR⁴R⁵;
R³ is halo or trifluoromethyl;
R⁴ is selected from (1-4C)alkyl, [optionally substituted by -OR⁵] and HET-2;
R⁵ is hydrogen or methyl;
R⁶ is selected from methyl, ethyl, bromo, chloro, fluoro, aminomethyl, N-methylaminomethyl, and dimethylaminomethyl;
HET-2 is selected from piperidinyl, piperazinyl, 3-oxopiperazinyl, 2-pyrrolidonyl,
2,5-dioxopyrrolidinyl, 2-oxazolidinonyl, 2-oxotetrahydrofuranyl, tetrahydrofuranyl, tetrahydropyranyl, 2-oxoimidazolidinyl, and 2,4-dioxoimidazolidinyl; and
R⁷ is (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from pyridyl and pyridazinyl;
R² is -CONR⁴R⁵ or -SO₂NR⁴R⁵;
R³ is halo or trifluoromethyl;
R⁴ is selected from (1-4C)alkyl, [optionally substituted by -OR⁵] and HET-2;
R⁵ is hydrogen or methyl;
R⁶ is selected from methyl, ethyl, bromo, chloro, fluoro, aminomethyl, N-methylaminomethyl, and dimethylaminomethyl;
HET-2 is piperidinyl or piperazinyl; and
R⁷ is (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from thiazolyl, isothiazolyl, thiadiazolyl, pyrazolyl, oxazolyl, isoxazolyl and oxadiazolyl;
R² is -CONR⁴R⁵ or -SO₂NR⁴R⁵;
R³ is halo or trifluoromethyl;
R⁴ and R⁵ together with the nitrogen to which they are attached form a morpholino, piperidinyl, piperazinyl, pyrrolidinyl or azetidinyl ring, which ring is optionally substituted on a carbon or nitrogen atom by R⁸;
R⁶ is selected from methyl, ethyl, bromo, chloro, fluoro, aminomethyl, N-methylaminomethyl, and dimethylaminomethyl; and
R⁸ is selected from hydroxy, (1-4C)alkoxy and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from thiazolyl, isothiazolyl, thiadiazolyl, pyrazolyl, oxazolyl, isoxazolyl and oxadiazolyl;
R² is -CONR⁴R⁵ or -SO₂NR⁴R⁵;
R³ is halo or trifluoromethyl;
R⁴ and R⁵ together with the nitrogen to which they are attached form a morpholino, piperidinyl, piperazinyl, pyrrolidinyl or azetidinyl ring, which ring is optionally substituted on a carbon or nitrogen atom by R⁸;
R⁶ is selected from methyl, ethyl, bromo, chloro, fluoro, aminomethyl, N-methylaminomethyl, and dimethylaminomethyl; and
R⁸ is pyrrolidine or piperidine;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from thiazolyl, isothiazolyl, thiadiazolyl, pyrazolyl, oxazolyl, isoxazolyl and oxadiazolyl;
R² is -CONR⁴R⁵ or -SO₂NR⁴R⁵;
R³ is halo or trifluoromethyl;
R⁴ and R⁵ together with the nitrogen to which they are attached form a morpholino, piperidinyl, piperazinyl, pyrrolidinyl or azetidinyl ring, which ring is optionally substituted on a carbon or nitrogen atom by (1-4C)alkyl; and
R⁶ is selected from methyl, ethyl, bromo, chloro, fluoro, aminomethyl, N-methylaminomethyl, and dimethylaminomethyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from pyridyl and pyridazinyl;
R² is -CONR⁴R⁵ or -SO₂NR⁴R⁵;
R³ is halo or trifluoromethyl;
R⁴ and R⁵ together with the nitrogen to which they are attached form a morpholino, piperidinyl, piperazinyl, pyrrolidinyl or azetidinyl ring, which ring is optionally substituted on a carbon or nitrogen atom by (1-4C)alkyl; and
R⁶ is selected from methyl, ethyl, bromo, chloro, fluoro, aminomethyl, N-methylaminomethyl, and dimethylaminomethyl;
or a salt or solvate thereof.

In a further aspect of the invention is provided a compound of the formula (I) as hereinbefore defined wherein
R¹ is methoxymethyl;
m is 1 and n is 0;
HET-1 is selected from thiazolyl, thiadiazolyl and pyrazolyl;
R² is -CONR⁴R⁵;
R⁴ and R⁵ together with the nitrogen to which they are attached form a piperidinyl, or piperazinyl ring, which ring is optionally substituted on a carbon or nitrogen atom by (1-4C)alkyl or by a pyrrolidinyl ring;
R⁶ is selected from methyl, ethyl, bromo, chloro, fluoro, aminomethyl, N-methylaminomethyl, and dimethylaminomethyl;
or a salt or solvate thereof.

In a further aspect of the invention is provided a compound of the formula (I) as hereinbefore defined wherein
R¹ is methoxymethyl;
m is 1 and n is 0;
HET-1 is selected from thiazolyl, thiadiazolyl and pyrazolyl;
R² is -CONR⁴R⁵;
R⁴ and R⁵ together with the nitrogen to which they are attached form an azetidinyl ring which ring is optionally substituted on a carbon atom by hydroxy;
R⁶ is selected from methyl, ethyl, bromo, chloro, fluoro, hydroxymethyl, aminomethyl, N-methylaminomethyl, and dimethylaminomethyl;
or a salt or solvate thereof.

In a further aspect of the invention is provided a compound of the formula (I) as hereinbefore defined wherein
R¹ is methoxymethyl;
m is 1 and n is 0;
HET-1 is selected from thiazolyl, thiadiazolyl and pyrazolyl;
R² is -CONR⁴R⁵;
R⁴ and R⁵ together with the nitrogen to which they are attached form a 7-membered ring HET-3 which ring is optionally substituted on a carbon or nitrogen atom by methyl;
R⁶ is selected from methyl, ethyl, bromo, chloro, fluoro, hydroxymethyl, aminomethyl, N-methylaminomethyl, and dimethylaminomethyl;
or a salt or solvate thereof.

In a further aspect of the invention is provided a compound of the formula (I) as hereinbefore defined wherein
R¹ is methoxymethyl;
m is 1 and n is 0;
HET-1 is selected from thiazolyl, thiadiazolyl and pyrazolyl;
R² is -CONR⁴R⁵;
R⁴ and R⁵ together with the nitrogen to which they are attached form a 6-10 membered bicyclic heterocyclic ring HET-3;
R⁶ is selected from methyl, ethyl, bromo, chloro, fluoro, hydroxymethyl, aminomethyl, N-methylaminomethyl, and dimethylaminomethyl;
or a salt or solvate thereof.

In a further aspect of the invention is provided a compound of the formula (I) as hereinbefore defined wherein
R¹ is methoxymethyl;
m is 1 and n is 0 or 1;
HET-1 is a 5- or 6-membered heteroaryl ring;
R² is -S(O)pR⁴;
p is 1 or 2;
R³ is halo or trifluoromethyl;
R⁴ is (1-4C)alkyl [optionally substituted by 1 or 2 substituents independently selected from HET-2, -OR⁵, -SO₂R⁵, (3-6C)cycloalkyl (optionally substituted with 1 group selected from R⁷) and -C(O)NR⁵R⁵];
R⁵ is hydrogen or methyl;
HET-2 is a 5- or 6- membered heterocyclyl ring as hereinbefore defined, containing 1 or 2 heteroatoms independently selected from O, N and S; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is a 5- or 6-membered heteroaryl ring;
R² is -S(O)pR⁴;
p is 1 or 2;
R³ is halo or trifluoromethyl;
R⁴ is (1-4C)alkyl [optionally substituted by 1 or 2 substituents independently selected from HET-2, -OR⁵, -SO₂R⁵, (3-6C)cycloalkyl (optionally substituted with 1 group selected from
R⁷) and -C(O)NR⁵R⁵];
R⁵ is hydrogen or methyl;
R⁶ is selected from methyl, ethyl, bromo, chloro, fluoro, aminomethyl, N-methylaminomethyl, and dimethylaminomethyl;
HET-2 is a 5- or 6- membered heterocyclyl ring as hereinbefore defined, containing 1 or 2 heteroatoms independently selected from O, N and S; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from thiazolyl, isothiazolyl, thiadiazolyl, pyrazolyl, imidazolyl, oxazolyl, isoxazolyl and oxadiazolyl;
R² is -S(O)pR⁴;
p is 1 or 2;
R³ is halo or trifluoromethyl;
R⁴ is (1-4C)alkyl [optionally substituted by 1 or 2 substituents independently selected from HET-2, -OR⁵, -SO₂R⁵, (3-6C)cycloalkyl and -C(O)NR⁵R⁵];
R⁵ is hydrogen or methyl;
R⁶ is selected from methyl, ethyl, bromo, chloro, fluoro, aminomethyl, N-methylaminomethyl, and dimethylaminomethyl;
HET-2 is selected from azetidinyl, morpholino, morpholinyl, piperidinyl, piperazinyl, 3-oxopiperazinyl, thiomorpholinyl, pyrrolidinyl, pyrrolidonyl, 2,5-dioxopyrrolidinyl, 1,1-dioxotetrahydrothienyl, 2-oxazolidinonyl, 2-oxotetrahydrofuranyl, tetrahydrofuranyl, tetrahydropyranyl, 1,1-dioxothiomorpholino, 1,3-dioxolanyl, 2-oxoimidazolidinyl, 2,4-dioxoimidazolidinyl, pyranyl and 4-pyridonyl; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from thiazolyl, isothiazolyl, thiadiazolyl, pyrazolyl, imidazolyl, oxazolyl, isoxazolyl and oxadiazolyl;
R² is -S(O)pR⁴;
p is 1 or 2;
R³ is halo or trifluoromethyl;
R⁴ is selected from hydrogen, (1-4C)alkyl, [optionally substituted by -OR⁵], (3-6C)cycloalkyl (optionally substituted with 1 group selected from R⁷) and HET-2;
R⁵ is hydrogen or methyl;
R⁶ is selected from methyl, ethyl, bromo, chloro, fluoro, aminomethyl, N-methylaminomethyl, and dimethylaminomethyl;
HET-2 is selected from furyl, thienyl, thiazolyl, isothiazolyl, thiadiazolyl, pyridyl, pyrazinyl, pyridazinyl, pyrazolyl, imidazolyl, pyrimidinyl, oxazolyl, isoxazolyl, oxadiazolyl, pyrrolyl, 1,2,4-triazolyl and 1,2,3-triazolyl; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from pyridyl, pyrazinyl, pyridazinyl and pyrimidinyl;
R² is -S(O)pR4;
p is 1 or 2;
R³ is halo or trifluoromethyl;
R⁴ is (1-4C)alkyl [optionally substituted by 1 or 2 substituents independently selected from HET-2, -OR⁵, -SO₂R⁵, (3-6C)cycloalkyl and -C(O)NR⁵R⁵];
R⁵ is hydrogen or methyl;
R⁶ is selected from methyl, ethyl, bromo, chloro, fluoro, aminomethyl, N-methylaminomethyl, and dimethylaminomethyl;
HET-2 is selected from azetidinyl, morpholino, morpholinyl, piperidinyl, piperazinyl, 3-oxopiperazinyl, thiomorpholinyl, pyrrolidinyl, pyrrolidonyl, 2,5-dioxopyrrolidinyl, 1,1-dioxotetrahydrothienyl, 2-oxazolidinonyl, 2-oxotetrahydrofuranyl, tetrahydrofuranyl, tetrahydropyranyl, 1,1-dioxothiomorpholino, 1,3-dioxolanyl, 2-oxoimidazolidinyl, 2,4-dioxoimidazolidinyl, pyranyl and 4-pyridonyl; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from pyridyl, pyrazinyl, pyridazinyl and pyrimidinyl;
R² is -S(O)pR⁴;
p is 1 or 2;
R³ is halo or trifluoromethyl;
R⁴ is selected from hydrogen, (1-4C)alkyl, [optionally substituted by -OR⁵], (3-6C)cycloalkyl (optionally substituted with 1 group selected from R⁷) and HET-2;
R⁵ is hydrogen or methyl;
R⁶ is selected from methyl, ethyl, bromo, chloro, fluoro, aminomethyl, N-methylaminomethyl, and dimethylaminomethyl;
HET-2 is selected from furyl, thienyl, thiazolyl, isothiazolyl, thiadiazolyl, pyridyl, pyrazinyl, pyridazinyl, pyrazolyl, imidazolyl, pyrimidinyl, oxazolyl, isoxazolyl, oxadiazolyl, pyrrolyl, 1,2,4-triazolyl and 1,2,3-triazolyl; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from thiazolyl, isothiazolyl, thiadiazolyl, pyrazolyl, imidazolyl, oxazolyl, isoxazolyl and oxadiazolyl;
R² is -S(O)pR⁴;
p is 1 or 2;
R³ is halo or trifluoromethyl;
R⁴ is (1-4C)alkyl;
R⁶ is selected from methyl, ethyl, bromo, chloro, fluoro, aminomethyl, N-methylaminomethyl, and dimethylaminomethyl;
or a salt or solvate thereof.

In a further aspect of the invention is provided a compound of the formula (I) as hereinbefore defined wherein
R¹ is methoxymethyl;
m is 1 and n is 0;
HET-1 is selected from thiazolyl, thiadiazolyl and pyrazolyl;
R² is -S(O)pR⁴;
p is 1 or 2;
R⁴ is (1-4C)alkyl;
R⁶ is methyl;
or a salt or solvate thereof.

In a further aspect of the invention is provided a compound of the formula (I) as hereinbefore defined wherein
R¹ is methoxymethyl;
m is 1 and n is 0;
HET-1 is selected from thiazolyl, thiadiazolyl and pyrazolyl;
R² is -S(O)pR⁴;
p is 1 or 2;
R⁴ is (3-6C)cycloalkyl;
R⁶ is methyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from pyridyl, pyrazinyl, pyridazinyl and pyrimidinyl;
R² is -S(O)pR⁴;
p is 1 or 2;
R³ is halo or trifluoromethyl;
R⁴ is (1-4C)alkyl;
R⁶ is selected from methyl, ethyl, bromo, chloro, fluoro, aminomethyl, N-methylaminomethyl, and dimethylaminomethyl;
or a salt or solvate thereof.

In a further aspect of the invention is provided a compound of the formula (I) as hereinbefore defined wherein
R¹ is methoxymethyl;
m is 1 and n is 0 or 1;
HET-1 is a 5- or 6-membered heteroaryl ring;
R² is HET-2;
R³ is halo or trifluoromethyl;
R⁵ is hydrogen or (1-4C)alkyl;
HET-2 is a 5- or 6- membered heterocyclyl ring as hereinbefore defined, containing 1 or 2 heteroatoms independently selected from O, N and S; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from thiazolyl, isothiazolyl, thiadiazolyl, pyrazolyl, imidazolyl, oxazolyl, isoxazolyl and oxadiazolyl;
R² is HET-2;
R³ is halo or trifluoromethyl;
R⁵ is hydrogen or methyl;
HET-2 is selected from azetidinyl, morpholino, morpholinyl, piperidinyl, piperazinyl, 3-oxopiperazinyl, thiomorpholinyl, pyrrolidinyl, pyrrolidonyl, 2,5-dioxopyrrolidinyl, 1,1-dioxotetrahydrothienyl, 2-oxazolidinonyl, 2-oxotetrahydrofuranyl, tetrahydrofuranyl, tetrahydropyranyl, 1,1-dioxothiomorpholino, 1,3-dioxolanyl, 2-oxoimidazolidinyl, 2,4-dioxoimidazolidinyl, pyranyl and 4-pyridonyl; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from thiazolyl, isothiazolyl, thiadiazolyl, pyrazolyl, imidazolyl, oxazolyl, isoxazolyl and oxadiazolyl;
R² is HET-2;
R³ is halo or trifluoromethyl;
R³ is hydrogen or methyl;
HET-2 is selected from furyl, thienyl, thiazolyl, isothiazolyl, thiadiazolyl, pyridyl, pyrazinyl, pyridazinyl, pyrazolyl, imidazolyl, pyrimidinyl, oxazolyl, isoxazolyl, oxadiazolyl, pyrrolyl, 1,2,4-triazolyl and 1,2,3-triazolyl; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from pyridyl, pyrazinyl, pyridazinyl and pyrimidinyl;
R² is HET-2;
R³ is halo or trifluoromethyl;
R⁵ is hydrogen or methyl;
HET-2 is selected from azetidinyl, morpholino, morpholinyl, piperidinyl, piperazinyl, 3-oxopiperazinyl, thiomorpholinyl, pyrrolidinyl, pyrrolidonyl, 2,5-dioxopyrrolidinyl, 1,1-dioxotetrahydrothienyl, 2-oxazolidinonyl, 2-oxotetrahydrofuranyl, tetrahydrofuranyl, tetrahydropyranyl, 1,1-dioxothiomorpholino, 1,3-dioxolanyl, 2-oxoimidazolidinyl, 2,4-dioxoimidazolidinyl, pyranyl and 4-pyridonyl; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from pyridyl, pyrazinyl, pyridazinyl and pyrimidinyl;
R² is HET-2;
R³ is halo or trifluoromethyl;
R⁵ is hydrogen or methyl;
HET-2 is selected from furyl, thienyl, thiazolyl, isothiazolyl, thiadiazolyl, pyridyl, pyrazinyl, pyridazinyl, pyrazolyl, imidazolyl, pyrimidinyl, oxazolyl, isoxazolyl, oxadiazolyl, pyrrolyl, 1,2,4-triazolyl and 1,2,3-triazolyl; and
R⁷ is selected from -OR⁵ and (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from thiazolyl, isothiazolyl, thiadiazolyl, pyrazolyl, imidazolyl, oxazolyl, isoxazolyl and oxadiazolyl;
R² is HET-2;
R³ is halo or trifluoromethyl;
R⁶ is selected from methyl, ethyl, bromo, chloro, fluoro, aminomethyl, N-methylaminomethyl, and dimethylaminomethyl;
HET-2 is selected from azetidinyl, morpholino, morpholinyl, piperidinyl, piperazinyl, 3-oxopiperazinyl, thiomorpholinyl, pyrrolidinyl, pyrrolidonyl, 2,5-dioxopyrrolidinyl, 1,1-dioxotetrahydrothienyl, 2-oxazolidinonyl, 2-oxotetrahydrofuranyl, tetrahydrofuranyl, tetrahydropyranyl, 1,1-dioxothiomorpholino, 1,3-dioxolanyl, 2-oxoimidazolidinyl, 2,4-dioxoimidazolidinyl, pyranyl and 4-pyridonyl; and
R⁷ is (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from thiazolyl, isothiazolyl, thiadiazolyl, pyrazolyl, imidazolyl, oxazolyl, isoxazolyl and oxadiazolyl;
R² is HET-2;
R³ is halo or trifluoromethyl;
R⁶ is selected from methyl, ethyl, bromo, chloro, fluoro, aminomethyl, N-methylaminomethyl, and dimethylaminomethyl;
HET-2 is selected from furyl, thienyl, thiazolyl, isothiazolyl, thiadiazolyl, pyridyl, pyrazinyl, pyridazinyl, pyrazolyl, imidazolyl, pyrimidinyl, oxazolyl, isoxazolyl, oxadiazolyl, pyrrolyl,
1,2,4-triazolyl and 1,2,3-triazolyl; and
R⁷ is (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from pyridyl, pyrazinyl, pyridazinyl and pyrimidinyl;
R² is HET-2;
R³ is halo or trifluoromethyl;
R⁶ is selected from methyl, ethyl, bromo, chloro, fluoro, aminomethyl, N-methylaminomethyl, and dimethylaminomethyl;
HET-2 is selected from azetidinyl, morpholino, morpholinyl, piperidinyl, piperazinyl, 3-oxopiperazinyl, thiomorpholinyl, pyrrolidinyl, pyrrolidonyl, 2,5-dioxopyrrolidinyl, 1,1-dioxotetrahydrothienyl, 2-oxazolidinonyl, 2-oxotetrahydrofuranyl, tetrahydrofuranyl, tetrahydropyranyl, 1,1-dioxothiomorpholino, 1,3-dioxolanyl, 2-oxoimidazolidinyl, 2,4-dioxoimidazolidinyl, pyranyl and 4-pyridonyl; and
R⁷ is (1-4C)alkyl;
or a salt or solvate thereof.

In a further aspect of the invention is provided a compound of the formula (I) as hereinbefore defined wherein
R¹ is methoxymethyl;
m is 1 and n is 0 or 1;
HET-1 is selected from pyridyl, pyrazinyl, pyridazinyl and pyrimidinyl;
R² is HET-2;
R³ is halo or trifluoromethyl;
R⁶ is selected from methyl, ethyl, bromo, chloro, fluoro, aminomethyl, N-methylaminomethyl, and dimethylaminomethyl;
HET-2 is selected from furyl, thienyl, thiazolyl, isothiazolyl, thiadiazolyl, pyridyl, pyrazinyl, pyridazinyl, pyrazolyl, imidazolyl, pyrimidinyl, oxazolyl, isoxazolyl, oxadiazolyl, pyrrolyl, 1,2,4-triazolyl and 1,2,3-triazolyl; and
R⁷ is (1-4C)alkyl;
or a salt or solvate thereof.

In a further aspect of the invention is provided a compound of the formula (I) as hereinbefore defined wherein
R¹ is methoxymethyl;
m is 0 or 1 and n is 0, 1 or 2;
HET-1 is selected from thiazolyl, pyrazolyl, N-methylpyrazol-3-yl, N-ethylpyrazol-3yl, 5-methylpyrazol-3-yl, 4-methylthiazol-2-yl, 5-methylthiazol-2-yl, 5-methyl-1,3,4-thiadiazol-2-yl, 4-methyl-1,3,5-thiadiazol-2-yl, 4-hydroxymethylthiazol-2-yl, 4-methoxymethylthiazol-2-yl and 5-bromopyridin-2-yl;
R³ is selected from chloro, fluoro and trifluoromethyl;
R² is selected from azetidinylcarbonyl, methoxyethylaminocarbonyl, imidazolylmethylaminocarbonyl, N-methylpiperidin-4-ylaminocarbonyl, N-methylpiperazin-4-ylcarbonyl, dimethylaminocarbonyl, morpholinocarbonyl, pyrrolidinylcarbonyl, 7-azabicyclo[2.2.1]hept-7-ylcarbonyl, dimethylaminosulfonyl, morpholinosulfonyl, isopropylaminosulfonyl, aminosulfonyl, N-methylpiperazin-4-ylsulfonyl, methoxyethylaminosulfonyl, cyano, ethylsulfonyl, methylsulfonyl, methylthio, methylsulfinyl, isopropylthio and isopropylsulfonyl;
or a salt or solvate thereof.

In a further aspect of the invention is provided a compound of the formula (I) as hereinbefore defined wherein
R¹ is methoxymethyl;
m is 0 or 1 and n is 0, 1 or 2;
HET-1 is selected from thiazolyl, pyrazolyl, N-methylpyrazol-3-yl, N-ethylpyrazol-3yl, 5-methylpyrazol-3-yl, 4-methylthiazol-2-yl, 5-methylthiazol-2-yl, 5-methyl-1,3,4-thiadiazol-2-yl, 4-methyl-1,3,5-thiadiazol-2-yl, 4-hydroxymethylthiazol-2-yl, 4-methoxymethylthiazol-2-yi and 5-bromopyridin-2-yl;
R³ is selected from chloro, fluoro, methoxy and trifluoromethyl;
R² is selected from azetidinylcarbonyl, methoxyethylaminocarbonyl, imidazolylmethylaminocarbonyl, N-methylpiperidin-4-ylaminocarbonyl, N-methylpiperazin-4-ylcarbonyl, dimethylaminocarbonyl, morpholinocarbonyl, pyrrolidinylcarbonyl, 7-azabicyclo[2.2.1]hept-7-ylcarbonyl, dimethylaminosulfonyl, morpholinosulfonyl, isopropylaminosulfonyl, aminosulfonyl, N-methylpiperazin-4-ylsulfonyl, methoxyethylaminosulfonyl, cyano, ethylsulfonyl, methylsulfonyl, methylthio, methylsulfinyl, isopropylthio and isopropylsulfonyl;
or a salt or solvate thereof.

Further preferred compounds of the invention are each of the Examples, each of which provides a further independent aspect of the invention. In further aspects, the present invention also comprises any two or more compounds of the Examples.

In one aspect, particular compounds of the invention comprise any one or more of:
3-(4-{[(2-methoxyethyl)amino]carbonyl}phenoxy)-5-[(1S)-2-methoxy-(1-methylethyl)oxy]-N-1,3-thiazol-2-ylbenzamide;
3-(4- {[(1H-imidazol-2-ylmethyl)amino]carbonyl}phenoxy)-5-[(1S)-2-methoxy-(1-methylethyl)oxy]-N-1,3-thiazol-2-ylbenzamide;
3-[4-(azetidin-1-ylcarbonyl)phenoxy]-5-[(1S)-2-methoxy-(1-methylethyl)oxy]-N-1,3-thiazol-2-ylbenzamide;
3-[(1S)-2-methoxy-(1-methylethyl)oxy]-5-{4-[(4-methylpiperazin-1-yl)carbonyl]phenoxy}-N-1,3-thiazol-2-ylbenzamide;
3-(3-{[(2-methoxyethyl)amino]carbonyl}phenoxy)-5-[(1S)-2-methoxy-(1-methylethyl)oxy]-N-1,3-thiazol-2-ylbenzamide;
3-(3-{[(1H-imidazol-2-ylmethyl)amino]carbonyl}phenoxy)-5-[(1S)-2-methoxy-(1-methylethyl)oxy]-N-1,3-thiazol-2-ylbenzamide;
3-[(1S)-2-methoxy-(1-methylethyl)oxy]-5-{4-[(4-methylpiperazin-1-yl)carbonyl]phenoxy}-N-(1-methyl-1H-pyrazol-3-yl)benzamide;
3-[(1S)-2-methoxy-(1-methylethyl)oxy]-N-(1-methyl-1H-pyrazol-3-yl)-5-[4-(morpholin-4-ylcarbonyl)phenoxy]benzamide;
3-[4-(azetidin-1-ylcarbonyl)phenoxy]-5-[(1S)-2-methoxy-(1-methylethyl)oxy]-N-(1-methyl-1 H-pyrazol-3-yl)benzamide;
3-[(S)-2-methoxy-(1-methylethyl)oxy]-N-(1-methyl-1H-pyrazol-3-yl)-5-[4-(pyrrolidin-1-ylcarbonyl)phenoxy]benzamide;
3-[4-(7-azabicyclo[2.2.1]hept-7-ylcarbonyl)phenoxy]-5-[(1S)-2-methoxy-(1-methylethyl)oxy]-N-(1-methyl-1H-pyrazol-3-yl)benzamide;
3-{2-chloro-4-[(dimethylamino)sulfonyl]phenoxy}-5-[(1S)-2-methoxy-(1-methylethyl)oxy]-N-(1-methyl-1H-pyrazol-3-yl)benzamide;
3-[(2-chloro-4-{[(1-methylethyl)amino]sulfonyl}phenyl)oxy]-5-[(1S)-2-methoxy-(1-methylethyl)oxy]-N-(1-methyl-1H-pyrazol-3-yl)benzamide;
3-{[2-chloro-4-({[2-(methyloxy)ethyl]amino}sulfonyl)phenyl]oxy}-5-[(1S)-2-methoxy-(1-methylethyl)oxy]-N-(1-methyl-1H-pyrazol-3-yl)benzamide;
3-({2-chloro-4-[(4-methylpiperazin-1-yl)sulfonyl]phenyl}oxy)-5-[(1S)-2-methoxy-(1-methylethyl)oxy]-N-(1-methyl-1H-pyrazol-3-yl)benzamide;
3-{4-[(dimethylamino)sulfonyl]phenoxy}-5-[(1S)-2-methoxy-(1-methylethyl)oxy]-N-(1-methyl-1H-pyrazol-3-yl)benzamide;
3-[(1S)-2-methoxy-(1-methylethyl)oxy]-5-({4-[(4-methylpiperazin-1-yl)sulfonyl]phenyl}oxy)-N-(1-methyl-1H-pyrazol-3-yl)benzamide;
3-{4-[((1-methylethyl)amino)sulfonyl]phenoxy}-5-[(1S)-2-methoxy-(1-methylethyl)oxy]-N-(1-methyl-1H-pyrazol-3-yl)benzamide;
3-(4-{[(2-methoxyethyl)amino]sulfonyl}phenoxy)-5-[(1S)-2-methoxy-(1-methylethyl)oxy]-N-(1-methyl-1H-pyrazol-3-yl)benzamide;
3-(4-cyanophenoxy)-5-[(1S)-2-methoxy-(1-methyl ethyl)oxy]-N-(1-methyl-1H-pyrazol-3-yl)benzamide;
3-{[4-(aminocarbonyl)phenyl]oxy}-5-[(1S)-2-methoxy-(1-methylethyl)oxy]-N-(1-methyl-1H-pyrazol-3-yl)benzamide;
3-[4-(ethylsulfonyl)phenoxy]-5-[(1S)-2-methoxy-(1-methylethyl)oxy]-N-1,3-thiazol-2-ylbenzamide;
3-[(1S)-2-methoxy-(1-methylethyl)oxyl-N-(1-methyl-1H-pyrazol-3-yl)-5-{[3-(methylthio)phenyl]oxy}benzamide;
3-({4-[(1-methylethyl)thio]phenyl}oxy)-5-[(1S)-2-methoxy-(1-methylethyl)oxy]-N-(1-methyl-1H-pyrazol-3-yl)benzamide;
3-[(1S)-2-methoxy-(1-methylethyl)oxy]-N-(1-methyl-1H-pyrazol-3-yl)-5-[3-(methylsulfonyl)phenoxy]benzamide;
3-[(1S)-2-methoxy-(1-methylethyl)oxy]-N-(1-methyl-1H-pyrazol-3-yl)-5-[3-(methylsulfinyl)phenoxy]benzamide;
3-({4-[(1-methylethyl)sulfonyl]phenyl}oxy)-5-[(1S)-2-methoxy-(1-methylethyl)oxy]-N-(1-methyl-1H-pyrazol-3-yl)benzamide;
3-[(1S)-2-methoxy-(1-methylethyl)oxy]-N-(1-methyl-1H-pyrazol-3-yl)-5-[4-(methylsulfonyl)phenoxy]benzamide;
3-[(1S)-2-methoxy-(1-methylethyl)oxy]-5-[4-(methylsulfonyl)phenoxy]-N-(4-methyl-1,3-thiazol-2-yl)benzamide;
3-[(1S)-2-methoxy-(1-methylethyl)oxy]-5-[4-(methylsulfonyl)phenoxy]-N-(5-methyl-1,3-thiazol-2-yl)benzamide;
3-[(1S)-2-methoxy-(1-methylethyl)oxy]-5-[4-(methylsulfonyl)phenoxy]-N-(5-methyl-1,3,4-thiadiazol-2-yl)benzamide;
3-[(1S)-2-methoxy-(1-methylethyl)oxy]-5-[4-(methylsulfonyl)phenoxy]-N-(3-methyl-1,2,4-thiadiazol-5-yl)benzamide;
N-(1-ethyl-1H-pyrazol-3-yl)-3-[(1S)-2-methoxy-(1-methylethyl)oxy]-5-[4-(methylsulfonyl)phenoxy]benzamide;
3-(3,5-difluorophenoxy)-5-[(1S)-2-methoxy-(1-methylethyl)oxy]-N-(1-methyl-1H-pyrazol-3-yl)benzamide;
N-(5-bromopyridin-2-yl)-3-(3,5-difluorophenoxy)-5-[(1S)-2-methoxy-(1-methylethyl)oxy]benzamide;
3-(3,5-difluorophenoxy)-N-[4-(hydroxymethyl)-1,3-thiazol-2-yl]-5-[(1S)-2-methoxy-(1-methylethyl)oxy]benzamide;
3-[(1S)-2-methoxy-(1-methylethyl)oxy]-N-(5-methyl-1H-pyrazol-3-yl)-5-[4-(methylsulfonyl)phenoxy]benzamide;
3-[(1S)-2-methoxy-(1-methylethyl)oxy]-N-[4-(methoxymethyl)-1,3-thiazol-2-yl]-5-[4-(methylsulfonyl)phenoxy]benzamide;
3-[4-(azetidin-1-ylcarbonyl)phenoxy]-5-[(1S)-2-methoxy-(1-methylethyl)oxy]-N-(3-methyl-1,2,4-thiadiazol-5-yl)benzamide ;
3-[(1S)-2-methoxy-(1-methylethyl)oxy]-5-(4-{[(1-methylpiperidin-4-yl)amino]carbonyl}phenoxy)-N-(3-methyl-1,2,4-thiadiazol-5-yl)benzamide;
3-[4-(azetidin-1-ylcarbonyl)-2-chlorophenoxy]-5-[(1S)-2-methoxy-(1-methylethyl)oxy]-N-(1-methyl-1H-pyrazol-3-yl)benzamide;
3-[4-(azetidin-1-ylcarbonyl)-2-fluorophenoxy]-5-[(1S)-2-methoxy-(1-methylethyl)oxy]-N-(1-methyl-1H-pyrazol-3-yl)benzamide;
3-[4-(azetidin-1-ylcarbonyl)-2-(trifluoromethyl)phenoxy]-5-[(1S)-2-methoxy-(1-methylethyl)oxy]-N-(1-methyl-1H-pyrazol-3-yl)bezamide; and
3-{4-[(dimethylamino)carbonyl]phenoxy}-5-[(1S)-2-methoxy-(1-methylethyl)oxy]-N-(1-methyl-1H-pyrazol-3-yl)benzamide; and/or is selected from 3-[4-(azetidin-1-ylcarbonyl)phenoxy]-5-[(1S)-2-methoxy-1-methylethoxy]-N-1H-pyrazol-3-ylbenzamide;
3-[4-(azetidin-1-ylcarbonyl)-2-fluorophenoxy]-5-[(1S)-2-methoxy-1-methylethoxy]-N-(5-methyl-1H-pyrazol-3-yl)benzamide;
3-[4-(azetidin-1-ylcarbonyl)-2-fluorophenoxy]-5-[(1S)-2-methoxy-1-methylethoxy]-N-1H-pyrazol-3-ylbenzamide;
3-[4-(azetidin-1-ylcarbonyl)-2-chlorophenoxy]-5-[(1S)-2-methoxy-1-methylethoxy]-N-(5-methyl-1H-pyrazol-3-yl)benzamide;
3-[4-(azetidin-1-ylcarbonyl)phenoxy]-5-[(1S)-2-methoxy-1-methylethoxy]-N-(5-methyl-1H-pyrazol-3-yl)benzamide; and
3-[(1S)-2-methoxy-1-methylethoxy]-N-(1-methyl-1H-pyrazol-3-yl)-5-[4-(1,2,4-oxadiazol-3-yl)phenoxy]benzamide;
or a salt or solvate thereof.

In another aspect, particular compounds of the invention comprise any one or more of: 3-(4-{[(2-methoxyethyl)amino]carbonyl}phenoxy)-5-[(1S)-2-methoxy-(1-methylethyl)oxy]-N-1,3-thiazol-2-ylbenzamide;
3-(4-{[(1H-imidazol-2-ylmethyl)amino]carbonyl}phenoxy)-5-[(1S)-2-methoxy-(1-methylethyl)oxy]-N-1,3-thiazol-2-ylbenzamide;
3-(3-{[(2-methoxyethyl)amino]carbonyl}phenoxy)-5-[(1S)-2-methoxy-(1-methylethyl)oxy]-N-1,3-thiazol-2-ylbenzamide;
3-(3-{[(1H-imidazol-2-ylmethyl)amino]carbonyl}phenoxy)-5-[(1S)-2-methoxy-(1-methylethyl)oxy]-N-1,3-thiazol-2-ylbenzamide;
3-{[2-chloro-4-({[2-(methyloxy)ethyl]amino}sulfonyl)phenyl]oxy}-5-[(1S)-2-methoxy-(1-methylethyl)oxy]-N-(1-methyl-1H-pyrazol-3-yl)benzamide;
3-(4-{[(2-methoxyethyl)amino]sulfonyl)phenoxy)-5-[(1S)-2-methoxy-(1-methylethyl)oxy]-N-(1-methyl-1H-pyrazol-3-yl)benzamide;
3-[(1S)-2-methoxy-(1-methylethyl)oxy]-5-(4-{[(1-methylpiperidin-4-yl)amino]carbonyl}phenoxy)-N-(3-methyl-1,2,4-thiadiazol-5-yl)benzamide;
or a salt or solvate thereof.

In another aspect, particular compounds of the invention comprise any one or more of:
3-[4-(azetidin-1-ylcarbonyl)phenoxy]-5-[(1S)-2-methoxy-(1-methylethyl)oxy]-N-1,3-thiazol-2-ylbenzamide;
3-[(1S)-2-methoxy-(1-methylethyl)oxy]-5-{4-[(4-methylpiperazin-1-yl)carbonyl]phenoxy}-N-1,3-thiazol-2-ylbenzamide;
3-[(1S)-2-methoxy-(1-methylethyl)oxy]-5-{4-[(4-methylpiperazin-1-yl)carbonyl]phenoxy}-N-(1-methyl-1H-pyrazol-3-yl)benzamide;
3-[(1S)-2-methoxy-(1-methylethyl)oxy]-N-(1-methyl-1H-pyrazol-3-yl)-5-[4-(morpholin-4-ylcarbonyl)phenoxy]benzamide;
3-[4-(azetidin-1-ylcarbonyl)phenoxy]-5-[(1S)-2-methoxy-(1-methylethyl)oxy]-N-(1-methyl-1H-pyrazol-3-yl)benzamide;
3-[(1S)-2-methoxy-(1-methylethyl)oxy]-N-(1-methyl-1H-pyrazol-3-yl)-5-[4-(pyrrolidin-1-ylcarbonyl)phenoxy]benzamide;
3-[4-(7-azabicyclo[2.2.1]hept-7-ylcarbonyl)phenoxy]-5-[(1S)-2-methoxy-(1-methylethyl)oxy]-N-(1-methyl-1H-pyrazol-3-yl)benzamide;
3-({2-chloro-4-[(4-methylpiperazin-1-yl)sulfonyl]phenyl}oxy)-5-[(1S)-2-methoxy-(1-methylethyl)oxy]-N-(1-methyl-1H-pyrazol-3-yl)benzamide;
3-[(1S)-2-methoxy-(1-methylethyl)oxy]-5-({4-[(4-methylpiperazin-1-yl)sulfonyl]phenyl}oxy)-N-(1-methyl-1H-pyrazol-3-yl)benzamide;
3-[4-(azetidin-1-ylcarbonyl)phenoxy]-5-[(1S)-2-methoxy-(1-methylethyl)oxy]-N-(3-methyl-1,2,4-thiadiazol-5-yl)benzamide;
3-[4-(azetidin-1-ylcarbonyl)-2-chlorophenoxy]-5-[(1S)-2-methoxy-(1-methylethyl)oxy]-N-(1-methyl-1H-pyrazol-3-yl)benzamide;
3-[4-(azetidin-1-ylcarbonyl)-2-fluorophenoxy]-5-[(1S)-2-methoxy-(1-methylethyl)oxy]-N-(1-methyl-1H-pyrazol-3-yl)benzamide;
3-[4-(azetidin-1-ylcarbonyl)-2-(trifluoromethyl)phenoxy]-5-[(1S)-2-methoxy-(1-methylethyl)oxy]-N-(1-methyl-1H-pyrazol-3-yl)benzamide;
3-[4-(azetidin-1-ylcarbonyl)phenoxy]-5-[(1S)-2-methoxy-1-methylethoxy]-N-1H-pyrazol-3-ylbenzamide;
3-[4-(azetidin-1-ylcarbonyl)-2-chlorophenoxy]-5-[(1S)-2-methoxy-1-methylethoxy]-N-(5-methyl-1H-pyrazol-3-yl)benzamide;
3-[4-(azetidin-1-ylcarbonyl)-2-fluorophenoxy]-5-[(1S)-2-methoxy-1-methylethoxy]-N-(5-methyl-1H-pyrazol-3-yl)benzamide;
3-[4-(azetidin-1-ylcarbonyl)-2-fluorophenoxy]-5-[(1S)-2-methoxy-1-methylethoxy]-N-1H-pyrazol-3-ylbenzamide;
3-[4-(azetidin-1-ylcarbonyl)phenoxy]-5-[(1S)-2-methoxy-1-methylethoxy]-N-(5-methyl-1H-pyrazol-3-yl)benzamide;
or a salt or solvate thereof.

In another aspect, particular compounds of the invention comprise any one or more of:
3-[4-(azetidin-1-ylcarbonyl)phenoxy]-5-[(1S)-2-methoxy-(1-methylethyl)oxy]-N-1,3-thiazol-2-ylbenzamide;
3-[4-(azetidin-1-ylcarbonyl)phenoxy]-5-[(1S)-2-methoxy-(1-methylethyl)oxy]-N-(1-methyl-1H-pyrazol-3-yl)benzamide;
3-[(1S)-2-methoxy-(1-methylethyl)oxy]-N-(1-methyl-1H-pyrazol-3-yl)-5-[4-(pyrrolidin-1-ylcarbonyl)phenoxy]benzamide;
3-[4-(azetidin-1-ylcarbonyl)phenoxy]-5-[(1S)-2-methoxy-(1-methylethyl)oxy]-N-(3-methyl-1,2,4-thiadiazol-5-yl)benzamide;
3-[4-(azetidin-1-ylcarbonyl)-2-chlorophenoxy]-5-[(1S)-2-methoxy-(1-methylethyl)oxy]-N-(1-methyl-1H-pyrazol-3-yl)benzamide;
3-[4-(azetidin-1-ylcarbonyl)-2-fluorophenoxy]-5-[(1S)-2-methoxy-(1-methylethyl)oxy]-N-(1-methyl-1H-pyrazol-3-yl)benzamide;
3-[4-(azetidin-1-ylcarbonyl)-2-(trifluoromethyl)phenoxy]-5-[(1S)-2-methoxy-(1-methylethyl)oxy]-N-(1-methyl-1H-pyrazol-3-yl)benzamide;
3-[4-(azetidin-1-ylcarbonyl)phenoxy]-5-[(1S)-2-methoxy-1-methylethoxy]-N-1H-pyrazol-3-ylbenzamide;
3-[4-(azetidin-1-ylcarbonyl)-2-chlorophenoxy]-5-[(1S)-2-methoxy-1-methylethoxy]-N-(5-methyl-1H-pyrazol-3-yl)benzamide;
3-[4-(azetidin-1-ylcarbonyl)-2-fluorophenoxy]-5-[(1S)-2-methoxy-1-methylethoxy]-N-(5-methyl-1H-pyrazol-3-yl)benzamide;
3-[4-(azetidin-1-ylcarbonyl)-2-fluorophenoxy]-5-[(1S)-2-methoxy-1-methylethoxy]-N-1H-pyrazol-3-ylbenzamide;
3-[4-(azetidin-1-ylcarbonyl)phenoxy]-5-[(1S)-2-methoxy-1-methylethoxy]-N-(5-methyl-1H-pyrazol-3-yl)benzamide;
or a salt or solvate thereof.

In another aspect, particular compounds of the invention comprises
3-[(1S)-2-methoxy-1-methylethoxy]-N-(1-methyl-1H-pyrazol-3-yl)-5-[4-(1,2,4-oxadiazol-3-yl)phenoxy]benzamide;
or a salt or solvate thereof.

In another aspect, particular compounds of the invention comprise any one or more of
3-{2-chloro-4-[(dimethylamino)sulfonyl]phenoxy}-5-[(1S)-2-methoxy-(1-methylethyl)oxy]-N-(1-methyl-1H-pyrazol-3-yl)benzamide;
3-[(2-chloro-4-{[(1-methylethyl)amino]sulfonyl} phenyl)oxy]-5-[(1S)-2-methoxy-(1-methylethyl)oxy]-N-(1-methyl-1H-pyrazol-3-yl)benzamide;
3-{4-[(dimethylamino)sulfonyl]phenoxy}-5-[(1S)-2-methoxy-(1-methylethyl)oxy]-N-(1-methyl-1H-pyrazol-3-yl)benzamide;
3-{4-[((1-methylethyl)amino)sulfonyl]phenoxy}-5-[(1S)-2-methoxy-(1-methylethyl)oxy]-N-(1-methyl-1H-pyrazol-3-yl)benzamide;
3-(4-cyanophenoxy)-5-[(1S)-2-methoxy-(1-methylethyl)oxy]-N-(1-methyl-1H-pyrazol-3-yl)benzamide;
3-{[4-(aminocarbonyl)phenyl]oxy}-5-[(1S)-2-methoxy-(1-methylethyl)oxy]-N-(1-methyl-1H-pyrazol-3-yl)benzamide;
3-[4-(ethylsulfonyl)phenoxy]-5-[(1S)-2-methoxy-(1-methylethyl)oxy]-N-1,3-thiazol-2-ylbenzamide;
3-[(1S)-2-methoxy-(1-methylethyl)oxy]-N-(1-methyl-1H-pyrazol-3-yl)-5-{[3-(methylthio)phenyl]oxy}benzamide;
3-({4-[(1-methylethyl)thio]phenyl}oxy)-5-[(1S)-2-methoxy-(1-methylethyl)oxy]-N-(1-methyl-1 H-pyrazol-3-yl)benzamide;
3-[(1S)-2-methoxy-(1-methylethyl)oxy]-N-(1-methyl-1H-pyrazol-3-yl)-5-[3-(methylsulfonyl)phenoxy]benzamide;
3-[(1S)-2-methoxy-(1-methylethyl)oxy]-N-(1-methyl-1H-pyrazol-3-yl)-5-[3-(methylsulfinyl)phenoxy]benzamide;
3-({4-[(1-methylethyl)sulfonyl]phenyl}oxy)-5-[(1S)-2-methoxy-(1-methylethyl)oxy]-N-(1-methyl-1H-pyrazol-3-yl)benzamide;
3-[(1S)-2-methoxy-(1-methylethyl)oxy]-N-(1-methyl-1H-pyrazol-3-yl)-5-[4-(methylsulfonyl)phenoxy]benzamide;
3-[(1S)-2-methoxy-(1-methylethyl)oxy]-5-[4-(methylsulfonyl)phenoxy]-N-(4-methyl-1,3-thiazol-2-yl)benzamide;
3-[(1S)-2-methoxy-(1-methylethyl)oxy]-5-[4-(methylsulfonyl)phenoxy]-N-(5-methyl-1,3-thiazol-2-yl)benzamide;
3-[(1S)-2-methoxy-(1-methylethyl)oxy]-5-[4-(methylsulfonyl)phenoxy]-N-(5-methyl-1,3,4-thiadiazol-2-yl)benzamide;
3-[(1S)-2-methoxy-(1-methylethyl)oxy]-5-[4-(methylsulfonyl)phenoxy]-N-(3-methyl-1,2,4-thiadiazol-5-yl)benzamide;
N-(1-ethyl-1H-pyrazol-3-yl)-3-[(1S)-2-methoxy-(1-methylethyl)oxy]-5-[4-(methylsulfonyl)phenoxy]benzamide;
3-(3,5-difluorophenoxy)-5-[(1S)-2-methoxy-(1-methylethyl)oxy]-N-(1-methyl-1H-pyrazol-3-yl)benzamide;
N-(5-bromopyridin-2-yl)-3-(3,5-difluorophenoxy)-5-[(1S)-2-methoxy-(1-methylethyl)oxy]benzamide;
3-(3,5-difluorophenoxy)-N-[4-(hydroxymethyl)-1,3-thiazol-2-yl]-5-[(1S)-2-methoxy-(1-methylethyl)oxy]benzamide;
3-[(1S)-2-methoxy-(1-methylethyl)oxy]-N-(5-methyl-1H-pyrazol-3-yl)-5-[4-(methylsulfonyl)phenoxy]benzamide;
3-[(1S)-2-methoxy-(1-methylethyl)oxy]-N-[4-(methoxymethyl)-1,3-thiazol-2-yl]-5-[4-(methylsulfonyl)phenoxy]benzamide;
3-{4-[(dimethylamino)carbonyl]phenoxy}-5-[(1S)-2-methoxy-(1-methylethyl)oxy]-N-(1-methyl-1H-pyrazol-3-yl)benzamide;
or a salt or solvate thereof.

The compounds of the invention may be administered in the form of a pro-drug. A pro-drug is a bioprecursor or pharmaceutically acceptable compound being degradable in the body to produce a compound of the invention (such as an ester or amide of a compound of the invention, particularly an in-vivo hydrolysable ester). Various forms of prodrugs are known in the art. For examples of such prodrug derivatives, see:
a) Design of Prodrugs, edited by H. Bundgaard, (Elsevier, 1985) and Methods in Enzymology, Vol. 42, p. 309-396, edited by K. Widder, et al. (Academic Press, 1985);
b) A Textbook of Drug Design and Development, edited by Krogsgaard-Larsen;
c) H. Bundgaard, Chapter 5 "Design and Application of Prodrugs", by H. Bundgaard p. 113-191 (1991);
d) H. Bundgaard, Advanced Drug Delivery Reviews, 8, 1-38 (1992);
e) H. Bundgaard, et al., Journal of Pharmaceutical Sciences, 77, 285 (1988); and
f) N. Kakeya, et al., Chem Pharm Bull, 32, 692 (1984).
The contents of the above cited documents are incorporated herein by reference.

Examples of pro-drugs are as follows. An in-vivo hydrolysable ester of a compound of the invention containing a carboxy or a hydroxy group is, for example, a pharmaceutically-acceptable ester which is hydrolysed in the human or animal body to produce the parent acid or alcohol. Suitable pharmaceutically-acceptable esters for carboxy include C₁ to C₆alkoxymethyl esters for example methoxymethyl, C₁ to C₆alkanoyloxymethyl esters for example pivaloyloxymethyl, phthalidyl esters, C₃ to C₈cycloalkoxycarbonyloxyC₁ to C₆alkyl esters for example 1-cyclohexylcarbonyloxyethyl; 1,3-dioxolen-2-onylmethyl esters, for example 5-methyl-1,3-dioxolen-2-onylmethyl; and C₁₋₆alkoxycarbonyloxyethyl esters.

An in-vivo hydrolysable ester of a compound of the invention containing a hydroxy group includes inorganic esters such as phosphate esters (including phosphoramidic cyclic esters) and α-acyloxyalkyl ethers and related compounds which as a result of the in-vivo hydrolysis of the ester breakdown to give the parent hydroxy group/s. Examples of α-acyloxyalkyl ethers include acetoxymethoxy and 2,2-dimethylpropionyloxy-methoxy. A selection of in-vivo hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl.

A suitable pharmaceutically-acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitable pharmaceutically-acceptable salt of a benzoxazinone derivative of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

A further feature of the invention is a pharmaceutical composition comprising a compound of Formula (I) as defined above, or a salt, solvate or prodrug thereof, together with a pharmaceutically-acceptable diluent or carrier.

According to another aspect of the invention there is provided a compound of Formula (1) as defined above for use as a medicament.

Further according to the invention there is provided a compound of Formula (I) for use in the preparation of a medicament for treatment of a disease mediated through GLK, in particular type 2 diabetes.

The compound is suitably formulated as a pharmaceutical composition for use in this way.

According to another aspect there is provided a method of treating GLK mediated diseases, especially diabetes, by administering an effective amount of a compound of Formula (I) or salt or solvate thereof, to a mammal in need of such treatment.

Specific diseases which may be treated by a compound or composition of the invention include: blood glucose lowering in type 2 Diabetes Mellitus without a serious risk of hypoglycaemia (and potential to treat type 1), dyslipidemia, obesity, insulin resistance, metabolic syndrome X, impaired glucose tolerance.

As discussed above, thus the GLK/GLKRP system can be described as a potential "Diabesity" target (of benefit in both Diabetes and Obesity). Thus, according to another aspect of the invention there is provided the use of a compound of Formula (I) or salt or solvate thereof, in the preparation of a medicament for use in the combined treatment or prevention of diabetes and obesity.

According to another aspect of the invention there if provided the use of a compound of Formula (I) or salt or solvate thereof, in the preparation of a medicament for use in the treatment or prevention of obesity.

According to a further aspect there is provided a method for the combined treatment of obesity and diabetes by administering an effective amount of a compound of Formula (I) or salt or solvate thereof, to a mammal in need of such treatment.

According to a further aspect there is provided a method for the treatment of obesity by administering an effective amount of a compound of Formula (I) or salt or solvate thereof, to a mammal in need of such treatment.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing). Dosage forms suitable for oral use are preferred.

The compositions of the invention may be obtained by conventional procedures using conventional pharmaceutical excipients, well known in the art. Thus, compositions intended for oral use may contain, for example, one or more colouring, sweetening, flavouring and/or preservative agents.

Suitable pharmaceutically acceptable excipients for a tablet formulation include, for example, inert diluents such as lactose, sodium carbonate, calcium phosphate or calcium carbonate, granulating and disintegrating agents such as corn starch or algenic acid; binding agents such as starch; lubricating agents such as magnesium stearate, stearic acid or talc; preservative agents such as ethyl or propyl p-hydroxybenzoate, and anti-oxidants, such as ascorbic acid. Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Compositions for oral use may be in the form of hard gelatin capsules in which the active ingredient is mixed with an inert solid diluent, for example, calcium carbonate, calcium phosphate or kaolin, or as soft gelatin capsules in which the active ingredient is mixed with water or an oil such as peanut oil, liquid paraffin, or olive oil.

Aqueous suspensions generally contain the active ingredient in finely powdered form together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives (such as ethyl or propyl p-hydroxybenzoate, anti-oxidants (such as ascorbic acid), colouring agents, flavouring agents, and/or sweetening agents (such as sucrose, saccharine or aspartame).

Oily suspensions may be formulated by suspending the active ingredient in a vegetable oil (such as arachis oil, olive oil, sesame oil or coconut oil) or in a mineral oil (such as liquid paraffin). The oily suspensions may also contain a thickening agent such as beeswax, hard paraffin or cetyl alcohol. Sweetening agents such as those set out above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavouring and colouring agents, may also be present.

The pharmaceutical compositions of the invention may also be in the form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurring gums such as gum acacia or gum tragacanth, naturally-occurring phosphatides such as soya bean, lecithin, an esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavouring and preservative agents.

Syrups and elixirs may be formulated with sweetening agents such as glycerol, propylene glycol, sorbitol, aspartame or sucrose, and may also contain a demulcent, preservative, flavouring and/or colouring agent.

The pharmaceutical compositions may also be in the form of a sterile injectable aqueous or oily suspension, which may be formulated according to known procedures using one or more of the appropriate dispersing or wetting agents and suspending agents, which have been mentioned above. A sterile injectable preparation may also be a sterile injectable solution or suspension in a non-toxic parenterally-acceptable diluent or solvent, for example a solution in 1,3-butanediol.

Compositions for administration by inhalation may be in the form of a conventional pressurised aerosol arranged to dispense the active ingredient either as an aerosol containing finely divided solid or liquid droplets. Conventional aerosol propellants such as volatile fluorinated hydrocarbons or hydrocarbons may be used and the aerosol device is conveniently arranged to dispense a metered quantity of active ingredient.

For further information on formulation the reader is referred to Chapter 25.2 in Volume 5 of Comprehensive Medicinal Chemistry (Corwin Hansch; Chairman of Editorial Board), Pergamon Press 1990.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 2 g of active agent compounded with an appropriate and convenient amount of excipients which may vary from about 5 to about 98 percent by weight of the total composition. Dosage unit forms will generally contain about 1 mg to about 500 mg of an active ingredient. For further information on Routes of Administration and Dosage Regimes the reader is referred to Chapter 25.3 in Volume 5 of Comprehensive Medicinal Chemistry (Corwin Hansch; Chairman of Editorial Board), Pergamon Press 1990.

The size of the dose for therapeutic or prophylactic purposes of a compound of the Formula (I) will naturally vary according to the nature and severity of the conditions, the age and sex of the animal or patient and the route of administration, according to well known principles of medicine.

In using a compound of the Formula (I) for therapeutic or prophylactic purposes it will generally be administered so that a daily dose in the range, for example, 0.5 mg to 75 mg per kg body weight is received, given if required in divided doses. In general lower doses will be administered when a parenteral route is employed. Thus, for example, for intravenous administration, a dose in the range, for example, 0.5 mg to 30 mg per kg body weight will generally be used. Similarly, for administration by inhalation, a dose in the range, for example, 0.5 mg to 25 mg per kg body weight will be used. Oral administration is however preferred.

The elevation of GLK activity described herein may be applied as a sole therapy or in combination with one or more other substances and/or treatments for the indication being treated. Such conjoint treatment may be achieved by way of the simultaneous, sequential or separate administration of the individual components of the treatment. Simultaneous treatment may be in a single tablet or in separate tablets. For example in the treatment of diabetes mellitus, chemotherapy may include the following main categories of treatment:
1) Insulin and insulin analogues;
2) Insulin secretagogues including sulphonylureas (for example glibenclamide, glipizide), prandial glucose regulators (for example repaglinide, nateglinide) ;
3) Agents that improve incretin action (for example dipeptidyl peptidase IV inhibitors, and GLP-1 agonists);
4) Insulin sensitising agents including PPARgamma agonists (for example pioglitazone and rosiglitazone), and agents with combined PPARalpha and gamma activity;
5) Agents that modulate hepatic glucose balance (for example metformin, fructose 1,6 bisphosphatase inhibitors, glycogen phopsphorylase inhibitors, glycogen synthase kinase inhibitors);
6) Agents designed to reduce the absorption of glucose from the intestine (for example acarbose);
7) Agents that prevent the reabsorption of glucose by the kidney (SGLT inhibitors);
8) Agents designed to treat the complications of prolonged hyperglycaemia (for example aldose reductase inhibitors);
9) Anti-obesity agents (for example sibutramine and orlistat);
10) Anti- dyslipidaemia agents such as, HMG-CoA reductase inhibitors (eg statins); PPARα agonists (fibrates, eg gemfibrozil); bile acid sequestrants (cholestyramine); cholesterol absorption inhibitors (plant stanols, synthetic inhibitors); bile acid absorption inhibitors (IBATi) and nicotinic acid and analogues (niacin and slow release formulations);
11) Antihypertensive agents such as, β blockers (eg atenolol, inderal); ACE inhibitors (eg lisinopril); Calcium antagonists (eg. nifedipine); Angiotensin receptor antagonists (eg candesartan), α antagonists and diuretic agents (eg. furosemide, benzthiazide);
12) Haemostasis modulators such as, antithrombotics, activators of fibrinolysis and antiplatelet agents; thrombin antagonists; factor Xa inhibitors; factor VIIa inhibitors); antiplatelet agents (eg. aspirin, clopidogrel); anticoagulants (heparin and Low molecular weight analogues, hirudin) and warfarin;
13) Agents which antagonise the actions of glucagon; and
14) Anti-inflammatory agents, such as non-steroidal anti-inflammatory drugs (eg. aspirin) and steroidal anti-inflammatory agents (eg. cortisone).

According to another aspect of the present invention there is provided individual compounds produced as end products in the Examples set out below and salts or solvates thereof.

A compound of the invention, or a salt thereof, may be prepared by any process known to be applicable to the preparation of such compounds or structurally related compounds. Functional groups may be protected and deprotected using conventional methods. For examples of protecting groups such as amino and carboxylic acid protecting groups (as well as means of formation and eventual deprotection), see T.W. Greene and P.G.M. Wuts, "Protective Groups in Organic Synthesis", Second Edition, John Wiley & Sons, New York, 1991.

Processes for the synthesis of compounds of Formula (I) are provided as a further feature of the invention. Thus, according to a further aspect of the invention there is provided a process for the preparation of a compound of Formula (I), which comprises a process a) to d) (wherein the variables are as defined hereinbefore for compounds of Formula (I) unless otherwise defined):
(a) reaction of an acid of Formula (III) or activated derivative thereof with a compound of Formula (IV), 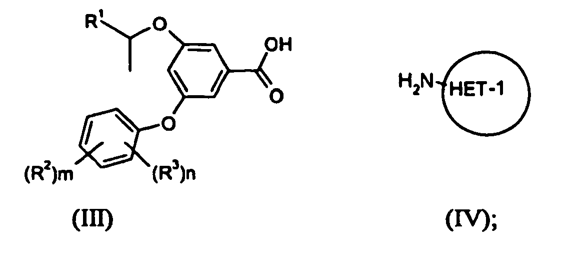 or
(b) reaction of a compound of Formula (V) with a compound of Formula (VI), 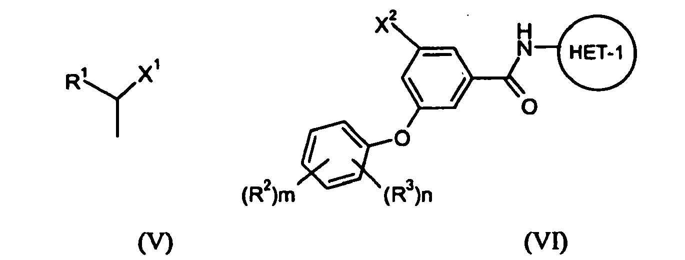 wherein X¹ is a leaving group and X² is a hydroxyl group or X¹ is a hydroxyl group and X² is a leaving group;
   process (b) could also be accomplished using the intermediate ester Formula (VII), wherein P¹ is a protecting group as hereinafter described, followed by ester hydrolysis and amide formation by procedures described elsewhere and well known to those skilled in the art; 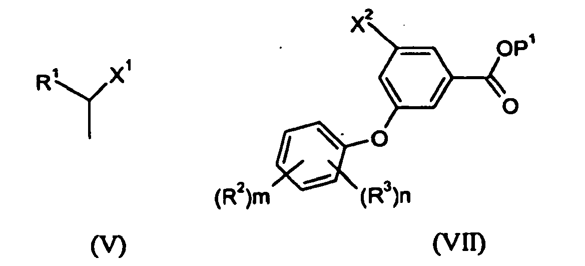 or
(c) reaction of a compound of Formula (VIII) with a compound of Formula (IX) 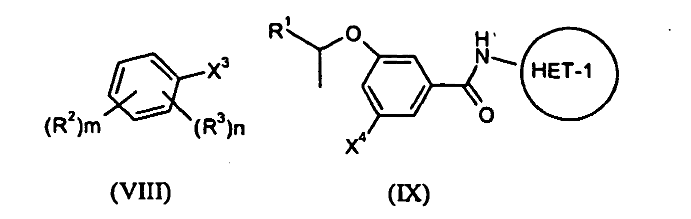 wherein X³ is a leaving group or an organometallic reagent and X⁴ is a hydroxyl group or X³ is a hydroxyl group and X⁴ is a leaving group or an organometallic reagent;
   process (c) could also be accomplished using the intermediate ester Formula (X), followed by ester hydrolysis and amide formation by procedures described elsewhere and well known to those skilled in the art; or 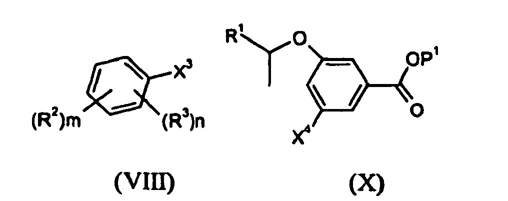
(d) reaction of a compound of Formula (XI) with a compound of Formula (XII), 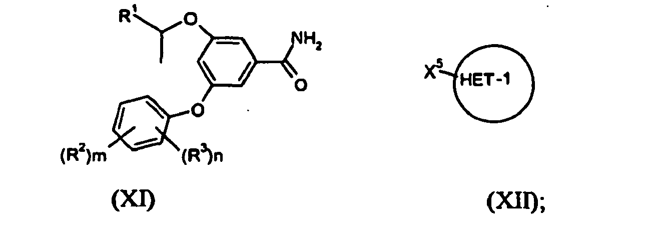 wherein X⁵ is a leaving group;
and thereafter, if necessary:
i) converting a compound of Formula (I) into another compound of Formula (I);
ii) removing any protecting groups; and/or
iii) forming a salt; or solvate thereof.

Suitable leaving groups X¹ to X⁵ for processes b) to d) are any leaving group known in the art for these types of reactions, for example halo, alkoxy, trifluoromethanesulfonyloxy, methanesulfonyloxy, or p-toulenesulfonyloxy; or a group (such as a hydroxy group) that may be converted into a leaving group (such as an oxytriphenylphosphonium group) *in situ.*

Compounds of Formulae (III) to (XII) are commercially available, or are known in the art, or may be made by processes known in the art as shown, for example, in the accompanying Examples. For further information on processes for making such compounds, we refer to our PCT publications WO 03/000267, WO 03/015774 and WO 03/000262 and references therein. In general it will be appreciated that any aryl-O or alkyl-O bond may be formed by nucleophilic substitution or metal catalysed processes, optionally in the presence of a suitable base.

Examples of conversions of a compound of Formula (I) into another compound of Formula (I), well known to those skilled in the art, include functional group interconversions such as hydrolysis, hydrogenation, hydrogenolysis, oxidation or reduction, and/or further functionalisation by standard reactions such as amide or metal-catalysed coupling, or nucleophilic displacement reactions;

Specific reaction conditions for the above reactions are as follows, wherein when P¹ is a protecting group P¹ is preferably C₁₋₄alkyl, for example methyl or ethyl:
*Process a)-* coupling reactions of amino groups with carboxylic acids to form an amide are well known in the art. For example,
   (i) using an appropriate coupling reaction, such as a carbodiimide coupling reaction performed with EDAC (1-(3-dimethylaminopropyl)-3-ethylcarbodiimide hydrochloride) in the presence of dimethylaminopyridine (DMAP) in a suitable solvent such as dichloromethane (DCM), chloroform or dimethylformamide (DMF) at room temperature; or
   (ii) reaction in which the carboxylic group is activated to an acid chloride by reaction with oxalyl chloride in the presence of a suitable solvent such as DCM. The acid chloride can then be reacted with a compound of Formula (IV) in the presence of a base, such as triethylamine or pyridine, in a suitable solvent such as chloroform or DCM at a temperature between 0°C and 80°C.
*Process b) -* compounds of Formula (V) and (VI) can be reacted together in a suitable solvent, such as DMF or tetrahydrofuran (THF), with a base such as sodium hydride or potassium *tert*-butoxide, at a temperature in the range 0 to 200°C, optionally using microwave heating or metal catalysis such as palladium(II)acetate, palladium on carbon, copper(II)acetate or copper(I)iodide; alternatively, compounds of Formula (V) and (VI) can be reacted together in a suitable solvent, such as THF or DCM, with a suitable phosphine such as triphenylphosphine, and azodicarboxylate such as diethylazodicarboxylate; process b) could also be carried out using a precursor to the ester of formula (VII) such as an aryl-nitrile or trifluoromethyl derivative, followed by conversion to a carboxylic acid and amide formation as previously described;
*Process c) -* compounds of Formula (VIII) and (IX) can be reacted together in a suitable solvent, such as DMF or THF, with a base such as sodium hydride or potassium tert-butoxide, at a temperature in the range 0 to 200°C, optionally using microwave heating or metal catalysis such as palladium(II)acetate, palladium on carbon, copper(II)acetate or copper(I)iodide; process c) could also be carried out using a precursor to the ester of formula (X) such as an aryl-nitrile or trifluoromethyl derivative, followed by conversion to a carboxylic acid and amide formation as previously described;
*Process d) -* reaction of a compound of Formula (XI) with a compound of Formula (XII) can be performed in a polar solvent, such as DMF or a non-polar solvent such as THF with a strong base, such as sodium hydride or potassium *tert*-butoxide at a temperature between 0 and 200°C, optionally using microwave heating or metal catalysis, such as palladium(II)acetate, palladium on carbon, copper(II)acetate or copper(I)iodide.

Certain intermediates of formula (III), (VI), (VII), (IX) and/or (XI) are believed to be novel and comprise an independent aspect of the invention.

Certain intermediates of formula (III), (IX) and/or (XI) wherein R¹ is methoxymethyl are believed to be novel and comprise an independent aspect of the invention.

During the preparation process, it may be advantageous to use a protecting group for a functional group within the molecule. Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower" signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned is of course within the scope of the invention.

A carboxy protecting group may be the residue of an ester-forming aliphatic or araliphatic alcohol or of an ester-forming silanol (the said alcohol or silanol preferably containing 1-20 carbon atoms). Examples of carboxy protecting groups include straight or branched chain (1-12C)alkyl groups (e.g. isopropyl, 1-butyl); lower alkoxy lower alkyl groups (e.g. methoxymethyl, ethoxymethyl, isobutoxymethyl; lower aliphatic acyloxy lower alkyl groups, (e.g. acetoxymethyl, propionyloxymethyl, butyryloxymethyl, pivaloyloxymethyl); lower alkoxycarbonyloxy lower alkyl groups (e.g. 1-methoxycarbonyloxyethyl, 1-ethoxycarbonyloxyethyl); aryl lower alkyl groups (e.g. p-methoxybenzyl, o-nitrobenzyl, p-nitrobenzyl, benzhydryl and phthalidyl); tri(lower alkyl)silyl groups (e.g. trimethylsilyl and t-butyldimethylsilyl); tri(lower alkyl)silyl lower alkyl groups (e.g. trimethylsilylethyl); and (2-6C)alkenyl groups (e.g. allyl and vinylethyl).

Methods particularly appropriate for the removal of carboxyl protecting groups include for example acid-, metal- or enzymically-catalysed hydrolysis.

Examples of hydroxy protecting groups include lower alkenyl groups (e.g. allyl); lower alkanoyl groups (e.g. acetyl); lower alkoxycarbonyl groups (e.g. t-butoxycarbonyl); lower alkenyloxycarbonyl groups (e.g. allyloxycarbonyl); aryl lower alkoxycarbonyl groups (e.g. benzoyloxycarbonyl, p-methoxybenzyloxycarbonyl, o-nitrobenzyloxycarbonyl, p-nitrobenzyloxycarbonyl); tri lower alkyl/arylsilyl groups (e.g. trimethylsilyl, t-butyldimethylsilyl, t-butyldiphenylsilyl); aryl lower alkyl groups (e.g. benzyl) groups; and triaryl lower alkyl groups (e.g. triphenylmethyl).

Examples of amino protecting groups include formyl, aralkyl groups (e.g. benzyl and substituted benzyl, e.g. p-methoxybenzyl, nitrobenzyl and 2,4-dimethoxybenzyl, and triphenylmethyl); di-p-anisylmethyl and furylmethyl groups; lower alkoxycarbonyl (e.g. t-butoxycarbonyl); lower alkenyloxycarbonyl (e.g. allyloxycarbonyl); aryl lower alkoxycarbonyl groups (e.g. benzyloxycarbonyl, p-methoxybenzyloxycarbonyl, o-nitrobenzyloxycarbonyl, p-nitrobenzyloxycarbonyl; trialkylsilyl (e.g. trimethylsilyl and t-butyldimethylsilyl); alkylidene (e.g. methylidene); benzylidene and substituted benzylidene groups.

Methods appropriate for removal of hydroxy and amino protecting groups include, for example, acid-, base, metal- or enzymically-catalysed hydrolysis, or photolytically for groups such as o-nitrobenzyloxycarbonyl, or with fluoride ions for silyl groups.

Examples of protecting groups for amide groups include aralkoxymethyl (e.g. benzyloxymethyl and substituted benzyloxymethyl); alkoxymethyl (e.g. methoxymethyl and trimethylsilylethoxymethyl); tri alkyl/arylsilyl (e.g. trimethylsilyl, t-butyldimethylsily, t-butyldiphenylsilyl); tri alkyl/arylsilyloxymethyl (e.g. 1-butyldimethylsilyloxymethyl, t-butyldiphenylsilyloxymethyl); 4-alkoxyphenyl (e.g. 4-methoxyphenyl); 2,4-di(alkoxy)phenyl (e.g. 2,4-dimethoxyphenyl); 4-alkoxybenzyl (e.g. 4-methoxybenzyl); 2,4-di(alkoxy)benzyl (e.g. 2,4-di(methoxy)benzyl); and alk-1-enyl (e.g. allyl, but-1-enyl and substituted vinyl e.g. 2-phenylvinyl).

Aralkoxymethyl, groups may be introduced onto the amide group by reacting the latter group with the appropriate aralkoxymethyl chloride, and removed by catalytic hydrogenation. Alkoxymethyl, tri alkyl/arylsilyl and tri alkyl/silyloxymethyl groups may be introduced by reacting the amide with the appropriate chloride and removing with acid; or in the case of the silyl containing groups, fluoride ions. The alkoxyphenyl and alkoxybenzyl groups are conveniently introduced by arylation or alkylation with an appropriate halide and removed by oxidation with ceric ammonium nitrate. Finally alk-1-enyl groups may be introduced by reacting the amide with the appropriate aldehyde and removed with acid.

The following examples are for illustration purposes and are not intended to limit the scope of this application. Each exemplified compound represents a particular and independent aspect of the invention. In the following non-limiting Examples, unless otherwise stated:
(i) evaporations were carried out by rotary evaporation in *vacuo* and work-up procedures were carried out after removal of residual solids such as drying agents by filtration;
(ii) operations were carried out at room temperature, that is in the range 18-25°C and under an atmosphere of an inert gas such as argon or nitrogen;
(iii) yields are given for illustration only and are not necessarily the maximum attainable;
(iv) the structures of the end-products of the Formula (I) were confirmed by nuclear (generally proton) magnetic resonance (NMR) with a field strength (for proton) of 300 or 400 MHz, and mass spectral techniques; proton magnetic resonance chemical shift values were measured on the delta scale and peak multiplicities are shown as follows: s, singlet; d, doublet; t, triplet; m, multiplet; br, broad; q, quartet, quin, quintet;
(v) intermediates were not generally fully characterised and purity was assessed by thin layer chromatography (TLC), high-performance liquid chromatography (HPLC), infra-red (IR) or NMR analysis; and
(vi) Biotage cartridges refer to pre-packed silica cartridges (from 40g up to 400g), eluted using a biotage pump and fraction collector system; Biotage UK Ltd, Hertford, Herts, UK.

### Abbreviations

- DCM: dichloromethane;
- DEAD: diethylazodicarboxylate;
- DIAD: diisopropylazodicarboxylate;
- DIPEA: *N,N-*Diisopropylethylamine;
- DMSO: dimethyl sulphoxide;
- DMA: dimethylacetamide;
- DMF: dimethylformamide;
- EDAC: 1-(3-dimethylaminopropyl)-3-ethylcarbodiimide hydrochloride;
- HATU: *O*-(7-azabenzotriazol-1-yl)-1,1,3,3-tetramethyluronium hexafluorophosphate;
- HPLC: high pressure liquid chromatography;
- HPMC: Hydroxypropylmethylcellulose;
- LCMS: liquid chromatography / mass spectroscopy;
- NMR: nuclear magnetic resonance spectroscopy;
- RT: room temperature;
- THF: tetrahydrofuran.

All compound names were derived using ACD NAME computer package.

### Example 1: 3-(4-{[(2-Methoxyethyl)amino]carbonyl}phenoxy)-5-(2-(1S)-methoxy-(1-methylethyl)oxy)-N-1,3-thiazol-2-ylbenzamide

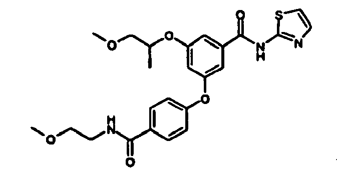

To a suspension of 4-({3-{[(1*S*)-2-methoxy-(1-methylethyl)oxy}-5-[(1,3-thiazol-2-ylamino) carbonyl] phenyl}oxy)benzoic acid (107 mg), HATU (122 mg) and 2-methoxyethylamine (38 mg) in DMF (2 mL), was added DIPEA (0.11 mL) and the mixture stirred at ambient temperature for 1 hour. Water (30 mL) was added and the mixture extracted with ethyl acetate (3 x 15 mL). The combined organic extracts were washed with brine, dried (MgSO₄), and evaporated to a residue which was chromatographed on silica with ethyl acetate as eluant to give the desired compound (63 mg).
¹H NMR δ (d₆-DMSO): 1.2 (d, 3H), 3.3 (s, 6H), 3.4-3.5 (m, 6H), 4.7-4.8 (m, 1H), 6.85 (s, 1H), 7.1 (d, 2H), 7.25 (m, 2H), 7.55 (d, 2H), 7.9 (d, 2H), 8.45 (s, 1H); *m*/*z* 486 (M+H)⁺

In a similar manner to that described above, **Examples 1a-1c** were also prepared:-

| **Example** | **Structure** | ***m*/*z*** | **NMR** |
|---|---|---|---|
| **1a** | 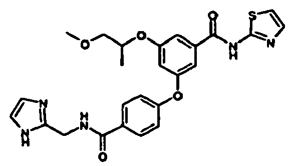 | 508 (M+H)⁺ | ¹H NMR δ (d₆-DMSO): 1.2 (d, 3H), 3.3 (s, 3H), 3.4-3.5 (d, 2H), 4.45 (d,2H), 4.7-4.8 (m, 1H), 6.85 (m, 3H), 7.1 (d, 2H), 7.25 (d, 2H), 7.55 (d, 2H) 7.95 (d, 2H), 8.95 (t, 1H) |
| **1b** | 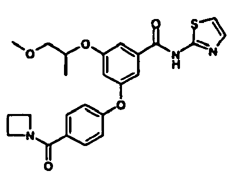 | 468 (M+H)⁺ | ¹H NMR δ (d₆-DMSO): 1.25 (d, 3H), 2.2-2.3 (m, 2H), 3.3 (s, 3H), 3.5 (m, 2H), 4.0 (m, 2H), 4.3 (m, 2H), 4.8 (m, 1H), 6.9 (s, 1H), 7.1 (d, 2H), 7.25 (m, 1H), 7.35 (s, 1H), 7.55 (d, 2H) 7.65 (d, 2H), 12.6 (s, 1H) |
| **1c** | 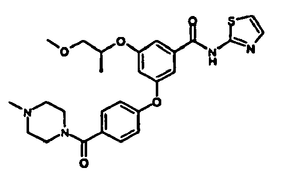 | 511 (M+H)⁺ | ¹H NMR δ (d₆-DMSO): 1.25 (d, 3H), 2.2 (s, 3H), 2.3 (m, 4H), 3.3 (s, 3H), 3.4-3.6 (m, 6H), 4.8 (m, 1H), 6.9 (s, 1H), 7.1 (d, 2H), 7.25 (m, 1H), 7.35 (s, 1H), 7.55 (d, 2H) 7.65 (d, 2H), 12.6 (s, 1H) |

The required acid for **Example 1** was prepared as described below:

### 4-({3-{[[(1S)-2-Methoxy-(1-methylethyl)oxy}-5-[(1,3-thiazol-2-ylamino)carbonyl] phenyl}oxy)benzoic acid

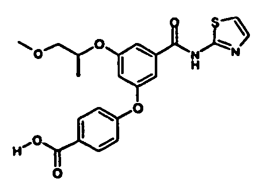

A solution of ethyl 4-({3-{[(1S)-2-methoxy-(1-methylethyl)oxy}-5-[(1,3-thiazol-2-ylamino)carbonyl] phenyl}oxy)benzoate (334 mg) in THF (10 mL) was added to a solution of lithium hydroxide monohydrate (82 mg) in water (5 mL). The mixture was stirred at ambient temperature for 16 hours and the THF removed *in vacuo.* The aqueous layer was acidified with 1M hydrochloric acid (1.83 mL), and the solid precipitate filtered off, washed with water and dried *in vacuo* to give the desired compound (268 mg).
¹H NMR δ (d₆-DMSO): 1.2 (d, 3H), 3.25 (s, 3H), 3.5 (m, 2H), 4.7-4.8 (m, 1H), 6.9 (t, 1H), 7.1 (d, 2H), 7.25 (d, 1H), 7.35 (s, 1H), 7.55 (d, 2H), 7.95 (d, 2H), 12.75 (s, 1H); *m*/*z* 429 (M+H)⁺

### Ethyl 4-({3-{[(1S)-2-methoxy-(1-methylethy)oxy}-5-[(1,3-thiazol-2-ylamino)carbonyl] phenyl}oxy)benzoate

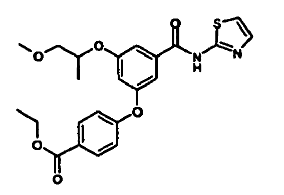

A solution of 3-hydroxy-5-{[(1*S*)-2-methoxy-(1-methylethyl)oxy}-*N*-1,3-thiazol-2-ylbenzamide (1.0g), 4-ethoxycarbonylphenylboronic acid (1.18 g), copper (II) acetate (1.19g), triethylamine (2.25 mL) and freshly activated 4A molecular sieves (4 g) in DCM (50 mL) was stirred at ambient temperature and under ambient atmosphere for 2 days. The reaction mixture was filtered through diatomaceous earth, washed with DCM (2x10 mL), the DCM removed *in vacuo* and the residual oil partitioned between ethyl acetate (75 mL) and 1M hydrochloric acid (30 mL). The ethyl acetate layer was separated, washed sequentially with aqueous sodium hydrogen carbonate solution and brine, dried (MgSO₄), and evaporated to a residue which was chromatographed on silica with 30% ethyl acetate in isohexane as eluant to give the desired compound (700 mg).
¹H NMR δ (CDCl₃): 1.3 (d, 3H), 1.4 (t, 3H), 3.4 (s, 3H), 3.5-3.6 (m, 2H), 4.35 (q, 2H), 4.5-4.6 (m, 1H), 6.85 (s, 1H), 6.95 (d, 1H), 7.0 (d, 2H), 7.15 (s, 1H), 7.2 (d, 1H), 7.35 (d, 1H), 8.05 (d, 2H); *mlz* 457 (M+H)⁺

### 3-Hydroxy-5-{[[(1S)-2-methoxy-(1-methylethyl)oxy}-N-1,3-thiazol-2-ylbenzamide

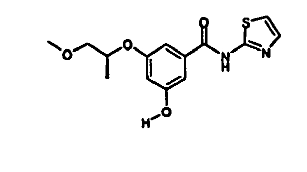

A solution of 3-{[(1*S*)-2-methoxy-(1-methylethyl)oxy}-5-{[(2-methylphenyl)methyl]oxy}-*N-*1,3-thiazol-2-ylbenzamide (6.9 g) and thioanisole (10 mL) in trifluoroacetic acid (65 mL) was stirred at ambient temperature for 16 hours. The trifluoroacetic acid was removed *in vacuo* and the residual oil partitioned between ethyl acetate (75 mL) and aqueous sodium hydrogen carbonate solution (200 mL). The aqueous layer was separated, extracted with ethyl acetate (2 x 75 mL), and the combined organic extracts washed with brine, dried (MgSO₄), and evaporated to a residue which was chromatographed on silica with 50% ethyl acetate in isohexane as eluant to give the desired compound (4.6 g).
¹H NMR δ (CDCl₃): 1.3 (d, 3H), 3.4 (s, 3H), 3.5-3.6 (m, 2H), 4.5-4.6 (m, 1H), 6.65 (s, 1H), 6.95 (d, 1H), 7.05 (s, 1H), 7.1 (s, 1H), 7.25 (d, 1H); *m*/*z* 309 (M+H)⁺

### 3-{[(1S)-2-Methoxy-(1-methylethyl)oxy}-5-{[(2-methylphenyl)methyl]oxy}-N-1,3-thiazol-2-ylbenzamide

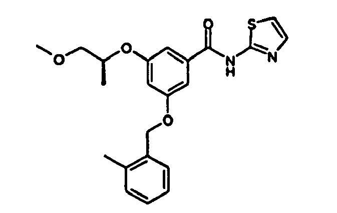

To a solution of 3-[(1*S*)-2-methoxy-(1-methylethyl)oxy]-5-{[(2-methylphenyl)methyl]oxy} benzoic acid (9.55 g) in DCM (140 mL) was added oxalyl chloride (2.83 mL), followed by DMF (1 drop), and the mixture stirred at ambient temperature for 16 hours. The DCM and excess oxalyl chloride were removed *in vacuo,* the residual oil dissolved in DCM (25 mL) and added to a solution of 2-aminothiazole (2.84g) and triethylamine (7.88 mL) in DCM (75 mL) at 0-5°C, and the mixture stirred at ambient temperature for 4 hours. The DCM and excess triethylamine were removed *in vacuo,* the residual oil partitioned between ethyl acetate (100 mL) and 1M hydrochloric acid (100 mL). The ethyl acetate layer was separated, washed sequentially with 1M hydrochloric acid, aqueous sodium hydrogen carbonate solution, and brine, dried (MgSO₄), and evaporated to a residue which was chromatographed on alumina with ethyl acetate as eluant to give the desired compound (11.0 g).
¹H NMR δ (CDCl₃): 1.3 (d, 3H), 2.35 (s,3H), 3.4 (s, 3H), 3.5-3.6 (m, 2H), 4.55-4.6 (m, 1H), 5.0 (s,2H), 6.8 (s, 1H), 6.95 (d, 1H), 7.15 (s, 1H), 7.25 (m, 5H), 7.4 (d, 1H); *mlz* 413 (M+H)⁺

### 3-[(1S)-2-Methoxy-(1-methylethyl)oxy]-5-{(2-methylphenyl)methyl]oxy}benzoic acid

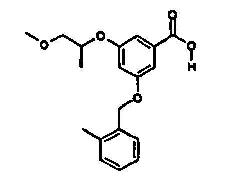

A solution of methyl 3-[{(1*S*)-2-methoxy-(1-methylethyl)oxy]-5-{[(2-methylphenyl) methyl] oxy}benzoate (10.65g) in THF (200 mL) and methanol (50 mL) was added to a solution of lithium hydroxide monohydrate (6.0g) in water (100 mL). The mixture was stirred at ambient temperature for 16 hours and the THF and methanol removed *in vacuo.* The aqueous layer was acidified to pH1 with hydrochloric acid, and extracted with ethyl acetate (3 x 50 mL). The combined organic extracts were washed with brine, dried (MgSO₄), and evaporated to give the desired compound (9.55 g).
*m*/*z* 329 (M-H)⁻

### Methyl 3-[{(1S)-2-methoxy-(1-methylethyl)oxy]-5-{(2-methylphenyl)methyl]oxy} benzoate

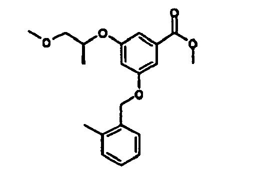

A stirred suspension of methyl 3-hydroxy-5-{[(2-methylphenyl)methyl]oxy}benzoate (15.3 g) and polymer-supported triphenyl phosphine (39.2 g) in dry DCM (900 mL) was cooled in an ice-bath and diisopropyl azodicarboxylate (11.88 mL) was added drop wise. The reaction mixture was stirred at 0-5°C for 30 minutes and (R)-1-methoxy-propan-2-ol was added dropwise. The reaction mixture was stirred at ambient temperature for 16 hours, filtered through diatomaceous earth and the DCM evaporated to a residue which was chromatographed on silica with 10% ethyl acetate in isohexane as eluant to give the desired compound (10.7 g).
¹H NMR δ (CDCl₃): 1.3 (d, 3H), 2.4 (s,3H), 3.4 (s, 3H), 3.5-3.6 (m, 2H), 3.9 (s, 3H), 4.55-4.6 (m, 1H), 5.0 (s,2H), 6.8 (s, 1H), 7.25 (m, 5H), 7.4 (d, 1H)

### Methyl 3-hydroxy-5-{[(2-methylphenyl)methyl]oxy}benzoate

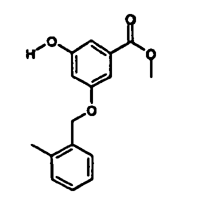

To a solution of methyl 3,5-dihydroxybenzoate (50 g, 0.30 mol) in DMF (500 mL) at 0°C was added sodium hydride (10.8 g, 0.27 mol) portionwise, maintaining the reaction temperature below 10°C. The reaction was allowed to warm to 15°C, and was stirred for 20 minutes. The mixture was cooled to 0°C and a solution of 2-methylbenzyl bromide (36 mL, 0.27 mol) in DMF (50 mL) was added over 30 minutes. The reaction was warmed to ambient temperature and concentrated *in vacuo,* the residual oil partitioned between ethyl acetate (500 mL) and water (250 mL), the ethyl acetate layer separated, washed sequentially with water and brine, dried (MgSO₄) and evaporated to a residue which was chromatographed on silica eluting with a gradient of 0-100% ethyl acetate in isohexane to give the desired compound (21.9 g). ¹H NMR δ (CDCl₃) 2.39 (s, 3H), 3.90 (s, 3H), 5.02 (s, 2H), 5.61 (s, 1H), 6.69 (t, 1H), 7.15-7.42 (m, 6H)

### Example 2: 3-(3-{[(2-Methoxyethyl)amino]carbonyl}phenoxy)-5-[(1S)-2-methoxy-(1-methylethyl)oxy]-N-1,3-thiazol-2-ylbenzamide

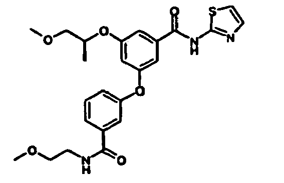

To a suspension of 3-({3-{[(1*S*)-2-methoxy-(1-methylethyl)oxy)-5-[(1,3-thiazol-2-ylamino)carbonyl] phenyl}oxy)benzoic acid (107 mg), HATU (122 mg) and 2-methoxyethylamine (38 mg) in DMF (2 mL) was added DIPEA (0.11 mL) and the mixture stirred at ambient temperature for 1 hour. Water (30 mL) was added and the mixture extracted with ethyl acetate (3 x 15 mL). The combined organic extracts were washed with brine, dried (MgSO₄), and evaporated to a residue which was chromatographed on silica, with ethyl acetate as eluant, to give the desired compound (85 mg).
¹H NMR δ (d₆-DMSO): 1.2 (d, 3H), 3.25 (s, 3H), 3.3 (s, 3H), 3.4-3.5 (m, 6H), 4.7-4.8 (m, 1H), 6.8 (s, 1H),7.2-7.25 (m, 3H), 7.55 (m, 4H), 7.7 (d, 1H) 8.55 (t, 1H), 12.6 (s, 1H); *m*/*z* 486 (M+H)⁺

In a similar manner, **Example 2a** was also prepared:-

| **Example** | **Structure** | ***m*/*z*** | **NMR** |
|---|---|---|---|
| **2a** | 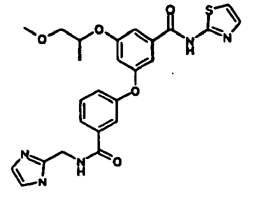 | 508 (M+H)⁺ | ¹H NMR δ (d₆-DMSO): 1.25 (d, 3H), 3.3 (s, 3H), 3.5 (m, 2H), 4.45 (d,2H), 4.7-4.8 (m, 1H), 6.8 (s, 1H), 6.85 (s, 2H), 7.2 (s, 1H), 7.25 (d, 2H), 7.5 (m, 3H), 7.6 (s, 1H), 7.75 (d, 2H), 9.0 (t, 1H) |

The required acid for **Example 2** was prepared as described below:

### 3-({3-{[(1S)-2-Methoxy-(1-methylethyl)oxy)-5-[(1,3-thiazol-2-ylamino)carbonyl] phenyl}oxy)benzoic acid

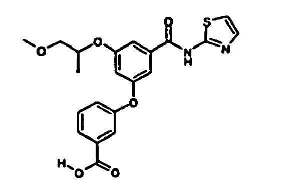

A solution of ethyl 3-({3-{[(1*S*)-2-methoxy-(1-methylethyl)oxy}-5-[(1,3-thiazol-2-ylamino) carbonyl]phenyl}oxy)benzoate (319 mg) in THF (10 mL) was added to a solution of lithium hydroxide monohydrate (78 mg) in water (5 mL). The mixture was stirred at ambient temperature for 16 hours and the THF removed in *vacuo.* The aqueous layer was acidified with 1M hydrochloric acid (1.75 mL), the solid precipitate filtered off, washed with water and dried *in vacuo* to give the desired compound (283 mg).
¹H NMR δ (d₆-DMSO): 1.2 (d, 3H), 3.25 (s, 3H), 3.5 (m, 2H), 4.7-4.8 (m, 1H), 6.85 (t, 1H), 7.25 (m, 2H), 7.35 (dd, 1H), 7.55 (m, 4H), 7.75 (d, 1H); *m*/*z* 429 (M+H)⁺

### Ethyl 3-({3-{[(1S)-2-methoxy-(1-methylethyl)oxy}-5-[(1,3-thiazol-2-ylamino)carbonyl]phenyl}oxy)benzoate

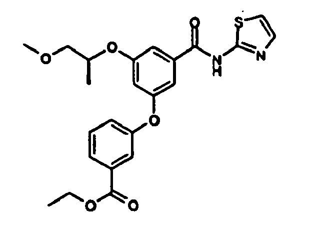

A solution of 3-hydroxy-5-{[(1*S*)-2-methoxy-(1-methylethyl)oxy}-*N*-1,3-thiazol-2-ylbenzamide (1.0 g), 3-ethoxycarbonylphenylboronic acid (1.18 g), copper (II) acetate (1.19 g), triethylamine (2.25 mL) and freshly activated 4A molecular sieves (4g) in DCM (50 mL) was stirred at ambient temperature and under ambient atmosphere for 2 days. The reaction mixture was filtered through diatomaceous earth, washed with DCM (2 x 10 mL), the DCM removed *in vacuo,* and the residual oil partitioned between ethyl acetate (75 mL) and 1 M hydrochloric acid (30 mL). The ethyl acetate layer was separated, washed sequentially with aqueous sodium hydrogen carbonate solution and brine, dried (MgSO₄), and evaporated to a residue which was chromatographed on silica (eluting with 30% ethyl acetate in isohexane) to give the desired ester (680 mg).
¹H NMR δ (CDCl₃): 1.3 (d, 3H), 1.4 (t, 3H), 3.4 (s, 3H), 3.5-3.6 (m, 2H), 4.35 (q, 2H), 4.5-4.6 (m, 1H), 6.8 (t, 1H), 6.95 (d, 1H), 7.1 (d, 1H), 7.2 (m, 2H), 7.3 (d, 1H), 7.4 (t, 1H), 7.7 (d, 1H), 7.85 (d, 1H), 11.6 (s, 1H); *m*/*z* 457 (M+H)⁺

The synthesis of 3-hydroxy-5-{[(1*S*)-2-methoxy-(1-methylethyl)oxy}-*N*-1,3-thiazol-2-ylbenzamide is described above in **Example 1**.

### Example 3: 3-[(1S)-2-Methoxy-(1-methylethyl)oxyl-5-{4-[(4-methylpiperazin-1-yl)carbonyl]phenoxy}-N-(1-methyl-1H-pyrazol-3-yl)benzamide

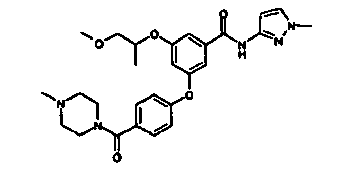

To a suspension of 4-[(3-{[(1*S*)-2-methoxy-(1-methylethyl)oxy}-5-{[(1-methyl-1*H*-pyrazol-3-yl)amino]carbonyl}phenyl)oxy]benzoic acid (212 mg), HATU (400 mg) and *N-*methylpiperazine (105 mg) in DMF (10 mL), was added DIPEA (0.35 mL) and the mixture stirred at ambient temperature for 24 hours. Water (30 mL) was added and the mixture extracted with ethyl acetate (3 x 15 mL). The combined organic extracts were washed with brine, dried (MgSO₄), and evaporated to a residue which was chromatographed on silica eluting with a gradient of 0-50% methanol in ethyl acetate to give the desired compound (130 mg).
¹H NMR δ (CDCl₃): 1.32 (d, 3H), 2.35 (s, 3H), 2.43 (m, 4H), 3.41 (s, 3H), 3.54 (m, 2H), 3.6-3.8 (m, 4H), 3.82 (s, 3H), 4.59 (m, 1H), 6.78 (m, 2H), 7.05 (t, 3 H), 7.22 (m, 1H), 7.27 (m, 1H), 7.42 (d, 2H), 8.30 (br s, 1H); *m*/*z* 508 (M+H)⁺

In a similar manner to that described above, **Examples 3a-3d** were also prepared:-

| **Example** | **Structure** | ***m*/*z*** | **NMR** |
|---|---|---|---|
| **3a** | 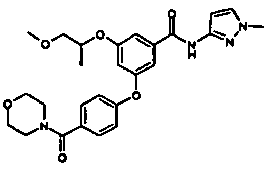 | 495 (M+H)⁺ | ¹H NMR δ (CDCl₃): 1.31 (d, 3H), 3.4 (s, 3H), 3.46-3.61 (m, 2H), 3.62-3.77 (m, 8H), 3.81 (s, 3H), 4.60 (m, 1H), 6.78 (m, 2H), 7.02 (s, 1H), 7.07 (m, 2H), 7.22 (m, 1H) 7.28 (m, 1H), 7.42 (d, 2H), 8.31 (br s, 1H) |
| **3b** | 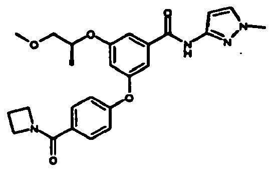 | 464 (M+H)⁺ | ¹H NMR δ (CDCl₃): 1.30 (d, 3H), 2.38 (m, 2H). 3.39 (s, 3H), 3.48-3.60 (m, 2H), 3.78 (s, 3H), 4.20-4.40 (m, 4H), 4.58 (m, 1H), 6.78 (m, 2H), 7.00 (d, 2H), 7.08 (s, 1H), 7.22 (s, 1H), 7.28 (s, 1H), 7.63 (d, 2H), 8.72 (s, 1H) |
| **3c** | 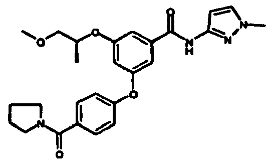 | 479 (M+H)⁺ | ¹H NMR δ (CDCl₃): 1.33 (d, 3H), 1.94 (m, 4H), 3.40 (s, 3H), 3.52 (m, 4H), 3.65 (m, 2H), 3.80 (s, 3H), 4.58 (m, 1H), 6.78 (m, 2H), 7.03 (d, 2H), 7.09 (m, 1H), 7.22 (m, 1H), 7.27 (m, 1H), 7.54 (d, 2H), 8.38 (brs, 1H) |
| **3d** | 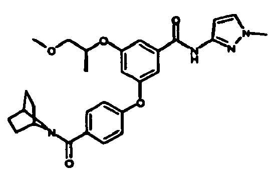 | 505 (M+H)⁺ | ¹H NMR δ (CDCl₃): 1.32 (d, 3H), 1.52 (m, 4H), 1.88 (m, 4H), 3.40 (s, 3H), 3.45-3.60 (m, 2H), 3.80 (s, 2H), 4.25 (m, 1H), 4.59 (m, 1H), 4.70 (m, 1H), 6.79 (m, 2H), 7.02 (d, 2H), 7.10 (m, 1H), 7.22 (m, 1H), 7.27 (m, 1H), 7.58 (d, 2H), 8.38 (s, 1H) |

The required acid for **Example 3** was prepared as described below:

### 4-[(3-[(1S)-2-Methoxy-(1-methylethyl)oxyl)-5-{[(1-methyl-1H-pyrazol-3-yl)amino]carbonyl}phenyl)oxy]benzoic acid

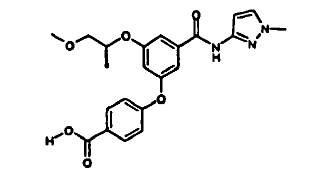

A solution of ethyl 4-[(3-[(1*S*)-2-methoxy-(1-methylethyl)oxy])-5-{[(1-methyl-1*H*-pyrazol-3-yl)amino]carbonyl}phenyl)oxy]benzoate (5.45 g) in THF (200 mL) was added to a solution of lithium hydroxide monohydrate (2.52 g) in water (100 mL). The mixture was stirred at ambient temperature for 48 hours and the THF removed *in vacuo.* The aqueous layer was acidified with 1M hydrochloric acid (60 mL), and the solid precipitate filtered off, washed with water and dried *in vacuo* to give the desired acid (5 g).
¹H NMR δ (d₆-DMSO): 1.22 (d, 3H), 3.26 (s, 3H), 3.45 (m, 2H), 3.75 (s, 3H), 4.71 (m, 1H), 6.51 (m, 1H), 6.84 (m, 1H), 7.08 (d, 2H), 7.24 (m, 1H), 7.44 (s, 1H), 7.57 (m, 1H), 7.95 (d, 2H), 10.84 (br s, 1H), 12.80 (br s, 1H); *m*/*z* 426 (M+H)⁺

### Ethyl 4[(3-[(1S)-2-methoxy-(1-methylethyl)oxyl]-5-{[(1-methyl-1H-pyrazol-3-yl)amino]carbonyl}phenyl)oxy]benzoate

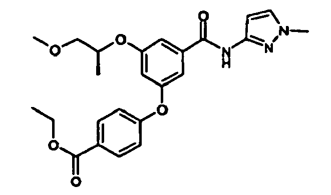

A solution of 3-hydroxy-5-[(1*S*)-2-methoxy-(1-methylethyl)oxy]-*N*-(1-methyl-1*H-*pyrazol-3-yl)benzamide (10.0 g), 4-ethoxycarbonylphenylboronic acid (9.4 g), copper (II) acetate (9 g), triethylamine (23 mL) and freshly activated 4Å molecular sieves (36 g) in DCM (500 mL) was stirred at ambient temperature and under ambient atmosphere for 2 days. The reaction mixture was filtered through celite, washed with DCM (2 x 50 mL), the DCM removed *in vacuo* and the residual oil partitioned between ethyl acetate (500 mL) and 1M hydrochloric acid (200 mL). The ethyl acetate layer was separated, washed sequentially with aqueous sodium hydrogen carbonate solution and brine, dried (MgSO₄), and evaporated to a residue which was chromatographed on silica eluting with a gradient of 50-100% ethyl acetate in isohexane to give the desired compound (5.47 g).
¹H NMR δ (CDCl₃): 1.3 (m, 3H), 1.41 (t, 3H), 3.39 (s, 3H), 3.49 (m, 1H), 3.58 (m, 1H), 3.78 (s, 3H), 4.38 (q, 2H), 4.58 (m, 1H), 6.79 (m, 2H), 7.01-7.1 (m, 3H), 7.26 (m, 2H), 8.01 (m, 2H), 8.61 (br s, 1H); *m*/*z* 454 (M+H)⁺

### 3-Hydroxy-5-[(1S)-2-methoxy-(1-methyllethyl)oxy]-N-(1-methyl-1H-pyrazol-3-yl)benzamide

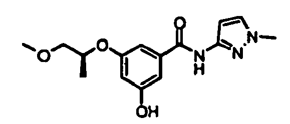

To a solution of 3-[(1*S*)-2-methoxy-(1-methylethyl)oxy]-*N*-(1-methyl-1*H*-pyrazol-3-yl)-5-[(phenylmethyl)oxy]benzamide (7.07 g) in THF (50 mL) and methanol (50 mL) was added 10% palladium on carbon (727 mg) as a slurry in THF (1mL) and methanol (1mL). The mixture was placed under vacuum and stirred under an atmosphere of hydrogen for 70 hours. The mixture was filtered through diatomaceous earth, and the diatomaceous earth washed with methanol (2 x 100 mL), followed by evaporation *in vacuo.* The residues were dissolved in ethyl acetate (10 mL), treated with isohexane (40 mL), the solid filtered off and washed with isohexane (50 mL) to afford the desired compound (5.17 g) which was used without further purification.
¹H NMR δ (d₆-DMSO): 1.22 (d, 3H), 3.28 (s, 3H, obscured by water), 3.38-3.53 (m, 2H), 3.76 (s, 3H), 4.65 (m, 1H), 6.44 (m, 1H), 6.54 (m, 1H), 6.93 (s, 1H), 7.04 (s, 1H), 7.57 (m, 1H), 9.63 (br s, 1H), 10.60 (s, 1H); *m*/*z* 306 (M+H)⁺, 304 (M-H)⁻

### 3-[(1S)-2-Methoxy-(1-methylethyl)oxy]-N-(1-methyl-1H-pyrazol-3-yl)-5-[(phenylmethyl)oxy]benzamide

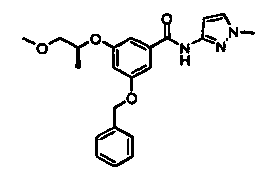

A solution of 3-[(1*S*)-2-methoxy-(1-methylethyl)oxy]-5-{[phenylmethyl]oxy}benzoic acid (8.73 g) in DCM (150 mL) was cooled to 0°C. Oxalyl chloride (4.81 mL) and DMF (0.15 mL) were slowly added with stirring. The mixture was allowed to warm to ambient temperature and stirred for 16 hours, following which the organics were removed *in vacuo,* and the residues azeotroped with toluene (75 mL). The crude material was dissolved in DCM (75 mL) and slowly added to a stirred suspension of 1-methyl-1*H*-pyrazol-3-amine (3.35 g) and DIPEA (14.4 mL) in DCM (75 mL). The mixture was stirred at ambient temperature for 18 hours, before the organics were evaporated *in vacuo* and the residue dissolved in ethyl acetate (150 mL). The organics were washed with 1M aqueous hydrochloric acid (100 mL) and brine (50 mL), and dried (MgSO₄), before evaporation *in vacuo* to give crude material. This was chromatographed on a 200g Biotage Flash 75 SiO₂ column (eluting with 30 to 90% ethyl acetate in isohexane), and evaporated *in vacuo* to afford the desired compound (7.07 g). ¹H NMR δ (d₆-DMSO): 1.23 (d, 3H), 3.28 (s, 3H, obscured by water), 3.40-3.52 (m, 2H), 3.77 (s, 3H), 4.70 (m, 1H), 5.03 (s, 2H), 6.56 (m, 1H), 6.71 (m, 1H), 7.18 (s, 1H), 7.24 (s, 1H), 7.32-7.47 (br m, 5H), 7.58 (m, 1H), 10.73 (s, 1H); *m*/*z* 396 (M+H)⁺.

### 3-[(1S)-2-Methoxy-(1-methylethyl)oxy]-5-{[phenylmethyl]oxy}benzoic acid

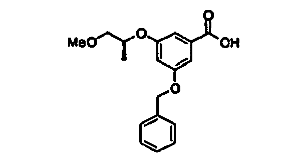

A solution of methyl 3-[(1*S*)-2-methoxy-(1-methylethyl)oxy]-5-{[phenylmethyl]oxy} benzoate (77.4 mmol) in a mixture of THF (232 mL) and methanol (232 mL) was treated with a solution of 2M sodium hydroxide (232 mmol), and the reaction mixture stirred for 4 hours at ambient temperature. The resulting solution was diluted with water (250 mL) and most of the organic solvent removed *in vacuo.* The resulting suspension was washed with diethyl ether (3 x 200 mL) and the organic washings discarded. The resulting aqueous solution was acidified to pH4 with 2M hydrochloric acid solution and extracted with ethyl acetate (2 x 200 mL). The extracts were combined, washed with brine, dried (MgSO₄), and evaporated to give the desired compound (99% yield).
¹H NMR δ (d₆-DMSO): 1.20 (d, 3H), 3.46 (m, 2H), 4.64 (m, 1H), 5.15 (s, 2H), 6.83 (app t, 1H), 7.06 (s, 1H), 7.13 (s, 1H), 7.30-7.49 (m, 5H), 12.67 (br s, 1H)

### Methyl 3-[(1S)-2-methoxy-(1-methylethyl)oxy]-5-{[phenylmethyl]oxy}benzoate

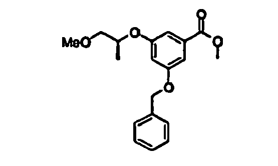

To a solution of methyl 3-hydroxy-5-{[phenylmethyl]oxy}benzoate (77.4 mmol) in THF was added polymer-supported triphenylphosphine (51.7g of 3 mmol/g loading, 155 mmol) and (*R*)-(-)-1-methoxy-2-propanol (102 mmol). The stirred solution was blanketed with argon and cooled in an ice bath. A solution of DIAD (116 mmol) was added dropwise by syringe over 10 minutes. The solution was stirred for 20 minutes and filtered, washing the residue with THF (500 mL). The filtrate and washings were combined, and evaporated to give the desired compound which was used without further purification.
¹H NMR δ (d₆-DMSO): 3.26 (s, 3H), 3.44 (m, 2H), 3.82 (s, 3H), 4.63 (m, 1H), 5.14 (s, 2H), 6.85 (s, 1H), 7.05 (s, 1H), 7.11 (s, 1H), 7.30-7.47 (m, 5H)
The ¹H NMR spectrum also contained signals consistent with a small amount of bis(1-methylethyl)hydrazine-1,2-dicarboxylate.

### Methyl 3-hydroxy-5-{[phenylmethyl]oxy}benzoate

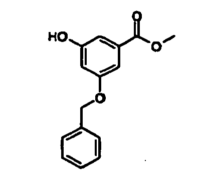

To a stirred solution of methyl 3,5-dihydroxybenzoate (5.95 mol) in DMF (6 L) was added potassium carbonate (9 mol), and the suspension stirred at ambient temperature under argon. To this was added benzyl bromide (8.42 mol) slowly over 1 hour, with a slight exotherm, and the reaction mixture stirred overnight at ambient temperature. The reaction was quenched cautiously with ammonium chloride solution (5 L) followed by water (35 L). The aqueous suspension was extracted with DCM (1 x 3 L and 2 x 5 L). The combined extracts were washed with water (10 L) and dried overnight (MgSO₄). The solution was evaporated in *vacuo,* and the crude product chromatographed in 3 batches (flash column, 3 x 2 kg silica, eluting with a gradient consisting of hexane containing 10% DCM, to neat DCM, to DCM containing 50% ethyl acetate) to eliminate starting material. The crude eluant was further chromatographed in 175 g batches (Amicon HPLC, 5 kg normal-phase silica, eluting with isohexane containing 20% v/v of ethyl acetate) to give the desired compound (21% yield). ¹H NMR δ (d₆-DMSO): 3.8 (s, 3H), 5.1 (s, 2H), 6.65 (m, 1 H), 7.0 (m, 1H), 7.05 (m, 1H), 7.3-7.5 (m, 5H), 9.85 (br s, 1H)

### Example 4: General Procedure for Preparation of Halogenated Sulphonamides

To a solution of the appropriate amine (1.8 mmol) in DCM (2 mL), was added the sulphonyl chloride (0.72 mmol) in DCM (2 mL), and the resulting mixture stirred for 18 hours. The mixture was treated with 1M aqueous hydrochloric acid (4 mL) and the organics separated. Evaporation *in vacuo* gave the crude fluorosulphonamide which was used without further purification.
To a solution of the crude fluorosulphonamide (7.2 mmol) in acetonitrile (3 mL), was added 3-hydroxy-5-[(1*S*)-2-methoxy-(1-methylethyl)oxy]-*N*-1-methyl-1*H*-pyrazol-3-yl)benzamide (0.36 mmol) and potassium carbonate (1.8 mmol). The mixture was heated to 170°C in a 'Smith Creator Microwave' for 100 minutes, before being filtered and the resultant organics evaporated *in vacuo.* The residues were then chromatographed on a Redisep (12g, SiO₂) cartridge using an Isco Optix chromatography system, eluting with 30 to 100% ethyl acetate in isohexane, and evaporated *in vacuo* to afford the desired compound.

### Examples 4a-4d were synthesised using the generic preparation described above:-

| **Example** | **Structure** | ***m*/*z*** | **NMR** |
|---|---|---|---|
| **4a** | 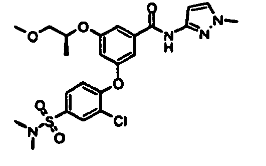 | 523, 525 (M+H)⁺ | ¹H NMR δ (d₆-DMSO): 1.24 (d, 3H), 2.65 (s, 6H), 3.27 (s, 3H, obscured by water), 3.42-3.54 (m, 2H), 3.76 (s, 3H), 4.72-4.81 (m, 1H), 6.55 (m, 1H), 6.93 (m, 1H), 7.20 (d, 1H), 7.26 (s, 1H), 7.48 (s, 1H), 7.58 (m, 1H), 7.70 (dd, 1H), 7.91 (m, 1H), 10.84 (s, 1H) |
| **4b** | 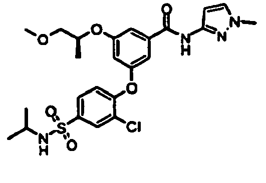 | 537, 539 (M+H)⁺ 535, 537 (M-H)⁻ | ¹H NMR δ (d₆-DMSO): 0.95 (d, 6H), 1.23 (d, 3H), 3.27 (s, 3H, obscured by water), 3.27 (m, 1H, obscured by water), 3.42-3.53 (m, 2H), 3.76 (s, 3H), 4.75 (m, 1H), 6.54 (m, 1H), 6.89 (m, 1H), 7.21 (s, 2H), 7.46 (s, 1H), 7.57 (m, 1H), 7.67 (d, 1H), 7.76 (dd, 1H), 7.95 (d, 1H), 10.84 (s, 1H) |
| **4c** | 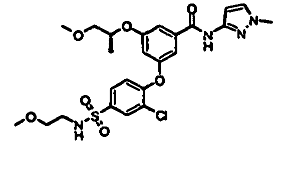 | 553, 555 (M+H)⁺ 551, 553 (M-H)⁻ | ¹H NMR δ (d₆-DMSO): 1.23 (d, 3H), 2.96 (m, 2H), 3.09 (m, 2H), 3.14 (s, 3H), 3.28 (s, 3H, obscured by water), 3.48 (m, 2H), 3.76 (s, 3H), 4.75 (m, 1H), 6.53 (m, 1H), 7.89 (m, 1H), 7.17-7.24 (m, 2H), 7.46 (m, 1H), 7.57 (m, 1H), 7.73 (dd, 1H), 7.82 (m, 1H), 7.96 (m, 1H), 10.84 (s, 1H) |
| **4d^{s}** | 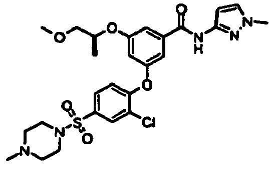 | 578, 580 (M+H)⁺ | ¹H NMR δ (d₆-DMSO): 1.24 (d, 3H), 2.13 (s, 3H), 2.35 (t, 4H), 2.94 (m, 4H), 3.28 (s, 3H, obscured by water), 3.42-3.53 (m, 2H), 3.75 (s, 3H), 4.76 (m, 1H), 6.54 (m, 1H), 6.93 (m, 1H), 7.21 (d, 1H), 7.28 (s, 1H), 7.49 (s, 1H), 7.58 (m, 1H), 7.69 (dd, 1H), 7.90 (m, 1H), 10.84 (s, 1H) |

| | | | |
|---|---|---|---|
| ^{$}The requisite sulphonamide for this example was prepared using a 1:1 ratio of amine : sulphonyl chloride, and isolated by treatment with I M aqueous sodium hydroxide | | | |

The synthesis of 3-hydroxy-5-[(1*S*)-2-methoxy-(1-methylethyl)oxy]-*N*-(1-methyl-1*H-*pyrazol-3-yl)benzamide is described in **Example 3** above.

### Example 5: General Procedure for Preparation of Sulphonamides

A solution of the requisite chlorosulphonamide from **Example 4** above (0.12 mmol) in THF (5 mL) and methanol (5 mL) was treated with 10% palladium on carbon (6 mg) and triethylamine (0.1 mL). The flask was put under vacuum, and stirred under an atmosphere of hydrogen gas. The resulting mixture was stirred at ambient temperature until starting material was consumed, before being filtered through diatomaceous earth and washed with methanol. Evaporation of the organics in vacuo, and azeotroping with diethyl ether (3 x 5 mL), followed by drying in vacuo, afforded the desired compound.

### Examples 5a-5d were synthesised using the generic procedure described above:-

| **Example** | **Structure** | ***m*/*z*** | **NMR** |
|---|---|---|---|
| **5a** | 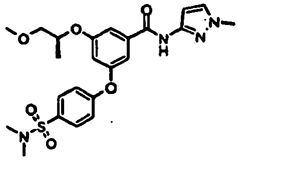 | 489 (M+H)⁺ | ¹H NMR δ (d₆-DMSO): 1.23 (d, 3H), 2.60 (s, 6H), 3.27 (s, 3H, obscured by water), 3.43-3.54 (m, 2H), 3.75 (s, 3H), 4.75 (m, 1H), 6.54 (m, 1H), 6.91 (m, 1H), 7.21 (d, 2H), 7.29 (s, 1H), 7.48 (s, 1H), 7.58 (m, 1H), 7.75 (d, 2H), 10.84 (s, 1H) |
| **5b** | 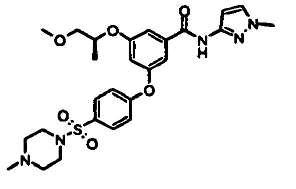 | 544 (M+H)⁺ | ¹H NMR δ (d₆-DMSO): 1.24 (d, 3H), 2.13 (s, 3H), 2.36 (s, 4H), 2.88 (s, 4H), 3.28 (s, 3H, obscured by water), 3.43-3.54 (m, 2H), 3.76 (s, 3H), 4.75 (m, 1H), 6.55 (m, 1H), 6.92(s, 1H), 7.22 (d, 2H), 7.30 (s, 1H), 7.49 (s, 1H), 7.58 (m, 1H), 7.75 (d, 2H), 10.84 (s, 1H) |
| **5c** | 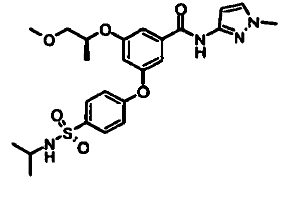 | 503 (M+H)⁺ | ¹H NMR δ (d₆-DMSO): 0.96 (d, 6H), 1.23 (d, 3H), 3.20 (m, 1H), 3.28 (s, 3H, obscured by water), 3.42-3.53 (m, 2H), 3.76 (s, 3H), 4.55 (m, 1H), 6.55 (m, 1H), 7.88 (m, 1H), 7.18 (d, 2H), 7.25 (s, 1H), 7.46 (s, 1H), 7.50 (d, 1H), 7.59 (m, 1H), 7.81 (d, 2H), 10.84 (s, 1H) |
| **5d** | 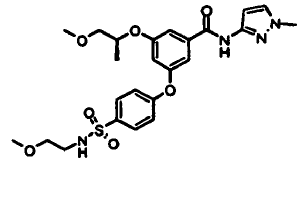 | 519 (M+H)⁺ 517 (M-H)⁻ | ¹H NMR δ (d₆-DMSO): 1.24 (d, 3H), 2.91 (m, 2H), 3.10 (m, 2H), 3.16 (s, 3H), 3.28 (s, 3H, obscured by water), 3.43-3.52 (m, 2H), 3.76 (s, 3H), 4.76 (m, 1H), 6.53 (, m, 1H), 6.87 (m, 1H), 7.19 (d, 2H), 7.25 (s, 1H), 7.45 (s, 1H), 7.57 (m, 1H), 7.64 (m, 1H), 7.80 (d, 2H), 10.84 (s, 1H) |

### Example 6: 3-(4-Cyanophenoxy)-5-[(S)-2-methoxy-(1-methylethyl)oxy]-N-(1-methyl-1H-pyrazol-3-yl)benzamide

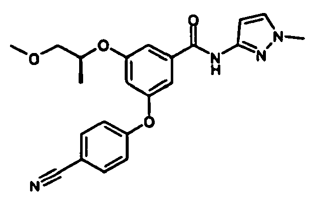

To a stirred solution of 3-hydroxy-5-[(1*S*)-2-methoxy-(1-methylethyl)oxy]-*N*-(1-methyl-1*H-*pyrazol-3-yl)benzamide (0.164 mmol) in DMF (1 mL) was added a 1M solution of sodium hexamethyldisilazide in THF (0.164 mmol). The reaction was stirred at room temperature for 10 minutes before adding 4-fluorobenzonitrile (0.164 mmol) The reaction was stirred overnight at room temperature, then heated to 60°C and stirred for a further 4 hours. The reaction was allowed to cool to room temperature, and treated with a further 0.2 equivalents of 4-fluorobenzonitrile and sodium hexamethyldisilazide, heated to 70°C and stirred at this temperature for 3 hours. The reaction was cooled to room temperature, and treated with a further 0.2 equivalents of sodium hexamethyldisilazide, warmed to 70°C, and stirred at this temperature overnight. The solvent was removed *in vacuo* and the residual oil partitioned between ethyl acetate and water. The water layer was separated and re-extracted with ethyl acetate. The combined organic layers were washed with brine, dried (MgSO₄), filtered and evaporated to a residue which was chromatographed on silica, using 0-1% methanol in DCM as the eluent, to give the desired product (60% yield).
¹H NMR δ (CDCl₃): 1.35 (d, 3H), 3.40 (s, 3H), 3.55 (m, 2H), 3.78 (s, 3H), 4.60 (m, 1H), 6.80 (m, 2H), 7.10 (m, 3H), 7.30 (m, 2H), 7.62 (d, 2H), 8.55 (br s, 1H); *m*/*z* 407 (M+H)⁺, 405 (M-H)⁻

The synthesis of 3-hydroxy-5-[(1*S*)-2-methoxy-(1-methylethyl)oxy]-*N*-(1-methyl-1*H-*pyrazol-3-yl)benzamide is described in **Example 3** above.

### Example 7: 3-{[4-(Aminocarbonyl)phenyl]oxy}-5-[(1S)-2-methoxy-(1-methylethyl)oxy]-N-(1-methyl-1H-pyrazol-3-yl)benzamide

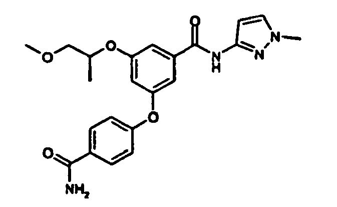

A suspension of 3-(4-cyanophenoxy)-5-[(1*S*)-2-methoxy-(1-methylethyl)oxy]-*N*-(1-methyl-1*H*-pyrazol-3-yl)benzamide (0.25 mmol), sodium azide (0.28 mmol) and zinc bromide (0.25 mmol) in water (2 mL) was heated to reflux and stirred at this temperature overnight. Isopropanol (2 mL) was added, and the reaction heated at reflux for a further 24 hours. The reaction was cooled to room temperature, evaporated to half volume *in vacuo,* and the residue partitioned between ethyl acetate and water. The water layer was separated and re-extracted with ethyl acetate. The combined organic layers were washed with brine, dried (MgSO₄), filtered and evaporated to a residue which was chromatographed on silica with 0-10% methanol in DCM as eluent to yield crude material. This material was dissolved in ethyl acetate and washed twice with 2M sodium hydroxide. The organic layer was washed with brine, dried (MgSO₄), filtered and evaporated. This material was dissolved in DCM and purified using an 'Isolute-NH2' ion-exchange column eluting with 10% methanol:DCM to yield the desired product.
¹H NMR δ (CDCl₃): 1.30 (d, 3H), 3.40 (s, 3H), 3.50 (m, 2H), 3.75 (s, 3H), 4.60 (m, 1H), 6.80 (m, 2H), 7.00 (d, 2H), 7.05 (s, 1H), 7.25 (m, 2H), 7.80 (d, 2H), 8.75 (br s, 1H); *m*/*z* 423 (M-H)⁻

The preparation of 3-(4-cyanophenoxy)-5-[(1*S*)-2-methoxy-(1-methylethyl)oxy]-*N*-(1-methyl-1*H*-pyrazol-3-yl)benzamide was described in **Example 6**.

### Example 8: 3-[4-(Ethylsulfonyl)phenoxy]-5-[(1S)-2-methoxy-(1-methylethyl)oxy]-N-1,3-thiazol-2-ylbenzamide

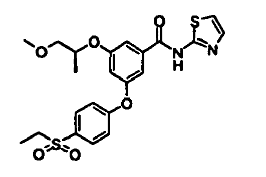

A solution of 3-hydroxy-5-[(1*S*)-2-methoxy-(1-methylethyl)oxy]-*N*-1,3-thiazol-2-ylbenzamide (154 mg), 4-ethanesulphonylbenzeneboronic acid (203 mg), copper (II) acetate (183 mg), triethylamine (0.345 mL) and freshly activated 4A molecular sieves (1 g) in DCM (10 mL), was stirred at ambient temperature and under ambient atmosphere for 3 days. The reaction mixture was filtered through diatomaceous earth, washed with DCM (10 mL), the DCM removed *in vacuo* and the residual oil dissolved in ethyl acetate (50 mL). The organic solution was washed with 1 M hydrochloric acid, saturated aqueous sodium hydrogen carbonate, brine, then dried (MgSO₄) and evaporated *in vacuo.* The residue was chromatographed on alumina with 5% methanol in ethyl acetate as eluant. Further chromatography on silica with 50% ethyl acetate in isohexane as eluant gave the desired compound (54 mg).
¹H NMR δ (CDCl₃): 1.2-1.35 (m, 6H), 3.15 (q, 2H), 3.4 (s, 3H), 3.5-3.6 (m, 2H), 4.5-4.6 (m, 1H), 6.8 (s, 1H), 6.95 (d, 1H), 7.2 (d, 2H), 7.25 (d, 2H), 7.4 (s, 1H), 7.85 (d, 2H). *m*/*z* 477 (M+H)⁺

The following compounds were also prepared in an analogous fashion from 3-hydroxy-5-[(1*S*)-2-methoxy-(1-methylethyl)oxy]-*N*-(1-methyl-1*H-*pyrazol-3-yl)benzamide:

| **Example** | **Structure** | ***m*/*z*** | **NMR** |
|---|---|---|---|
| **8a** | 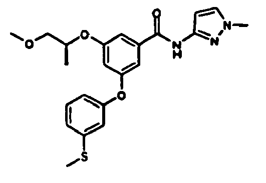 | 428 (M+H)⁺ | ¹H NMR δ (d₆-DMSO): 1.22 (d, 3H), 2.45 (s, 3H), 3.30 (s, 3H), 3.46 (m, 2H), 3.76 (s, 3H), 4.72 (m, 1H), 6.53 (m, 1H), 6.72 (m, 1H), 6.80 (m, 1H), 6.94 (m, 1H), 7.05 (d, 1H), 7.12 (s, 1H), 7.34 (t, 1H), 7.39 (s, 1H), 7.57 (m, 1H), 10.81 (bs, 1H) |
| **8b** | 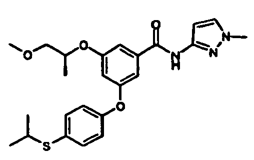 | 456 (M+H)⁺ | ¹H NMR δ (d₆-DMSO): 1.11 (m, 9H), 3.26 (s, 3H), 3.37 (m, 1H), 3.45 (m, 2H), 3.76 (s, 3H), 4.72 (m, 1H), 6.53 (m, 1H), 6.74 (m, 1H), 7.02 (d, 2H), 7.16 (s, 1H), 7.39 (m, 1H), 7.42 (d, 2H), 7.57 (m, 1H), 10.81 (bs, 1H) |

The syntheses of 3-hydroxy-5-[(1*S*)-2-methoxy-(1-methylethyl)oxy]-*N*-1,3-thiazol-2-ylbenzamide and 3-hydroxy-5-[(1*S*)-2-methoxy-(1-methylethyl)oxy]-*N*-(1-methyl-1*H-*pyrazol-3-yl)benzamide are described in **Examples 1** and **3** respectively.

### Example 9a: 3-1(1S)-2-Methoxy-(1-methylethyl)oxy]-N-(1-methyl-1H-pyrazol-3-yl)-5-[3-(methylsulfonyl)phenoxy]benzamide

### Example 9b: 3-[(1S)-2-Methoxy-(1-methylethyl)oxy]-N-(1-methyl-1H-pyrazol-3-yl)-5-[3-(methylsulfinyl)phenoxy]benzamide

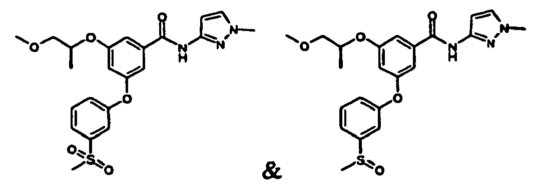

To a solution of 3-[(1*S*)-2-methoxy-(1-methylethyl)oxy]-*N*-(1-methyl-1*H-*pyrazol-3-yl)-5-{[3-(methylthio)phenyl]oxy}benzamide (prepared as described in **Example 8a** above, 270 mg) in DCM (5 mL) was added *m*-chloroperbenzoic acid (1.3 equivalents) and the reaction stirred at room temperature for 1 hour. A further 1.4 equivalents of *m*-chloroperbenzoic acid was added, and the reaction stirred at room temperature for a further 30 minutes. The reaction was added to saturated aqueous sodium metabisulphite and stirred for 20 minutes. The organic layer was separated, washed with brine, dried (MgSO4), and evaporated to a white foam. The crude mixture was purified using a 20 g Redisep column eluting with 0-5% methanol in DCM to yield the desired sulphone (117 mg).
¹H NMR δ (d₆-DMSO): 1.12 (d, 3H), 3.22 (s, 3H), 3.26 (s, 3H), 3.47 (m, 2H), 3.75 (s, 3H), 4.75 (m, 1H), 6.54 (m, 1H), 6.85 (m, 1H), 7.23 (s, 1H), 7.40 (m, 1H), 7.45 (s, 1H), 7.52 (m, 1H), 7.57 (m, 1H), 7.68 (m, 2H), 10.84 (br s, 1H); *mlz* 460 (M+H)⁺
A further fraction yielded the desired sulphoxide (105 mg).
¹H NMR δ (d₆-DMSO): 1.12 (d, 3H), 2.75 (s, 3H), 3.26 (s, 3H), 3.47 (m, 2H), 3.76 (s, 3H), 4.73 (m, 1H), 6.53 (m, 1H), 6.80 (m, 1H), 7.19 (m, 2H), 7.33 (m, 1H), 7.44 (m, 2H), 7.59 (m, 2H), 10.83 (br s, 1H); *m*/*z* 444 (M+H)⁺

### Example 10: 3-({4-[(1-Methylethyl)sulfonyl] phenyl}oxy)-5-[(1S)-2-methoxy-(1-methylethyl)oxy]-N-(1-methyl-1H-pyrazol-3-yl)benzamide

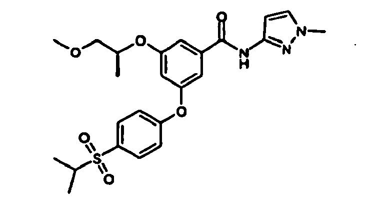

In a similar manner to that described above for **Example 9,** 3-({4-[(1-methylethyl)sulfonyl] phenyl}oxy)-5-[(1*S*)-2-methoxy-(1-methylethyl)oxy]-*N*-(1-methyl-1*H-*pyrazol-3-yl)benzamide was prepared from 3-({4-[(1-methylethyl)thio]phenyl}oxy)-5-[(1*S*)-2-methoxy-(1-methylethyl)oxy]-*N*-(1-methyl-1*H-*pyrazol-3-yl)benzamide.
¹H NMR δ (d₆-DMSO): 1.32 (m, 9H), 3.27 (m, 1H), 3.41 (s, 3H), 3.50 (dd, 1H), 3.58 (dd, 1H), 3.80 (s, 3H), 4.61 (m, 1H), 6.82 (m, 2H), 7.09 (d, 2H), 7.17 (m, 1H), 7.28 (m, 1H), 7.33 (m, 1H), 7.84 (d, 2H), 8.86 (br s, 1H); *m*/*z* 488 (M+H)⁺

The synthesis of 3-({4-[(1-methylethyl)thio]phenyl}oxy)-5-[(1*S*)-2-methoxy-(1-methylethyl) oxy]-*N*-(1-methyl-1*H-*pyrazol-3-yl)benzamide is described in **Example 8b** above.

### Example 11: General Procedure for Amide Synthesis - HATU Coupling

DIPEA (2.5 equivalents) was added to a suspension of 3-{(1*S*)-2-methoxy-(1-methylethyl)oxy}-5-{[4-(methylsulfonyl)phenyl] oxy}benzoic acid (1 equivalent), HATU(1.25 equivalents) and amine (1.25 equivalents) in DMF (20 mL). The initial suspension dissolved into a dark orange solution. The resulting mixture was stirred at ambient temperature for 2 hours. The DMF was removed *in vacuo,* and the residue azeotroped with toluene. Water was added and the mixture extracted with ethyl acetate. The extracts were combined and washed sequentially with 1M hydrochloric acid, saturated sodium hydrogen carbonate solution and brine. The solution was dried (MgSO₄), filtered, and evaporated *in vacuo* to give the crude product which was chromatographed (50% ethyl acetate in isohexane) to give desired compound (40-70% yield).

### Examples 11a and 11c-11g and Reference Example 11b were prepared using an analogous method to that described above from the appropriate acid and amino heterocycle:

| **Example/Reference Example** | **Structure** | ***m*/*z*** | **NMR** |
|---|---|---|---|
| **11a** | 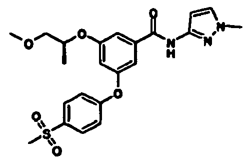 | 460 (M+H)⁺ | ¹H NMR δ (d₆-DMSO): 1.2 (d, 3H), 3.2 (s, 3H), 3.25 (s, 3H), 3.5 (m, 2H), 3.8 (s, 3H), 4.75 (m, 1H), 6.55 (s, 1H), 6.9 (s, 1H), 7.2 (d, 2H), 7.3 (s, 1H), 7.45 (s, 1H), 7.6 (s, 1H), 7.9 (d, 2H), 10.85 (br s, 1H) |
| **11b** | 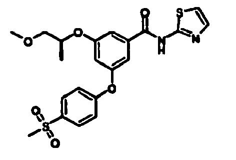 | 463 (M+H)⁺ 461 (M-H)⁻ | ¹H NMR δ (d₆-DMSO): 1.2 (d, 3H), 3.2 (s, 3H), 3.25 (s, 3H), 3.5 (m, 2H), 4.75 (m, 1H), 6.9 (s, 1H), 7.2 (d, 2H), 7.3 (s, 1H), 7.4 (s, 1H), 7.55 (d, 1H), 7.6 (s, 1H), 7.9 (d, 2H), 12.6 (br s, 1H) |
| **11c** | 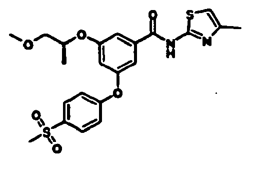 | 477 (M+H)⁺ 475 (M-H)⁻ | ¹H NMR δ (d₆-DMSO): 1.2 (d, 3H), 2.25 (s, 3H), 3.2 (s, 3H), 3.25 (s, 3H), 3.5 (m, 2H), 4.75 (m, 1H), 6.8 (s, 1H), 6.95 (s, 1H), 7.2 (d, 2H), 7.3 (s, 1H), 7.4 (s, 1H), 7.95 (d, 2H), 12.6 (br s, 1H) |
| **11d** | 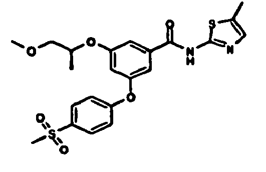 | 477 (M+H)⁺ 475 (M-H)⁻ | ¹H NMR δ (d₆-DMSO): 1.2 (d, 3H), 2.4 (s, 3H), 3.2 (s, 3H), 3.25 (s, 3H), 3.5 (m, 2H), 4.75 (m, 1H), 6.95 (s, 1H), 7.2 (s, 1H), 7.25 (d, 2H), 7.4 (s, 1H), 7.6 (s, 1H), 7.95 (d, 2H), 12.4 (br s, 1H) |
| **11e** | 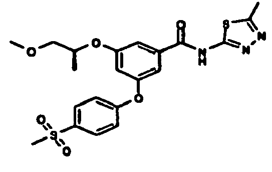 | 478 (M+H)⁺ 476 (M-H)⁻ | ¹H NMR δ (d₆-DMSO): 1.2 (d, 3H), 2.6 (s, 3H), 3.2 (s, 3H), 3.25 (s, 3H), 3.5 (m, 2H), 4.75 (m, 1H), 7.0 (s, 1H), 7.2 (d, 2H), 7.4 (s, 1H), 7.6 (s, 1H), 7.95 (d, 2H) |
| **11f*** | 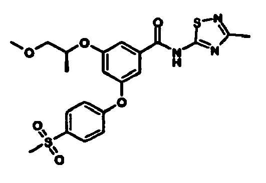 | 478 (M+H)⁺ 476 (M-H)⁻ | ¹H NMR δ (d₆-DMSO): 1.2 (d, 3H), 2.5 (s, 3H), 3.2 (s, 3H), 3.25 (s, 3H), 3.5 (m, 2H), 4.75 (m, 1H), 7.0 (s, 1H), 7.2 (d, 2H), 7.4 (s, 1H), 7.6 (s, 1H), 7.95 (d, 2H), 13.5 (br s, 1H) |
| **11g^{$}** | 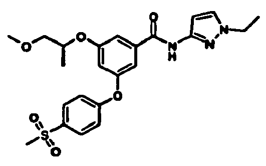 | 474 (M+H)⁺ | ¹H NMR δ (d₆-DMSO): 1.24 (d, 3H), 1.38 (t, 3H), 3.20 (s, 3H), 3.30 (s, 3H), 3.51 (m, 2H), 4.06 (s, 3H), 4.79 (m, 1H), 6.58 (s, 1H), 6.92 (s, 1H), 7.26 (d, 2H), 7.30 (s, 1H), 7.50 (s, 1H), 7.56 (s, 1H), 7.96 (d, 2H), 10.89 (s, 1H) |

| | | | |
|---|---|---|---|
| ***Example 11f** may be crystallised by allowing isohexane to vapour diffuse into a solution of the compound in ethylacetate, in a closed system, with subsequent slow evaporation of the mixture at room temperature over 4 days, mp 109-112. ^{$}The required amino pyrazole for **Example 11g** was prepared as follows: | | | |

Sodium hydride (60% dispersion in mineral oil, 39 mg, 0.973 mmol), was added to 5-nitro-1*H-*pyrazole (100 mg, 0.885 mmol) in dry DMF (2 mL) under an argon atmosphere. The solution was stirred for 5 minutes, then ethyl iodide (0.85 mL, 1.062 mmol) added and the reaction warmed to 80°C for 3 hours. Saturated aqueous sodium hydrogen carbonate (30 mL) was added, and the mixture extracted with diethyl ether (40 mL). The combined organic extracts were washed with brine, dried (MgSO₄), and evaporated to a residue which was purified by chromatography on silica (eluting with isohexane containing ethyl acetate, 33% v/v) to give the alkylated pyrazole (80 mg) which was used in the next step without further purification.
¹H NMR δ (CDCl₃): 1.58 (t, 3H), 4.26 (q, 2H), 6.91 (d, 1H), 7.48 (d, 1H).

To a solution of the alkylated pyrazole (70 mg, 0.50 mmol) in THF (5 mL) under an inert atmosphere was added 10% palladium on carbon (15 mg). The flask was evacuated and refilled 3 times with hydrogen gas, and stirred vigourously at room temperature for 3 hours. The reaction mixture was refilled with argon, and a further portion of 10% palladium on carbon (50 mg) added, followed by refilling as above with a hydrogen atmosphere. The reaction was stirred for 16 hours, filtered through diatomaceous earth, and evaporated to afford the title compound (56 mg) as a colourless oil which was used without further purification.
¹H NMR δ (CDCl₃): 1.42 (t, 3H), 3.58 (br. s, 2H), 3.98 (q, 2H), 5.59 (d, 1H), 7.16 (d, 1H)

The required acids for **Examples 11a and 11c-11g and Reference Example 11 b** were prepared as described below:

### 3-{(1S)-2-Methoxy-(1-methylethyl)oxy}-5-{[4-(methylsulfonyl)phenyl]oxy}benzoic acid

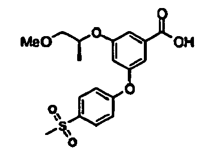

A solution of methyl 3-[(1*S*)-2-methoxy-(1-methylethyl)oxy]-5-{[4-(methylsulfonyl)phenyl] oxy}benzoate (60.9 mmol) in THF (400 mL) was treated with a solution of 1M sodium hydroxide (125 mmol), and the reaction mixture stirred for 13 hours at ambient temperature. Most of the organic solvent was removed *in vacuo,* and the remaining solution was diluted with water (150 mL). The resulting aqueous solution was acidified to pH4 with 1M citric acid solution, and extracted with ethyl acetate (2 x 100 mL). The extracts were combined, washed with brine, dried (MgSO₄), and evaporated to give the desired compound (83% yield).
¹H NMR δ (d₆-DMSO): 1.2 (d, 3H), 3.2 (s, 3H), 3.26 (s, 3H), 3.44 (m, 2H), 4.63 (m, 1H), 7.05 (s, 1H), 7.11 (s, 1H), 7.2 (d, 2H), 7.3 (s, 1H), 7.9 (d, 2H); m/z 479 (M-H)⁻

### Methyl 3-[(1S)-2-methoxy-(1-methylethyl)oxy]-5-{[4-(methylsulfonyl)phenyl]oxy} benzoate

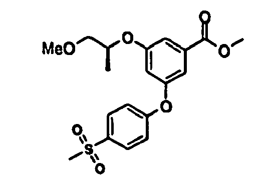

A suspension of methyl 3-hydroxy-5-[(1*S*)-2-methoxy-(1-methylethyl)oxy]benzoate (154 mmol), boronic acid (1.1 equivalents), copper (II) acetate (1.1 equivalents), triethylamine (5 equivalents) and freshly activated 4Å molecular sieves (200 g) in DCM (500 mL) was stirred at ambient temperature and under ambient atmosphere for 2 days. The reaction mixture was filtered, the DCM removed *in vacuo* and the residual oil partitioned between ethyl acetate and 1-2M hydrochloric acid. The ethyl acetate layer was separated, washed with aqueous sodium hydrogen carbonate and brine, dried (MgSO₄), and evaporated to a residue which was chromatographed on silica (with 20-60% ethyl acetate in isohexane as eluant) to give the desired ester (58% yield).
¹H NMR δ (d₆-DMSO): 1.2 (d, 3H), 3.2 (s, 3H), 3.26 (s, 3H), 3.44 (m, 2H), 3.8 (s, 3H), 4.65 (m, 1H), 7.05 (s, 1H), 7.11 (s, 1H), 7.2 (d, 2H), 7.3 (s, 1H), 7.9 (d, 2H)

### Methyl 3-Hydroxy-5-[(1S)-2-methoxy-(1-methylethyl)oxy]benzoate

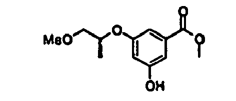

Methyl 3-[(1*S*)-2-methoxy-(1-methylethyl)oxy]-5- {[phenylmethyl]oxy}benzoate (50.0g; 0.152 mmol) was dissolved in a mixture of THF:ethanol (600 mL) and the flask evacuated and purged with nitrogen (3 times). 10% Palladium on carbon (5.0g) was added and the flask further evacuated and finally purged with hydrogen gas. The reaction mixture was stirred at ambient temperature for 20 hours until completion. The reaction mixture was evacuated and purged with nitrogen (3 times). The catalyst was filtered off, and the filtrate concentrated *in vacuo* to give the desired compound (36.7g). ¹H NMR δ (d₆-DMSO): 1.2 (d, 3H), 3.25 (s, 3H), 3.44 (m, 2H), 3.82 (s, 3H), 4.55 (m, 1H), 6.6 (s, 1H), 6.9 (s, 1H), 6.95 (s, 1H), 9.8 (s, 1H)

The synthesis of methyl 3-[(1*S*)-2-methoxy-(1-methylethyl)oxy]-5-{[phenylmethyl]oxy} benzoate is described above in **Example 1**.

### Example 12: General Procedure for Amide Synthesis - Oxalyl Chloride Coupling

To a stirred solution of 3-{(1*S*)-2-methoxy-(1-methylethyl)oxy}-5-{3,5-difluorophenoxy} benzoic acid (0.285 mmol) in dry DCM (2 mL), was added, dropwise under argon, oxalyl chloride (2 equivalents) and DMF (1 drop). The resulting solution was stirred at ambient temperature for 1-2 hrs. The solvent was removed *in vacuo* and the crude mixture taken up in pyridine (2 mL) and added to the appropriate amine (2.2 equivalents). The reaction mixture was stirred at room temperature, or heated if necessary, and monitored by TLC and/or LCMS. The pyridine was removed *in vacuo,* and water and ethyl acetate added. The organic layer was washed sequentially with 1 M citric acid and brine solution and dried (MgSO₄), concentrated *in vacuo,* and the residue chromatographed on silica (eluting with 30-90% ethyl acetate in isohexane) to give the desired product (typically 35-40% yield).

### Examples 12a & 12b were prepared using the appropriate amine:

| | | | |
|---|---|---|---|
| **12a** | 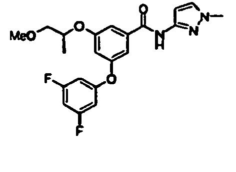 | 419 (M+H)⁺ | ¹H NMR δ (d₆-DMSO): 1.23 (d, 3H), 3.27 (s, 3H obscured by solvent peak), 3.47 (m, 2H), 3.76 (s, 3H), 4.74 (m, 1H), 6.55 (d, 1H), 6.80 (d, 2H), 6.86 (m, 1H), 7.02 (m, 1H), 7.24 (s, 1H), 7.44 (s, 1H), 7.57 (s, 1H), 10.82 (br s, 1H) |
| **12b^{$}** | 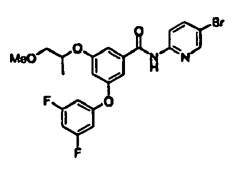 | 493,495 (M+H)⁺ | ¹H NMR δ (d₆-DMSO): 1.23 (d, 3H), 3.26 (s, 3H), 3.47 (m, 2H), 4.76 (m, 1H), 6.80 (dd, 2H), 6.92 (t, 1H), 7.02 (m, 1H), 7.26 (m, 1H), 7.45 (m, 1H), 8.04 (m, 1H), 8.13 (d, 1H), 8.49 (m, 1H), 11.01 (br s, 1H) |

| | | | |
|---|---|---|---|
| ^{$}In this example, the acid chloride was taken up in THF, followed by addition of pyridine and the appropriate amine. | | | |

The synthesis of 3-{(1*S*)-2-methoxy-(1-methylethyl)oxy)-5-{3,5-difluorophenoxy}benzoic acid is described below:

### 3-{(1S)-2-Methoxy-(1-methylethyl)oxy}-5-{3,5-difluorophenoxy}benzoic acid

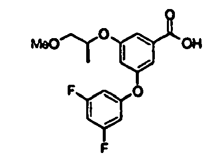

This was prepared from methyl 3-[(3,5-difluorophenyl)oxy]-5-[(1*S*)-2-methoxy-(1-methylethyl)oxy]benzoate using an analogous procedure to that described above for the synthesis of 3-{(1S)-2-methoxy-(1-methylethyl)oxy}-5-{[4-(methylsulfonyl)phenyl]oxy} benzoic acid:
¹H NMR δ (d₆-DMSO): 1.21 (d, 3H), 3.26 (s, 3H obscured by solvent peak), 3.46 (m, 2H), 4.67 (m, 1H), 6.81 (d, 2H), 6.96 - 7.08 (m, 3H), 7.27 (s, 1H), 13.13 (bs, 1H); *m*/*z* 337 (M-H)⁻

### Methyl 3-[(3,5-difluorophenyl)oxy]-5-[(1S)-2-methoxy-(1-methylethyl)oxy]benzoate

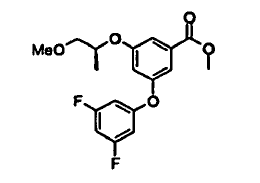

To a solution of methyl 3-[(3,5-difluorophenyl)oxy]-5-hydroxybenzoate (15.0 mmol), (*R*)-(-)-1-methoxy-2-propanol (18.75 mmol) and triphenylphosphine (18.0 mmol) in anhydrous THF (100 mL) at 0°C was added DIAD (18.0 mmol). The reaction was stirred at ambient temperature overnight, concentrated *in vacuo,* and the residue triturated with a 1:1 mixture of ethyl acetate:isohexane. The solid was removed by filtration and the filtrate concentrated *in vacuo,* chromatographed on silica (using a Biotage Flash 75 eluting with 10-15% ethyl acetate in isohexane) to give the title compound (75% yield).
¹H NMR δ (d₆-DMSO): 1.21 (d, 3H), 3.27 (s, 3H obscured by solvent peak), 3.46 (m, 2H), 3.82 (s, 3H), 4.69 (m, 1H), 6.81 (dd, 2H), 7.01-7.07 (m, 2H), 7.10 (s, 1H), 7.28 (s, 1H)

### Methyl 3-[(3,5-difluorophenyl)oxy]-5-hydroxybenzoate

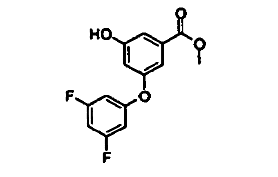

To a solution of methyl 3-[(3,5-difluorophenyl)oxy]-5-{[(4-methylphenyl)sulfonyl]oxy} benzoate (16.3 mmol) in methanol (60 mL) was added a 20% solution of potassium hydroxide in methanol (13.75 g). The mixture was heated at 50°C for 1 hour then allowed to cool. Water (20 mL) was added and the mixture immediately acidified with 1M hydrochloric acid. The methanol was removed *in vacuo* and the residue extracted with ethyl acetate. The organic phase was separated, washed with brine, dried (MgSO₄), and concentrated *in vacuo* to give the title compound (92% yield).
¹H NMR δ (d₆-DMSO): 3.80 (s, 3H), 6.72 (m, 1H), 6.79 (m, 2H), 6.98 - 7.05 (m, 2H), 7.19 (m, 1H), 10.18 (bs, 1H); *m*/*z* 279 (M-H)⁻

### Methyl 3-[(3,5-difluorophenyl)oxy]-5-{[(4-methylphenyl)sulfonyl]oxy}benzoate

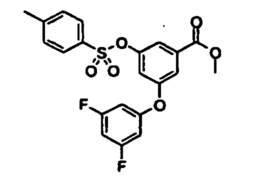

To a solution of methyl 3-hydroxy-5-{[(4-methylphenyl)sulfonyl]oxy}benzoate (30 mmol), copper (II) acetate (36 mmol), 3,5-difluorophenylboronic acid (42 mmol) and 4Å molecular sieves (30 g) in DCM (300 mL) was added triethylamine (150 mmol). The reaction was allowed to stir for 40 hours then filtered and concentrated *in vacuo.* The residue was dissolved in ethyl acetate, washed with 1 M citric acid solution, 1 M sodium hydrogen carbonate solution and brine, then dried (MgSO₄) and concentrated *in vacuo.* The residue was chromatographed on silica (Biotage Flash 75) eluting with 10- 25% ethyl acetate in isohexane to give the title compound (55% yield).
¹H NMR δ (d₆-DMSO): 2.39 (s, 3H), 3.83 (s, 3H), 6.74 (dd, 2H), 6.93 (m, 1H), 7.08 (m, 1H), 7.44 (m, 3H), 7.50 (s, 1H), 7.74 (d, 2H); *m*/*z* 452 (M+NH₄)⁺, 433 (M-H)⁻

### Methyl 3-hydroxy-5-{[(4-methylphenyl)sulfonyl]oxy}benzoate

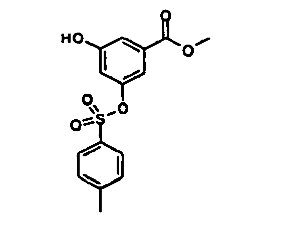

Methyl 3,5-dihydroxybenzoate (0.40 g) and 4-toluenesulphonylchloride (0.45 g) was stirred vigorously in diethyl ether (20 mL) with saturated aqueous sodium hydrogen carbonate (20 mL) at ambient temperature for 62 hours. The aqueous layer was removed and the residue washed sequentially with saturated aqueous sodium hydrogen carbonate, brine, dried (MgSO₄), filtered, and concentrated *in vacuo* to yield a colourless oil. The crude product was dissolved in diethyl ether, washed with saturated aqueous potassium carbonate then with brine, dried (MgSO₄), filtered and concentrated *in vacuo* to give a colourless oil which crystallised on standing to give the title compound (0.51 g).
¹H NMR δ (d₆-DMSO): 2.43 (s, 3H), 3.82 (s, 3H), 6.66 (m, 1H), 6.97 (s, 1H), 7.26 (s, 1H), 7.47 (d, 2H), 7.75 (d, 2H); *m*/*z* 340 (M+NH₄)⁺

### Example 13: 3-(3,5-Difluorophenoxy)-N-[4-(hydroxymethyl)-1,3-thiazol-2-yl]-5-[2-(1S)-methoxy-(1-methylethyl)oxy] benzamide

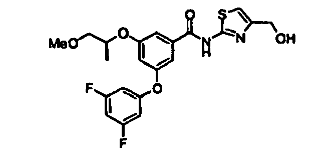

To a solution of 3-(3,5-difluorophenoxy)-*N*-[4-chloromethyl-1,3-thiazol-2-yl]-5-[(1*S*)-2-methoxy-(1-methylethyl)oxy]benzamide (0.107 mmol) in THF (1 mL) was added 0.5M sodium hydroxide solution (1 mL). The reaction was stirred at ambient temperature for 2 hours and the organics removed *in vacuo.* The residue was acidified with 1M citric acid and partitioned between ethyl acetate and water. The organic phase was separated, dried (MgSO₄), and concentrated *in vacuo.* The residue was chromatographed on silica eluting with 80% ethyl acetate in isohexane to give the title compound which was precipitated from a concentrated diethyl ether solution by the addition of isohexane to give a solid sample (35% yield).
¹H NMR δ (d₆-DMSO): 1.24 (d, 3H), 3.28 (s, 3H obscured by solvent peak), 3.48 (m, 2H), 4.49 (s, 2H), 4.75 (m, 1H), 6.83 (d, 2H), 6.93 (s, 1H), 6.98 (s, 1H), 7.04 (m, 1H), 7.32 (s, 1H), 7.54 (s, 1H); *m*/*z* 451 (M+H)⁺, 449 (M-H)⁻

### 3-(3,5-Difluorophenoxy)-N-[4-chloromethyl-1,3-thiazol-2-yl]-5-[(1S)-2-methoxy-(1-methylethyl)oxy]benzamide

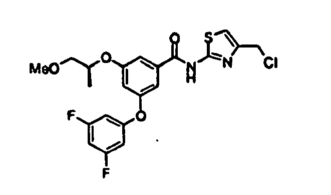

To a stirred solution of 3-{(1*S*)-2-methoxy-(1-methylethyl)oxy}-5-{3,5-difluorophenoxy}benzoic acid (3.06 mmol) in DCM (20 mL) was added 3 drops of DMF and oxalyl chloride (6.12 mmol; 2.0 equivalents) dropwise, and the resulting mixture stirred at ambient temperature for 5 hours. The reaction mixture was concentrated *in vacuo,* azeotroped with toluene and dried overnight at reduced pressure. The residue was dissolved in DCM and 4-(chloromethyl)-1,3-thiazol-2-amine (3.36 mmol), triethylamine (3.36 mmol) and dimethylaminopyridine (0.31 mmol) added. The resulting mixture was stirred for 16 hours at ambient temperature. The reaction mixture was washed sequentially with 2M hydrochloric acid and 1M sodium hydrogencarbonate solution, dried (MgSO₄), and concentrated *in vacuo.* The residue was chromatographed (eluting with 15-20% ethyl acetate in isohexane) to give the desired compound (33% yield).
¹H NMR δ (d₆-DMSO): 1.24 (d, 3H), 3.28 (s, 3H obscured by solvent peak), 3.49 (m, 2H), 4.76 (m, 3H), 6.84 (dd, 2H), 6.94 (s, 1H), 7.04 (m, 1H), 7.32 (m, 2H), 7.55 (s, 1H), 12.77 (bs, 1H); m/z 469, 471 (M+H)⁺, 467, 469 (M-H)⁻

The synthesis of 3-{(1*S*)-2-methoxy-(1-methylethyl)oxy}-5-{3,5-difluorophenoxy}benzoic acid is described in **Example 12** above.

The synthesis of 4-(chloromethyl)-1,3-thiazol-2-amine is described in the literature (J. Indian Chem. Soc. 1960, 37, 241).

### Reference Example 14: 3-[(1S)-2-Methoxy-(1-methylethyl)oxy]-5-[4-(methylsulfonyl)phenoxy]-N-1H-pyrazol-3-ylbenzamide

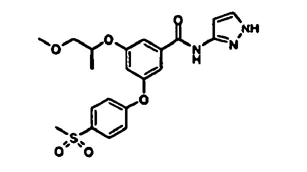

Trifluoroacetic acid (0.5 mL) was added to a solution of *tert*-butyl 3-({3-[(1*S*)-2-methoxy-(1-methylethyl)oxy]-5-[4-(methylsulfonyl)phenoxy]benzoyl}amino)-1*H-*pyrazole-1-carboxylate (180 mg, 0.330 mmol) in dry DCM (3 mL) and the reaction was stirred under argon for 3 h. A further portion of trifluoroacetic acid (0.2 mL) was then added and the reaction was stirred for 30 min, then evaporated in *vacuo.* The residue was taken up in ethyl acetate (30 mL) and saturated aqueous sodium hydrogencarbonate (15 mL) and the residue was evaporated, then dissolved in DCM and hexane and evaporated to produce the title compound (145 mg) as a colourless foam.
¹H NMR δ (d₆-DMSO): 1.27 (d, 3H), 3.22 (s, 3H), 3.31 (s, 3H), 3.60 (m, 2H, partially obscured by HOD), 4.78 (m, 1H), 6.62 (s, 1H), 6.93 (s, 1H), 7.27 (d, 2H), 7.32 (s, 1H), 7.53 (s, 1H), 7.65 (s, 1H), 7.96 (d, 2H), 10.86 (s, 1H); *mlz* 444 (M-H)⁻

### tert-Bulyl 3-({3-[(1S)-2-methoxy-(1-methylethyl)oxyl-5-[4-(methylsulfonyl)phenoxyl benzoyl}amino)-1H-pyrazole-1-carboxylate

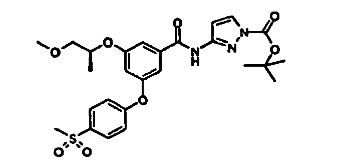

HATU (375 mg, 1.17 mmol) was added to 3-{(1*S*)-2-methoxy-(1-methylethyl)oxy}-5-{[4-(methylsulfonyl) phenyl]oxy}benzoic acid (300 mg, 0.79 mmol) followed by addition of DMF (5 mL), DIPEA (0.35 mL) and *tert*-butyl 3-amino-1*H*-pyrazole-1-carboxylate (155 mg, 0.85 mmol). The reaction was stirred under argon for 4 h, the solvent evaporated, and the residue dissolved in saturated aqueous sodium hydrogencarbonate (30 mL) and ethyl acetate (50 mL). The organic layer was separated, washed with saturated aqueous ammonium chloride (30 mL), then dried (MgSO₄), filtered and evaporated. Purification by column chromatography, eluting with 1:1 ethyl acetate:hexanes, afforded the title compound (185 mg, 43%) as a colourless oil. ¹H NMR δ (CDCl₃): 1.37 (d, 3H), 1.63 (s, 9H), 3.09 (s, 3H), 3.40 (s, 3H), 3.58 (m, 2H), 4.61 (m, 1H), 6.85 (s, 1H), 7.08 (m, 2H), 7.15 (d, 2H), 7.30 (s, 1H), 7.92 (d, 2H), 8.01 (d, 1H), 8.58 (br. s, 1 H); *m*/*z* 544 (M-H)⁻

### tert-Butyl 3-amino-1H-pyazole-1-carboxylate

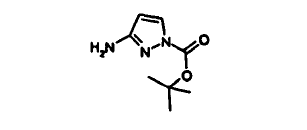

1*H*-Pyrazol-3-amine (428 mg, 5.15 mmol) was dissolved in DMF (5 mL) at 0°C and treated with sodium hydride (206 mg, 5.15 mmol) followed by stirring for a further 30 min. Warmed di-tert-butyl dicarbonate (1.12 g, 5.15 mmol) was then slowly added via syringe over 5 min and the reaction was allowed to warm to room temperature and stirred for a further 2 h. The reaction was taken up in saturated aqueous sodium hydrogencarbonate (50 mL) and ethyl acetate (100 mL). The organic layer was separated then dried (MgSO₄), filtered and evaporated. Purification by column chromatography (eluting with 1:1 ethyl acetate:hexanes to neat ethyl acetate) afforded the title compound (117 mg) as a white solid.
¹H NMR δ (CDCl₃): 1.62 (s, 9H), 4.00 (br. s, 2H), 5.81 (d, 1H), 7.82 (d, 1H)

The synthesis of 3- {(1S)-2-methoxy-(1-methylethyl)oxy}-5-{[4-(methylsulfonyl) phenyl]oxy}benzoic acid is described in **Example 11** above.

### Example 15: 3-[(1S)-2-Methoxy-(1-methylethyl)oxy]-N-(5-methyl-1H-pyrazol-3-yl)-5-[4-(methylsulfonyl)phenoxy]benzamide

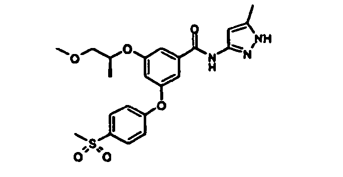

Trifluoroacetic acid (1.5 mL) was added to a solution of *tert*-butyl 3-({3-[(1*S*)-2-methoxy-(1-methylethyl)oxy]-5-[4-(methylsulfonyl)phenoxy]benzoyl}amino)-5-methyl-1*H-*pyrazole-1-carboxylate (500 mg, 0.330 mmol) in dry DCM (6 mL) and the reaction was stirred under argon for 2 h. The solvent was removed in *vacuo* and the residue was taken up in ethyl acetate (30 mL) and saturated aqueous sodium hydrogencarbonate (15 mL). The organic layer was separated, dried (MgSO₄), filtered, evaporated, then re-evaporated with DCM / hexanes to produce the title compound (350 mg) as a colourless foam.
¹H NMR δ (DMSO-d₆): 1.23 (d, 3H), 2.20 (s, 3H), 3.20 (s, 3H), 3.30 (s, 3H) (obscured by HOD), 3.50 (m, 2H) 4.78 (m, 1H), 6.38 (s, 1H), 6.90 (s, 1H), 7.22 (d, 2H), 7.30 (s, 1H), 7.45 (s, 1H), 7.93 (d, 2H), 10.71 (br. s, 1H), 12.08 (br. s, 1H); *m*/*z* 458 (M-H)⁻

### tert-Butyl 3-({3-[(1S)-2-methoxy-(1-methylethyl)oxyl-5-[4-(methylsulfonyl)phenoxy]benzoyl}amino)-5-methyl-1H-pyrazole-1-carboxylate

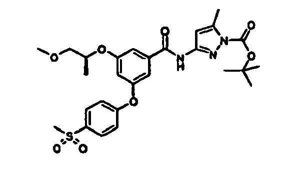

HATU (500 mg, 1.31 mmol) was added to 3-{(1S)-2-methoxy-(1-methylethyl)oxy}-5-{[4-(methylsulfonyl) phenyl]oxy}benzoic acid (400 mg, 1.05 mmol) followed by addition of DMF (6 mL), DIPEA (0.47 mL) and tert-butyl 3-amino-5-methyl-1*H*-pyrazole-1-carboxylate (380 mg, 1.93 mmol). The reaction was stirred under argon for 72 h, then dissolved in saturated aqueous sodium hydrogencarbonate (30 mL) and ethyl acetate (50 mL). The organic layer was separated, washed with saturated aqueous ammonium chloride (30 mL), then dried (MgSO₄), filtered and evaporated. Purification by column chromatography eluting with 1:1 to 2:1 ethyl acetate:hexanes afforded the title compound (500 mg, 85%) as a foam. ¹H NMR δ (CDCl₃): 1.37 (d, 3H), 1.62 (s, 9H), 2.54 (s, 3H), 3.08 (s, 3H), 3.40 (s, 3H), 3.58 (m, 2H), 4.60 (m, 1H), 6.82 (m, 2H), 7.08 (m, 1H), 7.15 (d, 2H), 7.30 (s, 1H), 7.93 (d, 2H), 8.52 (brs, 1H); *m*/*z* 558 (M-H)⁻

### tert-Butyl 3-amino-5-methyl-1H-pyrazole-1-carboxylate

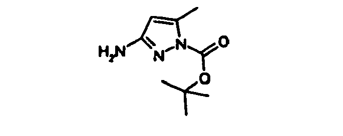

5-Methyl-1*H* pyrazol-3-amine (800 mg, 8.25 mmol) was dissolved in DMF (10 mL) at 0 °C and treated with sodium hydride (336 mg, 8.25 mmol) followed by stirring for a further 30 min. Warmed di-*tert*-butyl dicarbonate (1.80 g, 8.25 mmol) was then slowly added via syringe over 5 min and the reaction was allowed to warm to room temperature and stirred for a further 1 h. The reaction was taken up in saturated aqueous sodium hydrogencarbonate (50 mL) and ethyl acetate (100 mL). The organic layer was separated then dried (MgSO₄), filtered and evaporated. Purification by column chromatography (eluting with 1:1 ethyl acetate:hexanes to 100% ethyl acetate) afforded the title compound (380 mg, 23%) as a colourless oil.
¹H NMR δ (CDCl₃): 1.62 (s, 9H), 2.43 (s, 3H), 3.87 (br. s, 2H), 5.60 (s, 1H)

The synthesis of 3-{(1S)-2-methoxy-(1-methylethyl)oxy}-5-{[4-(methylsulfonyl) phenyl]oxy}benzoic acid is described in **Example 11** above.

### Example 16: 3-[(1S)-2-methoxy-(1-methylethyl)oxyl-N-[4-(methoxymethyl)-1,3-thiazol-2-yl]-5-[4-(methylsulfonyl)phenoxy]benzamide

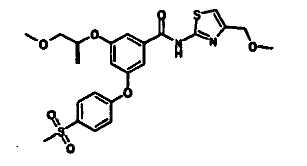

To a stirred solution of *N*-[4-(chloromethyl)-1,3-thiazol-2-yl]-3-[(1*S*)-2-methoxy-(1-methylethyl)oxy]-5-[4-(methylsulfonyl)phenoxy]benzamide (280mg; 0.55 mmol) in methanol (5 mL) was added sodium methoxide (1.1 mmol; 2.0 equiv; 25% weight in methanol) and the reaction mixture heated to 50°C and stirred overnight. The reaction mixture was concentrated *in vacuo* and chromatographed (eluting with 50-70% ethyl acetate:isohexane) to give the title compound (71 mg; 26%).
¹H NMR δ (d₆-DMSO): 1.21 (d, 3H), 2.50 (2s, 6H, partially obscured by water peak), 3.21 (s, 3H), 3.43-3.54 (m, 2H), 4.40 (s, 2H), 4.78 (m, 1H), 6.98 (s, 1H), 7.12 (s, 1H), 7.25 (d, 2H), 7.37 (s, 1H), 7.58 (s, 1H), 7.95 (d, 2H), 12.69 (br s, 1H); *m*/*z* 507 (M+H)⁺, 505 (M-H)⁻

### N-[4-(Chloromethyl)-1,3-thiazol-2-yl]-3-[(1S)-2-methoxy-(1-methylethyl)oxy]-5-[4-(methylsulfonyl)phenoxy]benzamide

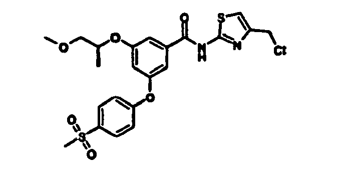

To a stirred solution of 3-{(1*S*)-2-methoxy-(1-methylethyl)oxy}-5-{[4-(methylsulfonyl) phenyl]oxy}benzoic acid (1.0 mmol) in DCM (10 mL) was added 1 drop of DMF and oxalyl chloride (2.0mmol; 2.0 equivalents) dropwise. The reaction mixture was stirred at ambient temperature under argon for two hours, then concentrated *in vacuo* and azeotroped with DCM. The residue was dissolved in DCM and 4-(chloromethyl)-1,3-thiazol-2-amine (1.0 mmol) in DCM was added along with DIPEA (2.5 mmol) and dimethylaminopyridine (0.1 mmol). The resulting mixture was stirred for 13 hours under argon at ambient temperature, then concentrated *in vacuo* and chromatographed (eluting with 50-60% ethyl acetate in isohexane) to give the title compound (53% yield).
¹H NMR δ (d₆-DMSO): 1.3 (d, 3H), 3.2 (s, 3H), 3.25 (s, 3H) 3.45 (m, 2H), 4.75 (s, 2H), 4.8 (m, 1H), 7.0 (s, 1H), 7.25 (d, 2H), 7.3 (s, 1H), 7.4 (s, 1H), 7.6 (s, 1H), 7.95 (d, 2H), 12.80 (br s, 1H)

The synthesis of 3-{(1S)-2-methoxy-(1-methylethyl)oxy}-5-{[4-(methylsulfonyl) phenyl]oxy}benzoic acid is described in **Example 11** above.

### Example 17: 3-[4-(Azetidin-1-ylcarbonyl)phenoxy]-5-[(1S)-2-methoxy-(1-methylethyl)oxy]-N-(3-methyl-1,2,4-thiadiazol-5-yl)benzamide

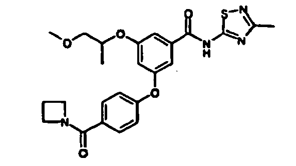

To a suspension of 4-({3-{[(1*S*)-2-methoxy-(1-methylethyl)oxy}-5-[(3-methyl-1,2,4-thiadiazol-2-ylamino)carbonyl] phenyl}oxy)benzoic acid (300 mg), HATU (336 mg) and azetidine hydrochloride (190 mg) in DMF (5 mL), was added DIPEA (0.68 mL) and the mixture stirred at ambient temperature for 16 h. Water (75 mL) was added and the mixture extracted with ethyl acetate (3 x 25 mL). The combined organic extracts were washed 1 M aqueous hydrochloric acid (25 mL), saturated aqueous sodium hydrogen carbonate solution (25 mL), brine, dried (MgSO₄), and evaporated to a residue which was chromatographed on silica with ethyl acetate as eluant to give the desired compound (190 mg).
¹H NMR δ (d₆-DMSO): 1.25 (d, 3H), 2.2-2.3 (m, 2H), 2.5 (m, 3H), 3.3 (s, 3H), 3.5 (m, 2H), 4.0 (m, 2H), 4.3 (m, 2H), 4.8 (m, 1H), 6.95 (s, 1H), 7.1 (d, 2H), 7.35 (s, 1H), 7.55 (s, 1H), 7.65 (d, 2H), 13.35 (s, 1H); *m*/*z* 483 (M+H)⁺

In a similar manner **Examples 17a** and Reference Example **17b** were also prepared:

| **Example/ Reference Example** | **Structure** | ***m*/*z*** | **NMR** |
|---|---|---|---|
| **17a** | 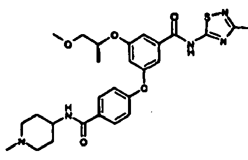 | 540 (M+H)⁺ | ¹H NMR δ (d₆-DMSO): 1.25 (d, 3H), 1.5-1.65 (m, 2H), 1.7-1.8 (m, 2H), 1.95-2.0 (m, 2H), 2.15 (s, 3H), 2.3 (s, 3H), 2.7-2.8 (m, 2H), 3.3 (s, 3H), 3.55 (m, 2H), 3.7 (m, 1H), 4.7 (m, 1H), 6.7 (s, 1H), 7.1 (d, 2H), 7.25 (s, 1H), 7.5 (s, 1H) 7.85 (d, 2H), 8.2 (d, 1H) |
| **17b** | 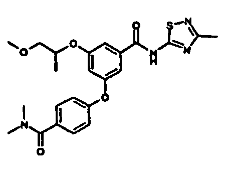 | 471 (M+H)⁺ | ¹H NMR δ (d₆-DMSO): 1.25 (d, 3H), 2.5 (s, 3H), 2.95 (s, 6H), 3.3 (s, 3H), 3.5 (m, 2H), 4.75 (m, 1H), 6.9 (s, 1H), 7.1 (d, 2H), 7.35 (s, 1H), 7.45 (d, 2H) and 7.55 (s, 1H) |

### 4-({3- {[(1S)-2-Methoxy-(1-methylethyl)oxyl}-5-[(3-methyl-1,2,4-thiadiazol-2-ylamino)carbonyl]phenyl}oxy)benzoic acid

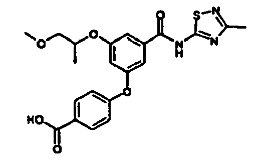

A solution of ethyl 4-({3-{[(1*S*)-2-methoxy-(1-methylethyl)oxy}-5-[(3-methyl-1,2,4-thiadiazol-2-ylamino)carbonyl] phenyl}oxy)benzoate (1.3g) in THF (40 mL) was added to a solution of lithium hydroxide monohydrate (310 mg) in water (20 mL). The mixture was stirred at ambient temperature for 16 hours and the THF removed *in vacuo.* The aqueous layer was acidified with 1M hydrochloric acid (6.9 mL), and the solid precipitate filtered off, washed with water and dried *in vacuo* to give the desired compound (1.12 g).
¹H NMR δ (d₆-DMSO): 1.2 (d, 3H), 2.45 (s, 3H), 3.25 (s, 3H), 3.5 (m, 2H), 4.7-4.8 (m, 1H), 6.95 (s, 1H), 7.1 (d, 2H), 7.35 (s, 1H), 7.6 (s, 1H), 7.95 (d, 2H); m/z 444 (M+H)⁺

### Ethyl 4-({3-{[(1S)-2-methoxy-(1-methylethyl)oxy}-5-[(3-methyl-1,2,4-thiadiazal-2-ylamino)carbonyl] phenyl}oxy)benzoate

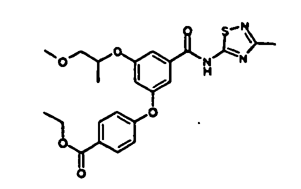

A solution of 3-hydroxy-5-{[(1*S*)-2-methoxy-(1-methylethyl)oxy}-*N*-(3-methyl-1,2,4-thiadiazol-2-yl)benzamide (3.23 g), 4-ethoxycarbonylphenylboronic acid (3.63 g), copper (II) acetate (3.63 g), triethylamine (6.9 mL) and freshly activated 4A molecular sieves (12.5g) in DCM (250 mL) was stirred at ambient temperature and under ambient atmosphere for 2 days. The reaction mixture was filtered through diatomaceous earth, washed with DCM (2 x 50 mL), the DCM removed *in vacuo* and the residual oil partitioned between ethyl acetate (300 mL) and 1M hydrochloric acid (200 mL). The ethyl acetate layer was separated, washed sequentially with aqueous sodium hydrogen carbonate solution and brine, dried (MgSO₄), and evaporated to a residue which was chromatographed on silica with 40% ethyl acetate in isohexane as eluant to give the desired compound (1.35 g).
¹H NMR δ (CDCl₃): 1.3 (d, 3H), 1.4 (t, 3H), 2.45 (s, 3H), 3.4 (s, 3H), 3.5-3.6 (m, 2H), 4.35 (q, 2H), 4.5-4.6 (m, 1H), 6.85 (s, 1H), 7.0 (d, 2H), 7.1 (s, 1H), 7.3 (d, 1H), 8.05 (d, 2H), 10.5 (s, 1H); *m*/*z* 472 (M+H)⁺

### 3-Hydroxy-5-{[(1S)-2-methoxy-(1-methylethyl)oxyl-N-(3-methyl-1,2,4-thiadiazol-2-yl)benzamide

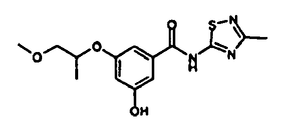

A solution of 3-{[(1S)-2-methoxy-(1-methylethyl)oxy}-5-{phenylmethyloxy}-*N*-(3-methyl-1,2,4-thiadiazol-2-yl)benzamide (9.53 g) and thioanisole (13.9 mL) in trifluoroacetic acid (45 mL) was stirred at ambient temperature for 16 hours. The trifluoroacetic acid was removed in *vacuo* and the residual oil partitioned between ethyl acetate (100 mL) and aqueous sodium hydrogen carbonate solution (300 mL). The aqueous layer was separated, extracted with ethyl acetate (2 x 100 mL), and the combined organic extracts washed with brine, dried (MgSO₄), and evaporated to a residue which was chromatographed on silica with 50% ethyl acetate in isohexane as eluant to give the desired compound (4.5 g).
¹H NMR δ (CDCl₃): 1.2 (d, 3H), 2.5 (s, 3H), 3.3 (s, 3H), 3.4-3.6 (m, 2H), 4.6-4.7 (m, 1H), 6.6 (s, 1H), 7.05 (s, 1H), 7.1 (s, 1H), 9.85 (s, 1H), 13.2 (s, 1H); *m*/*z* 324 (M+H)⁺

### 3-{[(1S)-2-Methoxy-(1-methylethyl)oxy}-5-{phenylmethyloxy}-N-(3-methyl-1,2,4-thiadiazol-2-yl)benzamide

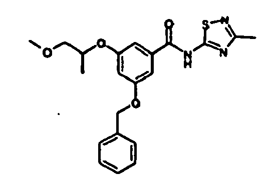

To a solution of 3-[(1*S*)-2-methoxy-(1-methylethyl)oxy]-5-{[phenylmethyl]oxy}benzoic acid (15.8 g) in DCM (260 mL) was added oxalyl chloride (5.24 mL), followed by DMF (1 drop), and the mixture stirred at ambient temperature for 16 hours. The DCM and excess oxalyl chloride were removed *in vacuo,* the residual oil dissolved in DCM (50 mL) and added to a solution of 5-amino-3-methyl-1,2,4 thiadiazole (6.05 g) and triethylamine (14.6 mL) in DCM (150 mL) at 0-5°C, and the mixture stirred at ambient temperature for 16 hours. The DCM and excess triethylamine were removed *in vacuo,* and the residual oil partitioned between ethyl acetate (250 mL) and 1M hydrochloric acid (150 mL). The ethyl acetate layer was separated, washed sequentially with 1 M hydrochloric acid, aqueous sodium hydrogen carbonate solution, and brine, dried (MgSO₄), and evaporated to a residue which was chromatographed on alumina with ethyl acetate as eluant, then on silica with 30% ethyl acetate in isohexane as eluant to give the desired compound (9.6 g).
¹H NMR δ (CDCl₃): 1.3 (d, 3H), 2.45 (s,3H), 3.4 (s, 3H), 3.5-3.6 (m, 2H), 4.55-4.6 (m, 1H), 5.05 (s,2H), 6.8 (s, 1H), 7.1 (m, 2H), 7.25 (m, 5H), 10.7 (s, 1H); *m*/*z* 414 (M+H)⁺

The synthesis of 3-[(1*S*)-2-methoxy-(1-methylethyl)oxy]-5-{[phenylmethyl]oxy}benzoic acid is described in **Example 3** above.

### Example 18:3-[4-(Azetidin-1-ylcarbonyl)-2-chlorophenoxyl-5-[(1S)-2-methoxy-(1-methylethyl)oxy]-N-(1-methyl-1H-pyrazol-3-yl)benzamide

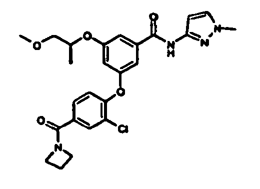

To a suspension of 3-chloro-4-[(3-{[(1*S*)-2-methoxy-(1-methylethyl)oxy}-5-{[(1-methyl-1*H-*pyrazol-3-yl)amino]carbonyl}phenyl)oxy]benzoic acid (344 mg), HATU (366 mg) and azetidine hydrochloride (88 mg) in DMF (10 mL), was added DIPEA (0.50 mL) and the mixture stirred at ambient temperature for 24 hours. Water (30 mL) was added and the mixture extracted with ethyl acetate (3x15 mL). The combined organic extracts were washed with brine, dried (MgSO₄), and evaporated to a residue which was chromatographed on silica eluting with a gradient of 50-100% ethyl acetate in hexane to give the desired compound (197 mg).
¹H NMR δ (CDCl₃): 1.3 (d, 3H), 2.4 (m, 2H), 3.4 (s, 3H), 3.5 (m, 2H), 3.8 (s, 3H), 4.2-4.4 (m, 4H), 4.6 (m, 1H), 6.7 (d, 2H), 7.0 (m, 2H), 7.2 (m, 2H), 7.5 (d, 1H), 7.8 (d, 1H), 8.60 (br s, 1H); *m*/*z* 499 (M+H)⁺

In a similar manner, **Examples 18a-c and Reference Examples 18 d-e** were also prepared:-

| **Example/ Reference Example** | **Structure** | ***m*/*z*** | **NMR** |
|---|---|---|---|
| **18a** | 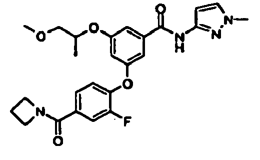 | 483 (M+H)⁺ | ¹H NMR δ (CDCl₃): 1.3 (d, 3H), 2.4 (m, 2H), 3.4 (s, 3H), 3.5 (m, 2H), 3.8 (s, 3H), 4.2-4.4 (m, 4H), 4.6 (m, 1H), 6.8 (m, 2H), 7.0 (m, 2H), 7.2 (m, 1H), 7.3 (m, 1H), 7.4 (d, 1H), 7.5 (d, 1H), 8.50 (s, 1H) |
| **18b** | 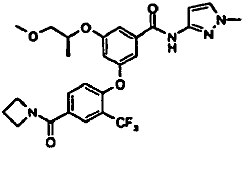 | 533 (M+H)⁺. | ¹H NMR δ (CDCl₃): 1.3 (d, 3H), 2.4 (m, 2H), 3.4 (s, 3H), 3.5 (m, 2H), 3.8 (s, 3H), 4.2-4.4 (m, 4H), 4.6 (m, 1H), 6.8 (m, 2H), 6.95 (d,1H), 7.1 (s, 1H), 7.3 (m, 2H), 7.75 (d, 1H), 8.0 (s, 1H), 8.50 (s, 1H) |
| **18c** | 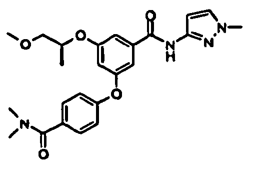 | 453 (M+H)⁺ 451 (M-H)⁻ | ¹H NMR δ (DMSO-d6): 1.2 (d, 3H), 2.95 (s, 6H), 3.25 (s, 3H), 3.5 (m, 2H), 3.8 (s, 3H), 4.75 (m, 1H), 6.55 (s, 1H), 6.8 (s, 1H), 7.05 (d, 2H), 7.2 (s, 1H), 7.4 (s, 1H), 7.45 (d, 2H), 7.6 (s, 1H), 10.85 (br s, 1H) |
| **18d** | 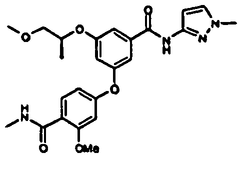 | 469 (M+H)⁺ | ¹H NMR δ (CDCl₃): 1.3 (d, 3H), 3.0 (d, 3H), 3.4 (s, 3H), 3.45-3.6 (m, 2H), 3.8 (s, 3H), 3.95 (s, 3H), 4.6 (m, 1H), 6.6 (m, 2H), 6.8 (m, 2H), 7.05 (s, 1H), 7.25 (m, 2H), 7.7 (b, 1H), 8.2 (d, 1H), 8.4 (b, 1H) |
| **18e** | 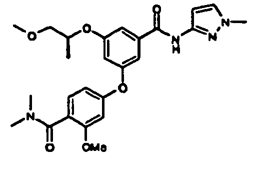 | 483 (M+H)⁺ | ¹H NMR δ (CDCl₃): 1.3 (d, 3H), 2.9 (s, 3H), 3.1 (s, 3H), 3.4 (s, 3H), 3.45-3.6 (m, 2H), 3.8 (s, 6H), 4.6 (m, 1H), 6.6 (m, 2H), 6.8 (m, 2H), 7.1 (s, 1H), 7.2 (m, 2H), 7.3 (m, 1H), 8.5 (b, 1H) |

The required acids for the preparation of **Examples 18a-c and Reference Examples 18 d-e** were prepared as described below:

### 3-Chloro-4-[(3-((1S)-2-methoxy-(1-methylethyl)oxyl)-5-{[(1-methyl-1H-pyrazol-3-yl)amino]carbonyl}phenyl)oxy]benzoic acid

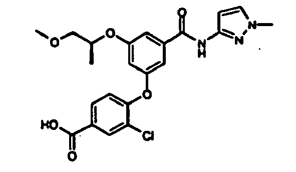

To a solution of methyl 3-chloro-4-(3-[(1*S*)-2-methoxy-(1-methylethyl)oxy]-5-{[(1-methyl-1*H*-pyrazol-3-yl)amino]carbonyl}phenoxy)benzoate (2.23 g) in THF (58 mL) was added a 1M solution of lithium hydroxide monohydrate in water (11.7 mL). The mixture was stirred at ambient temperature for 18 hours and the THF removed *in vacuo.* The aqueous layer was acidified with 2M hydrochloric acid (5.85 mL), and the solid precipitate filtered off, washed with water and dried in *vacuo* to give the desired acid (1.87g).
¹H NMR δ (CDCl₃): 1.4 (d, 3H), 3.4 (s, 3H), 3.6 (m, 2H), 3.8 (s, 3H), 4.7 (m, 1H), 6.95 (m, 1H), 7.05 (m, 1H), 7.1 (d, 1H), 7.3 (m, 2H), 7.6 (m, 2H), 8.1 (s, 1H), 10.75 (br s, 1H); *m*/*z* 460 (M+H)⁺

The acids required for the synthesis of **Examples 18a-c and Reference Examples 18 d-e** were made using an analogous method:

| **Structure** | ***m*/*z*** | **NMR** |
|---|---|---|
| 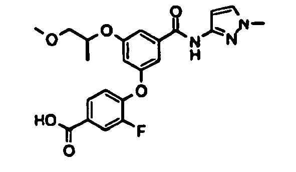 | 444 (M+H)⁺ | ¹H NMR δ (CDCl₃): 1.4 (d, 3H), 3.4 (s, 3H), 3.5 (m, 2H), 3.8 (s, 3H), 4.7 (m, 1H), 6.95 (m, 1H), 7.0 (m, 1H), 7.1 (m, 1H), 7.3 (m, 2 H), 7.6 (m, 2H), 7.8 (d, 1H), 10.8 (s, 1H) |
| 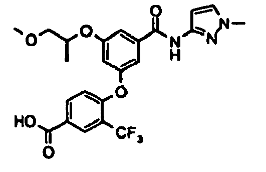 | 494 (M+H)⁺ | ¹H NMR δ (d₆DMSO): 1.2 (d, 3H), 3.3 (s, 3H), 3.5 (m, 2H), 3.8 (s, 3H), 4.7 (m, 1H), 6.5 (s, 1H), 6.8 (s, 1H), 7.05 (d, 1H), 7.2 (s, 1H), 7.5 (s, 1H), 7.6 (s, 1H), 8.1 (d, 1H), 8.5 (s, 1H), 10.85 (s, 1H) |
| 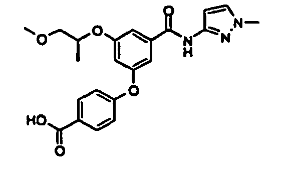 | 426 (M+H)⁺ 424 (M-H)⁻ | ¹H NMR δ (d₆DMSO): 1.2 (d, 3H), 3.25 (s, 3H), 3.5 (m, 2H), 3.8 (s, 3H), 4.75 (m, 1H), 6.55 (s, 1H), 6.85 (s, 1H), 7.1 (d, 2H), 7.2 (s, 1H), 7.4 (s, 1H), 7.6 (s, 1H), 8.0(d, 2H), 10.85 (br s, I H) |
| 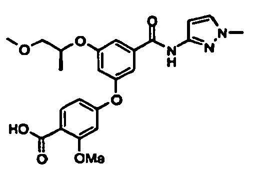 | 456 (M+H)⁺ | ¹H NMR δ (CDCl₃): 1.35 (d, 3H), 3.4 (s, 3H), 3.45-3.6 (m, 2H), 3.8 (s, 3H), 3.95 (s, 3H), 4.6 (m, 1H), 6.7 (m, 2H), 6.85 (m, 2H), 7.25 (s, 1H), 7.3 (s, 1H), 7.35 (s, 1H), 7.95 (d, 1H), 9.3 (b, 1H) |

The required esters for **Examples 18a-c and Reference Examples 18 d** were prepared as described below:

### Methyl 3-chloro-4-(3-[(1S)-2-methoxy-(1-methylethyl)oxy]-5-{[(1-methyl-1H-pyrazol-3-yl)amino]carbonyl}phenoxy)benzoate

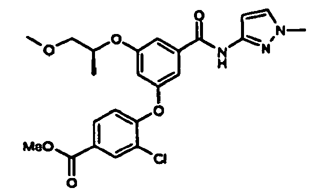

To a solution of 3-hydroxy-5-[(1*S*)-2-methoxy-(1-methylethyl)oxy]-*N*-(1-methyl-1*H*-pyrazol-3-yl)benzamide (832 mg, 2.72 mmol) and methyl-3-chloro-4-fluorobenzoate (504 mg, 2.72 mmol) in acetonitrile (20 mL) was added potassium carbonate (364 mg, 2.72 mmol) and the stirred mixture heated at 160°C in a 'Smith Creator Microwave' for 30 minutes. The mixture was allowed to return to ambient temperature and pressure, filtered and evaporated to a residue which was chromatographed on silica with 0 -50% ethyl acetate in hexane as eluant to give the desired compound (1.11 g).
*m*/*z* 474 (M+H)⁺

The esters required for the synthesis of **Examples 18a-c and Reference Examples 18 d-e** were prepared using an analogous method:

| **Structure**^{$} | ***m*/*z*** | **NMR** |
|---|---|---|
| 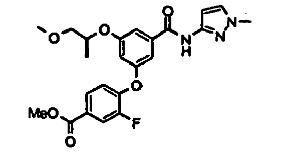 | 458 (M+H)⁺ | ¹H NMR δ (CDCl₃): 1.3(d, 3H), 3.4 (s, 3H), 3.5 (m, 2H), 3.8 (s, 3H), 3.9 (s, 3H), 4.6 (m, 1H), 6.9 (m, 2H), 7.1 (m, 2H), 7.3 (m, 2H), 7.8 (m, 2H), 8.6 (s, 1H) |
| 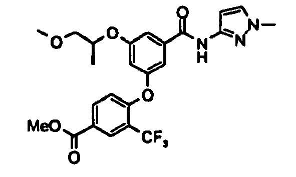 | 508 (M+H)⁺ | ¹H NMR δ (CDCl₃): 1.3 (d, 3H), 3.4 (s, 3H), 3.5 (m, 2H), 3.8 (s, 3H), 3.95 (s, 3H), 4.6 (m, 1H), 6.75 (d, 1H), 6.85 (m, 1H), 6.95 (d, 1H), 7.1 (m, 1H), 7.3 (m, 1H), 7.35(m, 1H), 8.1(d, 1H), 8.4 (s, 1H), 8.5 (s, I H) |
| 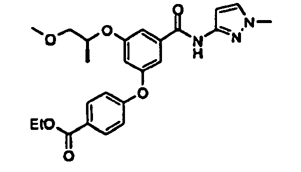 | 454 (M+H)⁺ | ¹H NMR δ (d₆DMSO): 1.2 (d, 3H), 1.3 (t, 3H), 3.25 (s, 3H), 3.5 (m, 2H), 3.8 (s, 3H), 4.3 (q, 2H), 4.8 (m, 1H), 6.55 (s, 1H), 6.8 (s, 1H), 7.1 (d, 2H), 7.2 (s, 1H), 7.45 (s, 1H), 7.6 (s, 1H), 8.0(d, 2H), 10.85 (br s, 1H) |
| 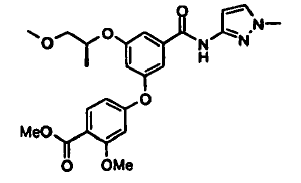 | 470 (M+H)⁺ | ¹H NMR δ (CDCl₃): 1.3 (d, 3H), 3.4 (s, 3H), 3.45-3.6 (m, 2H), 3.8 (s, 3H), 3.95 (m, 6H), 4.6 (m, 1H), 6.55 (d, 1H), 6.65 (s, 1H), 6.8 (m, 2H), 7.1 (s, 1H), 7.25 (m, 2H), 7.8 (d, 1H), 8.4 (b, 1H) |

| | | |
|---|---|---|
| ^{$}The precursor for **Example 18c** was prepared at 150°C in DMF for 4 hours, using 1.2 equivalents of the fluoro-ester. The precursor for Reference **Examples 18d**-**e** was prepared at 150°C in DMF for 2 hours. | | |

The methyl 4-fluoro-2-methoxybenzoate used in the preparation of the precursor for Reference **Examples 18d-e** was prepared from 4-fluoro-2-methoxybenzoic acid according to the procedure described in WO98/13332.

The synthesis of 3-hydroxy-5-[(1*S*)-2-methoxy-(1-methylethyl)oxy]-*N*-(1-methyl-1*H*-pyrazol-3-yl)benzamide is described in **Example** 3.

### Reference Example 19: 3-{4-[(Dimethylamino)carbonyl]phenoxy}-5-[(1S)-2-methoxy-1-methylethoxy]-N-1H-pyrazol-3-ylbenzamide

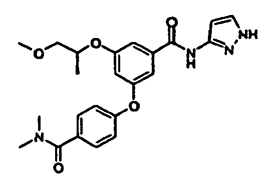

To a suspension of 4-{3-[(1*S*)-2-methoxy-1-methylethoxy]-5-[(1*H*-pyrazol-3-ylamino)carbonyl]phenoxy}benzoic acid (280 mg, 0.55 mmol), HATU (260 mg, 0.685 mmol) and dimethylamine (0.345 mL of 2.0M solution in THF, 0.685 mmol) in DMF (1 mL) was added DIPEA (0.238 mL, 1.37 mmol) and reaction mixture stirred for 16 hours at ambient temperature. Water was then added to reaction mixture and extracted into ethyl acetate (3x25 mL). Organic layer was washed with saturated sodium hydrogen carbonate and saturated brine solution and dried (MgSO₄). Filtrate was concentrated *in vacuo* and residue chromatographed (50-100% ethyl acetate in isohexane) to give a white solid (95 mg; 40%).
¹H NMR δ (d₆-DMSO): 1.2 (d, 3H), 2.95 (s, 6H), 3.3 (s, 3H), 3.5 (m, 2H), 4.75 (m, 1H), 6.6 (s, 1H), 6.8 (s, 1H), 7.05 (d, 2H), 7.2 (s, 1H), 7.4 (d, 2H), 7.45 (s, 1H), 7.6 (s, 1H), 10.8 (s, 1H). *m*/*z* 439 (M+H)⁺

In a similar manner, **Examples 19a and-d and Reference Examples 19 b and c** were also prepared:

| **Example/ Reference Example** | **Structure** | ***m*/*z*** | **NMR** |
|---|---|---|---|
| **19a** | 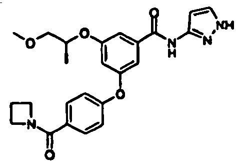 | 451 (M+H)⁺, 449 (M-H)⁻ | is NMR δ (d₆-DMSO): 1.2 (d, 3H), 2.2 (m, 2H), 3.25 (s, 3H), 3.5 (m, 2H), 4.0 (m, 2H), 4.3 (m, 2H), 4.75 (m, 1H), 6.6 (s, 1H), 6.8 (s, 1H), 7.05 (d, 2H), 7.2 (s, 1H), 7.45 (s, 1H), 7.6 (s, 1H), 7.65 (d, 2H), 10.8 (s, 1H), 12.4 (s, br, 1H) |
| **19b** | 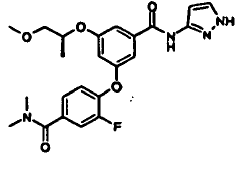 | 457 (M+H)⁺, 455 (M-H)⁺ | ¹H NMR δ (d₆-DMSO): 1.2 (d, 3H), 2.95 (s, 6H), 3.3 (s, 3H obscured by water peak), 3.5 (m, 2H), 4.75 (m, 1H), 6.6 (s, 1H), 6.8 (s, 1H), 7.15 (s, 1H), 7.2 (t, 1H), 7.25 (s, 1H), 7.4 (t, 1H), 7.45 (s, 1H), 7.6 (s, 1H), 10.8 (s, 1H), 12.4 (s br, 1H) |
| **19c** | 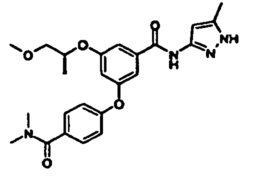 | 453 (M+H)⁺, 451 (M-H)⁺ | ¹H NMR δ (d₆-DMSO): 1.2 (d, 3H), 2.2 (s, 3H), 2.95 (s, 6H), 3.25 (s, 3H obscured by water peak), 3.5 (m, 2H), 4.75 (m, 1H), 6.35 (s, 1H), 6.8 (s, 1H), 7.05 (d, 2H), 7.2 (s, 1H), 7.4 (s, 1H), 7.45 (d, 2H), 10.7 (s, 1H), 12.1 (s br, 1H) |
| **19d** | 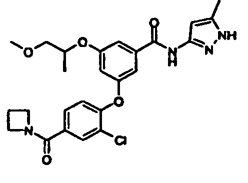 | 499 (M+H)⁺, 497 (M-H)⁺ | ¹H NMR δ (d₆-DMSO): 1.2 (d, 3H), 2.2 (s, 3H), 2.25 (m, 2H), 3.25 (s, 3H obscured by water peak), 3.5 (m, 2H), 4.0 (m, 2H), 4.3 (m, 2H), 4.75 (m, 1H), 6.35 (s, 1H), 6.8 (s, 1H), 7.1 (d, 1H), 7.15 (s, 1H), 7.4 (s, 1H), 7.6 (d, 1H), 7.8 (s, 1H), 10.7 (s, 1H), 12.1 (s br, 1H) |

The required acid for the preparation of Reference **Examples 19** and **Example 19a** was prepared as described below:

### 4-{3-[(1S)-2-Methoxy-1-methylethoxy]-5-[(1H-pyrazol-3-ylamino)carbonyl]phenoxy}benzoic acid

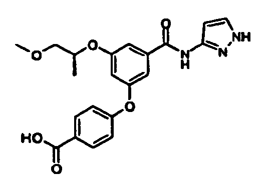

To a solution of *tert*-butyl 3-({3-[4-(ethoxycarbonyl)phenoxy]-5-[(1*S*)-2-methoxy-1-methylethoxy]benzoyl}amino)-1*H*-pyrazole-1-carboxylate (1.75 g, 3.25 mmol) in THF (16 mL) and water (8 mL) was added 1M solution of sodium hydroxide (16 mL; 5.0equiv) and reaction mixture allowed to stir at room temperature for 16 hours. The THF was removed *in vacuo* and 1M citric acid added until pH 3-4. Pale yellow precipitate was filtered off and washed with water to give a pale yellow solid which was dried *in vacuo* (1.18g, 71%).
¹H NMR δ (d₆-DMSO): 1.2 (d, 3H), 3.25 (s, 3H obscured by water peak), 3.4-3.5 (m, 2H), 4.75 (m, 1H), 6.55 (s, 1H), 6.85 (s, 1H), 7.1 (d, 1H), 7.25 (s, 1H), 7.45 (s, 1H), 7.6 (d, 1H), 7.95 (d, 1H), 10.85 (s, 1H); *m*/*z* 412 (M+H)⁺.

The acids required for the preparation of Reference **Examples 19b-c and Example d** were prepared in a similar manner:

| **Structure** | ***m*/*z*** | **NMR** |
|---|---|---|
| 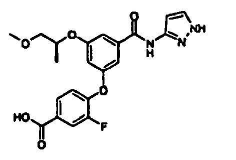 | | ¹H NMR (d₆-DMSO): 1.2 (d, 3H), 3.25 (s, 3H obscured by water peak), 3.5 (m, 2H), 4.75 (m, 1H), 6.6 (d, 1H), 6.85 (s, 1H), 7.2 (s, 1H), 7.25 (t, 1H), 7.4 (s, 1H), 7.6 (d, 1H), 7.8 (d, 1H), 7.85 (d, 2H), 10.8 (brs, 1H) |
| 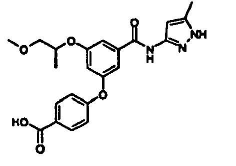 | | ¹H NMR (d₆-DMSO): 1.2 (d, 3H), 2.2 (s, 3H), 3.25 (s, 3H obscured by water peak), 3.5 (m, 2H), 4.75 (m, 1H), 6.35 (s, 1H), 6.85 (s, 1H), 7.1 (d, 2H), 7.22 (s, 1H), 7.42 (s, 1H), 7.95 (d, 2H), 10.7 (s, 1H) |
| 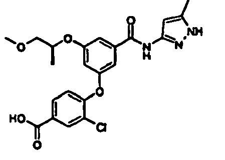 | | ¹H NMR (d₆-DMSO): 1.2 (d, 3H), 2.2 (s, 3H), 3.25 (s, 3H obscured by water peak), 3.4 (m, 2H), 4.6 (m, 1H), 6.4 (s, 1H), 6.7 (s, 1H), 6.9 (d, 1H), 7.1 (s, 1H), 7.3 (s, 1H), 7.7 (dd, 1H), 7.95 (d, 1H), 10.0 (s, 1H) |

The ester required for the preparation of Reference **Examples 19** and **Example 19a** was prepared as follows:

### tert-Butyl 3-({3-[4-(ethoxycarbonyl)phenoxy]-5-[(1S)-2-methoxy-1-methylethoxy]benzoyl}amino)-1H-pyrazole-1-carboxylate

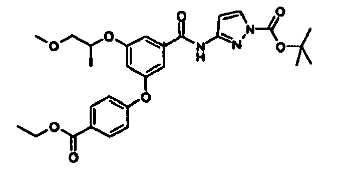

*tert*-Butyl 3-({3-hydroxy-5-[(1*S*)-2-methoxy-1-methylethoxy]benzoyl}amino)-1*H*-pyrazole-1-carboxylate (391 mg, 1 mmol), ethyl-4-boronic acid benzoate (388 mg, 2.0 equiv), copper (II) acetate (363 mg, 2.0 equiv) and triethylamine (0.7 mL; 5.0 equiv) were suspended in dry DCM over freshly activated powdered 4A molecular sieves (ca. 1 g) for 7 hours under an ambient atmosphere. Reaction mixture filtered through diatomaceous earth was washed with DCM (x3). Filtrate concentrated *in vacuo,* taken up in ethyl acetate and washed with 1M hydrochloric acid, saturated sodium hydrogen carbonate, saturated brine and dried (MgSO₄). Filtered, filtrate concentrated *in vacuo* and chromatographed (0-50% ethyl acetate/isohexane) to give a brown oil (210 mg, 39%)
¹H NMR δ (CDCl₃): 1.3 (d, 3H), 1.4 (t, 3H), 1.6 (s, 9H), 3.4 (s, 3H), 3.5 (m, 2H), 4.35 (q, 2H), 4.5 (m, 1H), 6.8 (s, 1H), 7.0 (d, 2H), 7.05 (s, 2H), 7.2 (s, 1H), 8.0 (s, 1H), 8.05 (d, 2H), 9.2 (s, br, 1H); *m*/*z* 440 (M+H)⁺.

### tert-Butyl 3-({3-hydroxy-5-[(1S)-2-methoxy-1-methylethoxy]benzoyl}amino)-1H-pyrazole-1carboxylate

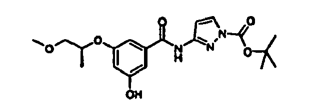

A solution of tert-butyl 3-({3-(benzyloxy)-5-[(1*S*)-2-methoxy-1-methylethoxy]benzoyl}amino)-1*H*-pyrazole-1-carboxylate (23 g, 47.8 mmol) in THF (140 mL) and ethanol (140 mL) was evacuated and purged with nitrogen (x3). 10% Palladium on carbon (2.3 g; 10% w/w) was added and reaction mixture was evacuated and finally purged with hydrogen gas. Reaction mixture was left to stir at ambient temperature under a hydrogen balloon for 16 hours. Pd/C was filtered through diatomaceous earth and the filtrate concentrated *in vacuo* to give a white foam (18 g, 97%).
¹H NMR δ (d₆-DMSO): 1.2 (d, 3H), 1.55 (s, 9H), 3.25 (s, 3H obscured by water peak), 3.4-3.5 (m, 2H), 4.7 (m, 1H), 6.5 (s, 1H), 6.95 (d, 1H), 7.0 (s, 1H), 7.1 (s, 1H), 8.2 (d, 1H), 9.65 (s, 1H), 11.2 (s, br, 1H); m/z 392 (M+H)⁺

### tert-Butyl 3-({3-(benzyloxy)-5-[(1S)-2-methoxy-1-methylethoxy]benzoyl}amino)-1H-pyrazole-1-carboxylate

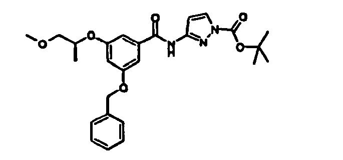

To a suspension of 3-[(1*S*)-2-methoxy-(1-methylethyl)oxy]-5-{[phenylmethyl]oxy}benzoic acid (20.7 g, 65.6 mmol), HATU (31.2 g, 82.0 mmol) and tert-butyl 3-amino-1*H*-pyrazole-1-carboxylate (15.0 g, 82.0 mmol) in DMF (30 mL) was added DIPEA (28.5 mL, 164 mmol) and reaction mixture stirred for 16 hours at ambient temperature. Water (250 mL) was then added to reaction mixture and extracted into diethyl ether (3x150 mL). Organic layer was washed with saturated brine solution and dried (MgSO₄). Filtrate was concentrated *in vacuo* and residue crystallised on standing. Washed with isohexane to give yellow crystals (23.4 g; 73%).
*m*/*z* 482 (M+H)⁺.

The preparation of 3-[(1*S*)-2-methoxy-(1-methylethyl)oxy]-5-{[phenylmethyl]oxy}benzoic acid was described in **Example 3.**

The preparation of *tert*-butyl 3-amino-1*H-*pyrazole-1-carboxylate was described in **Example 14**.

The ester required for the preparation of **Reference Example 19b** was prepared as follows:

### Ethyl 3-fluoro-4-{3-[(1S)-2-methoxy-1-methylethoxy]-5-[(1H-pyrazol-3-ylamino)carbonyl]phenoxy}benzoate

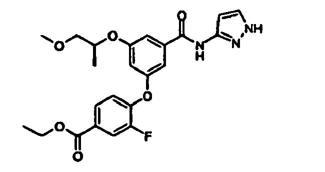

To a suspension of *tert*-butyl 3-({3-hydroxy-5-[(1*S*)-2-methoxy-1-methylethoxy]benzoyl}amino)-1*H*-pyrazole-1-carboxylate (587 mg, 1.5 mmol), caesium carbonate (488 mg, 1.5 mmol) in DMA (3 mL) was added ethyl 3,4-difluorobenzoate (279 mg, 1.5 mmol). This mixture was heated at 110°C for 16 hours. The reaction mixture was filtered and concentrated *in vacuo* then the residue chromatographed on silica, eluting with 0-70% ethyl acetate in hexane, to give the desired compound as a yellow oil (271 mg, 40%)
¹H NMR (CDCl₃): 1.3 (d, 3H), 1.4 (t, 3H), 3.4 (s, 3H), 3.5 (m, 2H), 4.4 (q, 2H), 4.6 (m, 1H), 6.75 (s, 1H), 6.85 (s, 1H), 7.1 (s, 1H), 7.15 (s, 1H), 7.3 (s, 1H), 7.5 (d, 1H), 7.8 (d, 1H), 7.85 (d, 1H), 9.4 (s, 1H)

The preparation of tert-butyl 3-({3-hydroxy-5-[(1*S*)-2-methoxy-1-methylethoxy]benzoyl}amino)-1*H*-pyrazole-1-carboxylate was described in **Reference Example 19.**

The ester required for the preparation of **Reference Example 19c** was prepared as follows:

### tert-Butyl 3-({3-[4-(ethoxycarbonyl)phenoxy]-5-[(1S)-2-methoxy-1-methylethoxy]benzoyl}amino)-5-methyl-1H-pyrazole-1-carboxylate

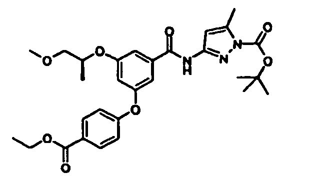

Freshly activated 4Å molecular sieves (1.5 g) were added to a solution of *tert*-butyl 3-({3-hydroxy-5-[(1*S*)-2-methoxy-1-methylethoxy]benzoyl}amino)-5-methyl-1*H*-pyrazole-1-carboxylate (1.0 g, 2.47 mmol), (4-ethoxycarbonylphenyl)boronic acid (718 mg, 3.7 mmol), copper (II) acetate (672 mg, 3.7 mmol) and triethylamine (1.7 mL, 12.3 mmol) in DCM (40 mL). The mixture was stirred at ambient temperature for 2 days then filtered through diatomaceous earth and the DCM removed *in vacuo.* The residual oil was partitioned between ethyl acetate (35 mL) and IN hydrochloric acid (35 mL), the ethyl acetate layer separated, washed with saturated aqueous sodium hydrogen carbonate solution (35 mL), brine (35 mL), dried (MgSO₄) and evaporated to a residue which was chromatographed on silica, eluting with 40 - 60% ethyl acetate in hexane, to give the desired compound as an orange oil (80mg, 6%). ¹H NMR (CDCl₃): 1.3 (d, 3H), 1.4 (t, 3H), 1.6 (s, 9H), 2.55 (s, 3H), 3.4 (s, 3H), 3.5 (m, 2H), 4.4 (q, 2H), 4.6 (m, 1H), 6.8 (s, 1H), 6.9 (s, 1H), 7.05 (d, 2H), 7.2 (s, 1H), 7.35 (s, 1H), 8.05 (d, 2H), 9.4 (s, 1H)

*tert*-Butyl 3-({3-hydroxy-5-[(1*S*)-2-methoxy-1-methylethoxy]benzoyl}amino)-5-methyl-1*H-*pyrazole-1-carboxylate was prepared in an analogous fashion to *tert*-butyl 3-({3-hydroxy-5-[(1*S*)-2-methoxy-1-methylethoxy]benzoyl}amino)-1*H*-pyrazole-1-carboxylate, described in **Reference Example 19**, starting from 3-[(1*S*)-2-methoxy-(1-methylethyl)oxy]-5-{[phenylmethyl]oxy}benzoic and *tert*-butyl 3-amino-5-methyl-1*H*-pyrazole-1-carboxylate. *tert*-Butyl 3-({3-hydroxy-5-[(1*S*)-2-methoxy-1-methylethoxy]benzoyl}amino)-5-methyl-1*H-*pyrazole-1-carboxylate 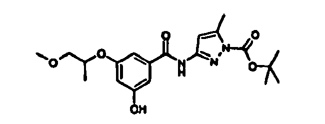 ¹H NMR δ (d₆-DMSO): 1.2 (d, 3H), 1.55(s, 9H), 3.2 -3.3 (s, 3H obscured by water peak), 3.2 -3.3 (s, 3H obscured by water peak), 3.4 - 3.5 (m, 2H), 4.65 (m, 1H), 6.45 (s, 1H), 6.75 (s, 1H), 6.95 (s, 1H), 7.1 (s, 1H), 9.65 (s, 1H), 11.05 (brs, 1H); *m*/*z* 406 (M+H)⁺.

### tert-Butyl 3-({3-(benzyloxy)-5-[(1S)-2-methoxy-1-methylethoxy]benzoyl}amino)-5-methyl-1H-pyrazole-1-carboxylate

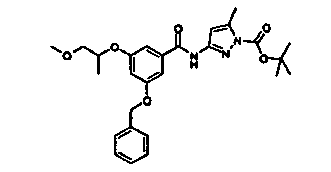

¹H NMR δ (d₆-DMSO): 1.2 (d, 3H), 1.55 (s, 9H), 3.25 (s, 3H obscured by water peak), 3.4-3.5 (m, 2H), 4.7 (m, 1H), 5.15 (s, 2H), 6.7 (s, 1H), 6.8 (s, 1H), 7.2 (s, 1H), 7.25 (s, 1H), 7.3 - 7.5 (m, 5H), 11.15 (brs, 1 H); *m*/*z* 496 (M+H)⁺.

The ester required for the preparation of **Example 19d** was prepared as follows:

### Ethyl 3-chloro-4-(3-[(1S)-2-methoxy-1-methylethoxy]-5-{[(5-methyl-1H-pyrazol-3-yl)amino]carbonyl}phenoxy)benzoate

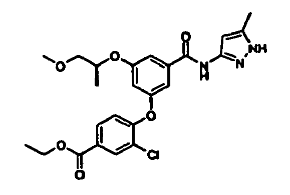

Ethyl 3-chloro-4-fluorobenzoate (242 mg, 1.2 mmol) was added to a suspension of *tert*-butyl 3-({3-hydroxy-5-[(1*S*)-2-methoxy-1-methylethoxy]benzoyl}amino)-5-methyl-1*H*-pyrazole-1-carboxylate (405 mg, 1 mmol) and potassium carbonate (1 mmol) in butyronitrile (5 mL). This mixture was placed in a microwave and heated at 190°C for 2.5 hours. The reaction mixture was concentrated *in vacuo* and the residue partitioned between ethyl acetate and water then extracted with ethyl acetate (3 x 25 mL). The organics were dried (MgSO₄) and concentrated *in vacuo.* The crude mixture (420mg 86%) was used in the next step without further purification.

The preparation of tert-butyl 3-({3-hydroxy-5-[(1*S*)-2-methoxy-1-methylethoxy]benzoyl}amino)-5-methyl-1*H*-pyrazole-1-carboxylate was described in **Reference Example 19c**. The preparation of ethyl 3-chloro-4-fluorobenzoate is described in the literature (Journal of Fluorine Chemistry, 1991, 53(2), 301-305).

### Reference Example 20 : 3-[4-(Ethylsulfonyl)-2-fluorophenoxyl-5-[(1S)-2-methoxy-1-methylethoxy]-N-1H-pyrazol-3-ylbenzamide

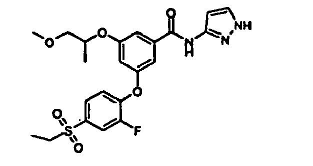

A suspension of *tert*-butyl 3-({3-hydroxy-5-[(1*S*)-2-methoxy-1-methylethoxy]benzoyl}amino)-1*H*-pyrazole-1-carboxylate (391 mg, 1 mmol), cesium carbonate (325 mg, 1 mmol) and 3,4-difluorophenyl ethyl sulfone (206 mg, 1 mmol) in DMA (3 mL) was heated to 120°C for 4 hours. Water (20 mL) was added to the reaction mixture and then was extracted into ethyl acetate (3x30 mL) and washed with brine. Organic phase was dried (MgSO₄), concentrated *in vacuo* and residue chromatographed (50-100% ethyl acetate/isohexane) to give a white solid (120 mg, 25%).
¹H NMR δ (d₆-DMSO): 1.1 (t, 3H), 1.2 (d, 3H), 3.25 (s, 3H), 3.3 (q, 2H), 3.5 (m, 2H), 4.75 (m, 1H), 6.6 (s, 1H), 6.95 (s, 1H), 7.3 (s, 1H), 7.35 (t, 1H), 7.45 (s, 1H), 7.6 (s, 1H), 7.7 (d, 1H), 7.95 (dd, 1H), 10.8 (s, br 1H). *m*/*z* 477 (M+H)⁺

The following reference examples 20a and 20b, and example 20c were prepared in an analogous fashion.

| **Example/ Reference Example** | **Structure** | ***m*/*z*** | **NMR** |
|---|---|---|---|
| **20a** | 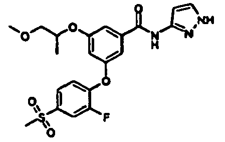 | 463 (M+H)⁺ | ¹H NMR δ (d₆-DMSO): 1.2 (d, 3H), 3.25 (s, 3H), 3.5 (m, 2H), 4.75 (m, 1H), 6.6 (s, 1H), 6.95 (s, 1H), 7.25 (s, 1H), 7.35 (t, 1H), 7.45 (s, 1H), 7.6 (s, 1H), 7.75 (d, 1H), 8.0 (dd, 1H), 10.8 (s, br 1H) |
| **20b** | 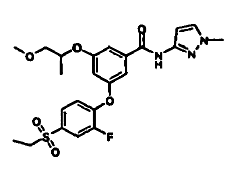 | 492 (M+H)⁺ | ¹H NMR δ (d₆-DMSO): 1.11 (t, 3H), 1.23 (d, 3H), 3.27 (s, 3H), 3.35 (assumed q, 2H, hidden under H₂O), 3.46 (m, 2H), 3.76 (s, 3H), 4.76 (m, 1H), 6.54 (m, 1H), 6.94 (m, 1H), 7.26 (m, 1H), 7.32 (t, 1H), 7.46 (m, 1H), 7.58 (m, 1H), 7.71 (d, 1H), 7.92 (dd, 1H), 7.88 (s, br, 1H) |
| **20c** | 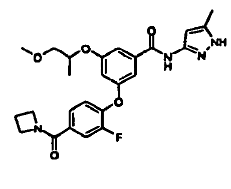 | 483 (M+H)⁺ 481 (M-H)⁻ | ¹H NMR δ (d₆-DMSO): 1.2 (d, 3H), 2.2 (s, 3H), 2.25 (m, 2H), 3.25 (s, 3H), 3.45 (m, 2H), 4.05 (s, br, 2H), 4.35 (s, br, 2H), 4.75 (m, 1H), 6.35 (s, 1H), 6.8 (s, 1H), 7.15 (s, 1H), 7.2 (t, 1H), 7.4 (s, 1H), 7.5 (d, 1H), 7.6 (app d, 1H), 10.7 (s, 1H), 12.1 (s, 1H) |

The preparation of *tert*-butyl 3-({3-hydroxy-5-[(1*S*)-2-methoxy-1-methylethoxy]benzoyl}amino)-1*H*-pyrazole-1-carboxylate used in the preparation of Reference **Examples 20** and **20a** was described in **Example 19**.

The preparation of 3-hydroxy-5-[(1*S*)-2-methoxy-(1-methylethyl)oxy]-*N*-(1-methyl-1*H-*pyrazol-3-yl)benzamide used in the preparation of Reference **Example 20b** was described in **Example 3**

The preparation of *tert*-butyl 3-({3-hydroxy-5-[(1*S*)-2-methoxy-1-methylethoxy]benzoyl}amino)-5-methyl-1*H*-pyrazole-1-carboxylate used in the preparation of **Example 20c** was described in **Example 19**.

3,4-Difluorophenyl ethyl sulfone used in the preparation of Reference **Example 20** and **20b** was prepared as described below.

### 3,4-Difluorophenyl ethyl sulfone

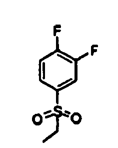

To a solution of 3,4-difluorophenyl ethyl sulfide (1.50 g) in DCM (50 mL) was added 75% m-chloroperbenzoic acid (2.97 g) and the mixture stirred at ambient temperature for 16h. The mixture was washed successively with saturated potassium carbonate (20 mL) and brine (30 mL) then dried with magnesium sulphate, filtered and reduced *in vacuo.* The resultant clear oil was chromatographed on silica (eluting with 0-50% ethyl acetate in iso-hexane) and the faster running product isolated (0.90 g). The required 3,4-difluorophenyl ethyl sulfone was used without further characterisation.

3,4-Difluorophenyl methyl sulfone used in the preparation of Reference **Example 20a** was prepared in an analogous manner from 3,4-difluorophenyl methyl sulfide.

| **Structure** | **m/z** | **NMR** |
|---|---|---|
| 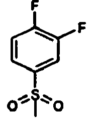 | | ¹H NMR δ (CDCl₃): 3.05 (s, 3H), 7.2 (q, 1H), 7.7-7.8 (m, 2H) |

The 1-(3,4-difluorobenzoyl)azetidine used in the preparation of **Example 20c** was prepared as described below.

### 1-(3,4-Difluorobenzoyl)azetidine

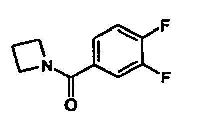

Oxalyl chloride (1.05 mL, 12.0 mmol) was added to a solution of 3,4-difluorobenzoic acid (1.58 g, 10 mmol) in DCM (50 mL) containing DMF (1 drop). The reaction was stirred at ambient temperature for 16 h then evapourated to dryness. The residue was redissolved in DCM (25 mL) and azetidine hydrochloride (1.12 g, 12.0 mmol) added followed by triethylamine (4.18 mL, 30.0 mmol). The mixture was stirred at ambient temperature for 2 h then concentrated *in vacuo.* The residue was partitioned between ethyl acetate and 1N hydrochloric acid, the organic phase washed with a saturated aqueous solution of sodium bicarbonate followed by brine, dried (MgSO₄), and concentrated *in vacuo.* The title compound was crystallized from an ethyl acetate / hexane mixture to give a white crystalline solid (1.0 g, 51%).
¹H NMR δ (CDCl₃): 2.4 (m, 2H), 4.3 (m, 4H), 7.2 (m, 1H), 7.4 (m, 1H), 7.5 (t, 1H).

### Example 21: 3-[4-(Azetidin-1-ylcarbonyl)-2-fluorophenoxyl-5-[(1S)-2-methoxy-1-methylethoxyl-N-1H-Dyrazol-3-ylbenzamide

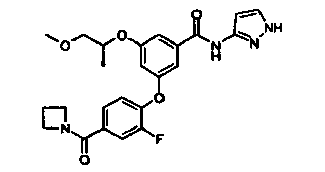

To a suspension of 3-[4-(azetidin-1-ylcarbonyl)-2-fluorophenoxy]-5-[(1*S*)-2-methoxy-1-methylethoxy]benzoic acid (300 mg, 0.75 mmol), HATU (356 mg, 0.938 mmol) and *tert-*butyl 3-amino-1*H*-pyrazole-1-carboxylate (172 mg, 0.938 mmol) in DMF (2 mL) was added DIPEA (0.326 mL, 1.88 mmol) and reaction mixture stirred for 16 hours at ambient temperature. Water was then added to reaction mixture and extracted into ethyl acetate (3x25 mL). Organic layer washed with saturated sodium hydrogen carbonate and saturated brine solution and dried (MgSO₄) Filtrate concentrated *in vacuo* to give an orange oil. This was dissolved in DCM (4 mL) and trifluoroacetic acid (0.445 mL, 8.0 equiv) was added. Reaction mixture stirred at ambient temperature for 8 hours. Saturated sodium carbonate was added to the reaction mixture and the phases separated. Organic phase was dried (MgSO₄) and concentrated *in vacuo* to give a white foam (26 mg, 7%).
¹H NMR δ (CDCl₃): 1.3 (d, 3H), 2.4 (m, 2H), 3.4 (s, 3H), 3.55 (m, 2H), 4.2 (m, 2H), 4.35 (m, 2H), 4.6 (m, 1H), 6.75 (s, 1H), 6.8 (app s, 1H), 7.05 (t, 1H), 7.1 (s, 1H), 7.3 (s, 1H), 7.4 (d, 1H), 7.5 (app d, 1H), 7.5 (app s, 1H), 9.6 (s, br, 1H). *m*/*z* 469 (M+H)⁺, 467 (M-H)⁻

### 3-[4-(Azetidin-1-ylcarbonyl)-2-fluorophenoxy]-5-[(1S)-2-methoxy-1-methylethoxy]benzoic acid

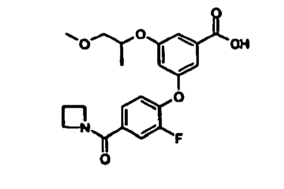

To a solution of methyl 3-[4-(azetidin-1-ylcarbonyl)-2-fluorophenoxy]-5-[(1*S*)-2-methoxy-1-methylethoxy]benzoate (400 mg, 1 mmol) in THF (6 mL) and water (1mL) was added 1M solution of sodium hydroxide (3 mL; 5.0equiv) and reaction mixture allowed to stir at room temperature for 3 hours. The THF was removed *in vacuo* and 1 M citric acid added until pH 3-4. Ethyl acetate was added and the phases separated. The organic phase was dried (MgSO₄) and concentrated *in vacuo* to give a clear oil (305 mg, 79%).
¹H NMR δ (CDCl₃): 1.3 (d, 3H), 2.4 (m, 2H), 3.4 (s, 3H), 3.5 (m, 2H), 4.2-4.4 (m, 4H), 4.6 (m, 1H), 6.8 (s, 1H), 7.05 (t, 1H), 7.25 (s, 1H), 7.4 (s, 1H), 7.45 (d, 1H), 7.5 (d, 1H); m/z 403 (M+H)⁺.

### Methyl 3-[4-(azetidin-1-ylcarbonyl)-2-fluorophenoxy]-5-[(1S)-2-methoxy-1-methylethoxy]benzoate

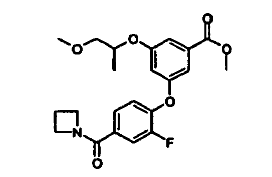

To a solution of methyl 3-hydroxy-5-[(1*S*)-2-methoxy-(1-methylethyl)oxy]benzoate (480 mg, 2 mmol) in DMA (2 mL) were added potassium carbonate (552 mg, 4 mmol), and a solution of 1-(3,4-difluorobenzoyl)azetidine (394 mg, 2 mmol) in DMA (2 mL). Reaction mixture was heated to 110°C and left to stir for 16 hours. Reaction mixture filtered and water (20 mL) added to reaction mixture. Extracted into ethyl acetate, washed with saturated sodium hydrogen carbonate solution and brine. Solution dried (MgSO₄) and concentrated *in vacuo* to an oil (400 mg, 48%). Residue used without further purification or characterisation.

The preparation of methyl 3-hydroxy-5-[(1*S*)-2-methoxy-(1-methylethyl)oxy]benzoate was described in **Example 11**. The preparation of 1-(3,4-difluorobenzoyl)azetidine was described in **Example 20c.**

### Example 22: 3-[4-(Azetidin-1-ylcarbonyl)phenoxyl-5-[(1S)-2-methoxy-1-methylethoxy]-N-(5-methyl-1H-pyrazol-3-yl)benzamide

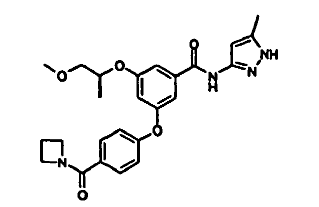

A solution of 3-[4-(azetidin-1-ylcarbonyl)-2-chlorophenoxy]-5-[(1*S*)-2-methoxy-1-methylethoxy]-*N*-(5-methyl-1*H*-pyrazol-3-yl)benzamide (100 mg, 0.2 mmol) and triethylamine (0.139 mL, 1 mmol) in THF (2.5 mL) and ethanol (2.5 mL) was evacuated and purged with nitrogen (x3). 10% w/w Palladium on carbon (10 mg) was added, the reaction mixture evacuated and purged with hydrogen gas. The reaction mixture was left to stir at ambient temperature under hydrogen for 48 hours. The solid residues were removed by filtration through diatomaceous earth and the mixture partitioned between ethyl acetate and 1M hydrochloric acid solution. The organic phase was dried (MgSO₄) and the filtrate concentrated *in vacuo.* The residue was chromatographed on silica, eluting with 0-70% methanol in ethyl acetate, to give the product (14 mg).
¹H NMR δ (d₆-DMSO): 1.2 (d, 3H), 2.2 (s, 3H), 2.25 (m, 2H), 3.25 (s, 3H obscured by water peak), 3.5 (m, 2H), 4.00 (m, 2H), 4.3 (m, 2H), 4.75 (m, 1H), 6.35 (s, 1H), 6.8 (s, 1 H), 7.05 (d, 2H), 7.2 (s, 1H), 7.4 (s, 1H), 7.65 (d, 2H), 10.7 (s, 1H), 12.1 (s br, 1H). *mlz* 465 (M+H)⁺, 463 (M-H)⁺

The preparation of 3-[4-(azetidin-1-ylcarbonyl)-2-chlorophenoxy]-5-[(1*S*)-2-methoxy-1-methylethoxy]-*N*-(5-methyl-1*H*-pyrazol-3-yl)benzamide was described in **Example 19d**.

### Example 23: 3-[(1S)-2-Methoxy-1-methylethoxy]-N-(1-methyl-1H-pyrazol-3-yl)-5-[4-(1,2,4-oxadiazol-3-yl)phenoxy]benzamide

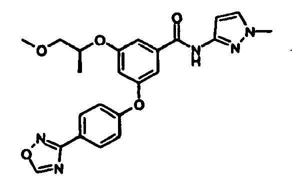

3-{4-[(Hydroxyamino)(imino)methyl]phenoxy}-5-[(1*S*)-2-methoxy-1-methylethoxy]-*N*-(1-methyl-1*H*-pyrazol-3-yl)benzamide was taken up in trimethyl orthoformate (3 mL) and 2 drops of BF₃.etherate added. The resulting solution was heated to 55°C in a CEM explorer microwave for 80 mins. The volatiles were removed under reduced pressure and the resulting oil chromatographed on silica, eluting with 0-100% ethyl acetate in iso-hexane, to give the desired compound as a white foam (295 mg)
¹H NMR δ(d₆-DMSO) δ 1.23 (d, 3H), 3.40 - 3.58 (m, 2H), 3.75 (s, 3H), 4.71 m, 1H), 6.54 (s, 1H), 6.86 (s, 1H), 7.18 - 7.28 (m, 3H), 7.44 (s, 1H), 7.57 (s, 1 H), 8.06 (d, 2H), 9.65 (s, 1H), 10.82 (s, 1H); m/z 450 (M+H)⁺.

### 3-{4-[(Hydroxyamino)(imino)methyl]phenoxy}-5-[(1S)-2-methoxy-1-methylethoxyl-N-(1-methyl-1H-pyrazol-3-yl)benzamide

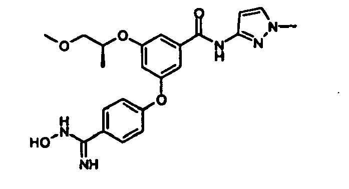

Hydroxylamine (50% w/w solution, 1 mL) was added to a solution of 3-(4-cyanophenoxy)-5-[(1*S*)-2-methoxy-(1-methylethyl)oxy]-*N-*(1-methyl-1*H*-pyrazol-3-yl)benzamide (300 mg, 0.74 mmol) in ethanol (3 mL) and the reaction mixture allowed to stir at room temperature for 18 hours. The volatiles were removed *in vacuo* to give the desired compound as a colourless foam (325 mg).
*m*/*z* = 440 (M+H)⁺

The preparation of 3-(4-cyanophenoxy)-5-[(1*S*)-2-methoxy-(1-methylethyl)oxy]-*N*-(1-methyl-1*H*-pyrazol-3-yl)benzamide was described in **Example 6**.

### BIOLOGICAL

### Tests:

The biological effects of the compounds of formula (I) may be tested in the following way:

### (1) Enzymatic activity

Enzymatic activity of recombinant human pancreatic GLK may be measured by incubating GLK, ATP and glucose. The rate of product (ie G-6-P) formation may be determined by coupling the assay to a G-6-P dehydrogenase, NADP/NADPH system and measuring the linear increase with time of optical density at 340nm (Matschinsky et al 1993). Activation of GLK by compounds can be assessed using this assay in the presence or absence of GLKRP as described in Brocklehurst et al (Diabetes 2004, 53, 535-541).

### Production of recombinant GLK and GLKRP

Human GLK and GLKRP cDNA was obtained by PCR from human pancreatic and hepatic mRNA respectively, using established techniques described in Sambrook J, Fritsch EF & Maniatis T, 1989. PCR primers were designed according to the GLK and GLKRP cDNA sequences shown in Tanizawa et al 1991 and Bonthron, D.T. *et al* 1994 (later corrected in Warner, J.P. 1995).

### Cloning in Bluescript II vectors

GLK and GLKRP cDNA was cloned in E. coli using pBluescript II, (Short et al 1998) a recombinant cloning vector system similar to that employed by Yanisch-Perron C *et al* (1985), comprising a colEI-based replicon bearing a polylinker DNA fragment containing multiple unique restriction sites, flanked by bacteriophage T3 and T7 promoter sequences; a filamentous phage origin of replication and an ampicillin drug resistance marker gene.

### Transformations

E. Coli transformations were generally carried out by electroporation. 400 mL cultures of strains DH5a or BL21(DE3) were grown in L-broth to an OD 600 of 0.5 and harvested by centrifugation at 2,000g. The cells were washed twice in ice-cold deionised water, resuspended in 1 mL 10% glycerol and stored in aliquots at -70°C. Ligation mixes were desalted using Millipore V series^{™} membranes (0.0025mm) pore size). 40mL of cells were incubated with 1mL of ligation mix or plasmid DNA on ice for 10 minutes in 0.2cm electroporation cuvettes, and then pulsed using a Gene Pulser^{™} apparatus (BioRad) at 0.5kVcm⁻¹, 250mF. Transformants were selected on L-agar supplemented with tetracyline at 10mg/mL or ampicillin at 100mg/mL.

### Expression

GLK was expressed from the vector pTB375NBSE in E.coli BL21 cells, producing a recombinant protein containing a 6-His tag immediately adjacent to the N-terminal methionine. Alternatively, another suitable vector is pET21 (+)DNA, Novagen, Cat number 697703. The 6-His tag was used to allow purification of the recombinant protein on a column packed with nickel-nitrilotriacetic acid agarose purchased from Qiagen (cat no 30250).

GLKRP was expressed from the vector pFLAG CTC (IBI Kodak) in E.coli BL21 cells, producing a recombinant protein containing a C-terminal FLAG tag. The protein was purified initially by DEAE Sepharose ion exchange followed by utilisation of the FLAG tag for final purification on an M2 anti-FLAG immunoaffinity column purchased from Sigma-Aldrich (cat no. A 1205).

### (2) Oral Glucose Tolerance Test (OGTT)

Oral glucose tolerance tests were done on conscious Zucker obese fa/fa rats (age 12-13 weeks or older) fed a high fat diet (45 % kcal fat) for at least two weeks prior to experimentation. The animals were fasted for 2 hours before use for experiments. A test compound or a vehicle was given orally 120 minutes before oral administration of a glucose solution at a dose of 2 g/kg body weight. Blood glucose levels were measured using a Accucheck glucometer from tail bled samples taken at different time points before and after administration of glucose (time course of 60 minutes). A time curve of the blood glucose levels was generated and the area-under-the-curve (AUC) for 120 minutes was calculated (the time of glucose administration being time zero). Percent inhibition was determined using the AUC in the vehicle-control group as zero percent inhibition.

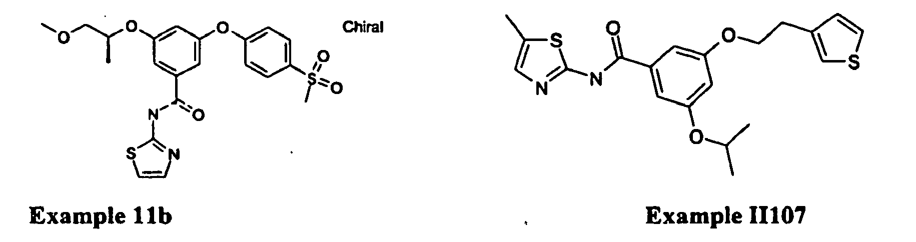

Compounds of the invention generally have an activating activity for glucokinase with an EC₅₀ of less than about 500nM. For example, Example 11b has an EC₅₀ of 30nM.

Example 11b and Example II107 in WO 03/015774 have broadly similar EC₅₀ values. However Example 11b has superior oral exposure and exhibits 29% OGTT activity at 10 mg/kg but Example II107 in WO 03/015774 is not active at 10 mg/kg.

### REFERENCES

1 Printz, R. L., Magnuson, M. A. and Granner, D. K. (1993) Annual Review of Nutrition 13, 463-96
2 DeFronzo, R. A. (1988) Diabetes 37, 667-87
3 Froguel, P., Zouali, H., Vionnet, N., Velho, G., Vaxillaire, M., Sun, F., Lesage, S., Stoffel, M., Takeda, J. and Passa, P. (1993) New England Journal of Medicine 328, 697-702
4 Bell, G. I., Pilkis, S. J., Weber, I. T. and Polonsky, K. S. (1996) Annual Review of Physiology 58, 171-86
5 Velho, G., Petersen, K. F., Perseghin, G., Hwang, J. H., Rothman, D. L., Pueyo, M. E., Cline, G. W., Froguel, P. and Shulman, G. I. (1996) Journal of Clinical Investigation 98, 1755-61
6 Christesen, H. B., Jacobsen, B. B., Odili, S., Buettger, C., Cuesta-Munoz, A., Hansen, T., Brusgaard, K., Massa, O., Magnuson, M. A., Shiota, C., Matschinsky, F. M. and Barbetti, F. (2002) Diabetes 51, 1240-6
6a Gloyn, A.L., Noordam, K., Willemsen, M.A.A.P., Ellard, S., Lam, W.W.K., Campbell, I. W., Midgley, P., Shiota, C., Buettger, C., Magnuson, M.A., Matschinsky, F.M., and Hattersley, A.T.; Diabetes 52: 2433-2440
7 Glaser, B., Kesavan, P., Heyman, M., Davis, E., Cuesta, A., Buchs, A., Stanley, C. A., Thornton, P. S., Permutt, M. A., Matschinsky, F. M. and Herold, K. C. (1998) New England Journal of Medicine 338, 226-30
8 Caro, J. F., Triester, S., Patel, V. K., Tapscott, E. B., Frazier, N. L. and Dohm, G. L. (1995) Hormone & Metabolic Research 27, 19-22
9 Desai, U. J., Slosberg, E. D., Boettcher, B. R., Caplan, S. L., Fanelli, B., Stephan, Z., Gunther, V. J., Kaleko, M. and Connelly, S. (2001) Diabetes 50, 2287-95
10 Shiota, M., Postic, C., Fujimoto, Y., Jetton, T. L., Dixon, K., Pan, D., Grimsby, J., Grippo, J. F., Magnuson, M. A. and Cherrington, A. D. (2001) Diabetes 50, 622-9
11 Ferre, T., Pujol, A., Riu, E., Bosch, F. and Valera, A. (1996) Proceedings of the National Academy of Sciences of the United States of America 93, 7225-30
12 Seoane, J., Barbera, A., Telemaque-Potts, S., Newgard, C. B. and Guinovart, J. J. (1999) Journal of Biological Chemistry 274, 31833-8
13 Moore, M. C., Davis, S. N., Mann, S. L. and Cherrington, A. D. (2001) Diabetes Care 24, 1882-7
14 Alvarez, E., Roncero, I., Chowen, J. A., Vazquez, P. and Blazquez, E. (2002) Journal of Neurochemistry 80, 45-53
15 Lynch, R. M., Tompkins, L. S., Brooks, H. L., Dunn-Meynell, A. A. and Levin, B. E. (2000) Diabetes 49, 693-700
16 Roncero, I., Alvarez, E., Vazquez, P. and Blazquez, E. (2000) Journal of Neurochemistry 74, 1848-57
17 Yang, X. J., Kow, L. M., Funabashi, T. and Mobbs, C. V. (1999) Diabetes 48, 1763-1772
18 Schuit, F. C., Huypens, P., Heimberg, H. and Pipeleers, D. G. (2001) Diabetes 50, 1-11
19 Levin, B. E. (2001) International Journal of Obesity 25, supp 5, S68-S72
20 Alvarez, E., Roncero, I., Chowen, J. A., Thorens, B. and Blazquez, E. (1996) Journal of Neurochemistry 66, 920-7
21 Mobbs, C. V., Kow, L. M. and Yang, X. J. (2001) American Journal of Physiology - Endocrinology & Metabolism 281, E649-54
22 Levin, B. E., Dunn-Meynell, A. A. and Routh, V. H. (1999) American Journal of Physiology 276, R1223-31
23 Spanswick, D., Smith, M. A., Groppi, V. E., Logan, S. D. and Ashford, M. L. (1997) Nature 390, 521-5
24 Spanswick, D., Smith, M. A., Mirshamsi, S., Routh, V. H. and Ashford, M. L. (2000) Nature Neuroscience 3, 757-8
25 Levin, B. E. and Dunn-Meynell, A. A. (1997) Brain Research 776, 146-53
26 Levin, B. E., Govek, E. K. and Dunn-Meynell, A. A. (1998) Brain Research 808, 317-9
27 Levin, B. E., Brown, K. L. and Dunn-Meynell, A. A. (1996) Brain Research 739, 293-300
28 Rowe, I. C., Boden, P. R. and Ashford, M. L. (1996) Journal of Physiology 497, 365-77
29 Fujimoto, K., Sakata, T., Arase, K., Kurata, K., Okabe, Y. and Shiraishi, T. (1985) Life Sciences 37, 2475-82
30 Kurata, K., Fujimoto, K. and Sakata, T. (1989) Metabolism: Clinical & Experimental 38,46-51
31 Kurata, K., Fujimoto, K., Sakata, T., Etou, H. and Fukagawa, K. (1986) Physiology & Behavior 37, 615-20
32 Jetton T.L., Liang Y., Pettepher C.C., Zimmerman E.C., Cox F.G., Horvath K., Matschinsky F.M., and Magnuson M.A., J. Biol. Chem., Feb 1994; 269: 3641 - 3654.
33 Reimann F. and Gribble F. M., Diabetes 2002 51: 2757-2763
34 Cheung A. T., Dayanandan B., Lewis J. T., Korbutt G. S., Rajotte R. V., Bryer-Ash M., Boylan M. O., Wolfe M. M., Kieffer T. J., Science, Vol 290, Issue 5498, 1959-1962 , 8 December 2000

## Claims

1. A compound of Formula (I), or a salt or solvate thereof: 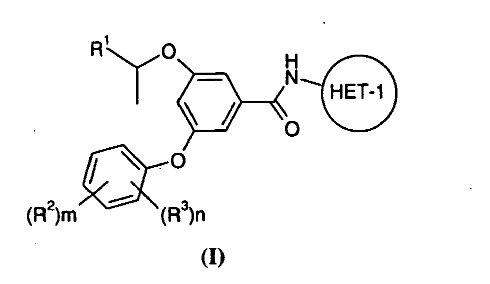 wherein:
R¹ is methoxymethyl;
R² is selected from -C(O)NR⁴R⁵, -SO₂NR⁴R⁵, -S(O)ₚR⁴ and HET-2;
HET-1 is a 5- or 6-membered, C-linked heteroaryl ring containing a nitrogen atom in the 2-position and optionally 1 or 2 further ring heteroatoms independently selected from O, N and
S; which ring is optionally substituted on an available carbon atom, or on a ring nitrogen atom provided it is not thereby quaternised, with 1 or 2 substituents independently selected from R⁶;
HET-2 is a 4-, 5- or 6-membered, C- or N-linked heterocyclyl ring containing 1, 2, 3 or 4 heteroatoms independently selected from O, N and S, wherein a -CH₂- group can optionally be replaced by a -C(O)- , and wherein a sulphur atom in the heterocyclic ring may optionally be oxidised to an S(O) or S(O)₂ group, which ring is optionally substituted on an available carbon or nitrogen atom by 1 or 2 substituents independently selected from R⁷;
R³ is selected from halo, fluoromethyl, difluoromethyl, trifluoromethyl, methyl, methoxy and cyano;
R⁴ is selected from hydrogen, (1-4C)alkyl [optionally substituted by 1 or 2 substituents independently selected from HET-2, -OR⁵, -SO₂R⁵, (3-6C)cycloalkyl (optionally substituted with 1 group selected from R⁷) and -C(O)NR⁵R⁵], (3-6C)cycloalkyl (optionally substituted with 1 group selected from R⁷) and HET-2;
R⁵ is hydrogen or (1-4C)alkyl;
or R⁴ and R⁵ together with the nitrogen atom to which they are attached may form a heterocyclyl ring system as defined by HET-3;
R⁶ is independently selected from (1-4C)alkyl, halo, hydroxy(1-4C)alkyl, (1-4C)alkoxy(1-4C)alkyl, (1-4C)alkylS(O)p(1-4C)alkyl, amino(1-4C)alkyl, (1-4C)alkylamino(1-4C)alkyl, di(1-4C)alkylamino(1-4C)alkyl and HET-4;
R⁷ is selected from -OR⁵,(1-4C)alkyl, -C(O)(1-4C)alkyl, -C(O)NR⁴R⁵, (1-4C)alkoxy(1-4C)alkyl, hydroxy(1-4C)alkyl and -S(O)pR⁵;
HET-3 is an N-linked, 4, 5 or 6- membered, saturated or partially unsaturated heterocyclyl ring, optionally containing 1 or 2 further heteroatoms (in addition to the linking N atom) independently selected from O, N and S, wherein a -CH₂- group can optionally be replaced by a -C(O)- and wherein a sulphur atom in the ring may optionally be oxidised to an S(O) or S(O)₂ group; which ring is optionally substituted on an available carbon or nitrogen atom by 1 or 2 substituents independently selected from R⁸; or
HET-3 is an N-linked, 7 membered, saturated or partially unsaturated heterocyclyl ring, optionally containing 1 further heteroatom (in addition to the linking N atom) independently selected from O, S and N, wherein a -CH₂- group can optionally be replaced by a -C(O)-group and wherein a sulphur atom in the ring may optionally be oxidised to an S(O) or S(O)₂ group; which ring is optionally substituted on an available carbon or nitrogen atom by 1 or 2 substituents independently selected from R⁸; or
HET-3 is an 6-10 membered bicyclic saturated or partially unsaturated heterocyclyl ring, optionally containing 1 further nitrogen atom (in addition to the linking N atom), wherein a -CH₂- group can optionally be replaced by a -C(O)-; which ring is optionally substituted on an available carbon or nitrogen atom by 1 substituent selected from hydroxy and R³;
R⁸ is selected from -OR⁵, (1-4C)alkyl, -C(O)(1-4C)alkyl, -C(O)NR⁴R⁵, (1-4C)alkylamino, di(1-4C)alkylamino, HET-3 (wherein said ring is unsubstituted), (1-4C)alkoxy(1-4C)alkyl, hydroxy(1-4C)alkyl and -S(O)pR ⁵;
HET-4 is a 5- or 6-membered, C-or N- linked unsubstituted heteroaryl ring containing 1, 2 or 3 ring heteroatoms independently selected from O, N and S;
p is (independently at each occurrence) 0, 1 or 2;
m is 0 or 1;
n is 0, 1 or 2;
provided that when m is 0, then n is 1 or 2;
and that the compound of Formula (I) is other than:
3-(4-methanesulfonylphenoxy)-5-(2-methoxy-1-methyl-ethoxy)-N-thiazol-2-yl-benzamide; N-(4-hydroxymethyl-thiazol-2-yl)-3-(4-methanesulfonylphenoxy)-5-(2-methoxy-1-methylethoxy)-benzamide;
N-[4-(1-hydroxy-ethyl)-thiazol-2-yl]-3-(4-methanesulfonylphenoxy)-5-(2-methoxy-1-methylethoxy)-benzamide;
N-[3-hydroxymethyl-1,2,4-thiadiazol-5-yl]-3-(4-methanesulfonylphenoxy)-5-2-methoxy-1-methyl-ethoxy)benzamide; or
3-(4-methanesulfonylphenoxy)-5-(2-methoxy-1-methyl-ethoxy)-(pyrazol-3-yl)benzamide.

2. A compound of the formula (I) as claimed in Claim 1 or a salt or solvate thereof wherein R¹ has the (S) configuration.

3. A compound of the formula (I) as claimed in Claim 1 or a salt or solvate thereof wherein m is 1, n is 0 and R² is in the para position relative to the ether linkage.

4. A compound of the formula (I) as claimed in Claim 1 or Claim 2 or Claim 3 or a salt or solvate thereof, wherein HET-1 is a 5-membered ring.

5. A compound of the formula (I) as claimed in any one of Claims 1 to 4 or a salt or solvate thereof, wherein R² is selected from -C(O)NR⁴R⁵ and -SO₂NR⁴R⁵ and R⁴ and R⁵ together with the nitrogen atom to which they are attached may form a heterocyclyl ring system as defined by HET-3.

6. A compound of the formula (I) as claimed in any one of Claims 1 to 5, or a salt or solvate thereof, wherein HET-3 is a 4- to 6-membered ring.

7. A compound of the formula (I) as claimed in Claim 1 or Claim 2 or Claim 3, or a salt or solvate thereof, wherein R² is selected from -C(O)NR⁴R⁵ and -SO₂NR⁴R⁵ and R⁴ is selected from (1-4C)alkyl [substituted by 1 or 2 substituents independently selected from HET-2, -OR⁵, -SO₂R⁵, (3-6C)cycloalkyl (optionally substituted with 1 group selected from R⁷) and -C(O)NR⁵R⁵], (3-6C)cycloalkyl (optionally substituted with 1 group selected from R⁷) and HET-2.

8. A compound of the formula (I) as claimed in Claim 1 or Claim 2 or Claim 3, or a salt or solvate thereof, wherein R² is -SO₂R⁴ and R⁴ is selected from (1-4C)alkyl [substituted by 1 or 2 substituents independently selected from HET-2, -OR⁵, -SO₂R⁵, (3-6C)cycloalkyl (optionally substituted with 1 group selected from R⁷) and -C(O)NR⁵R⁵], (3-6C)cycloalkyl (optionally substituted with 1 group selected from R⁷) and HET-2.

9. A compound of the formula (I) as claimed in Claim 1 or Claim 2 or Claim 3, or a salt or solvate thereof, wherein R² is HET-2.

10. A pharmaceutical composition comprising a compound according to any one of Claims 1 to 9, or a salt or solvate thereof, together with a pharmaceutically acceptable diluent or carrier.

11. A compound according to any one of Claims 1 to 9 or a pharmaceutically-acceptable salt or solvate thereof for use as a medicament.

12. A compound according to any one of Claims 1 to 9 for use in the preparation of a medicament for treatment of a disease mediated through GLK.

13. A compound according to any one of Claims 1 to 9 for use in the preparation of a medicament for treatment of type 2 diabetes.

14. Use of a compound of Formula (I) as claimed in any one of Claims 1 to 9, or salt or solvate thereof, for the manufacture of a medicament for treating a mammalian disease mediated by GLK.

15. A use according to Claim 14 wherein the GLK mediated disease is type 2 diabetes.

16. A process for the preparation of a compound of Formula (I) as claimed in any one of Claims 1 to 9, or a salt or solvate thereof which comprises (wherein variables are as defined in Claim 1 unless otherwise stated):
(a) reaction of an acid of Formula (III) or activated derivative thereof with a compound of Formula (IV), 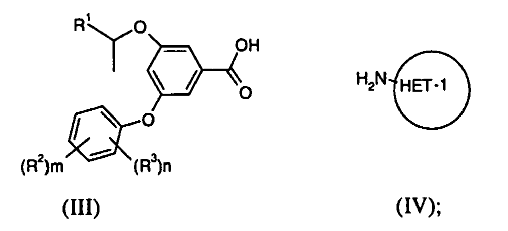 or
(b) reaction of a compound of Formula (V) with a compound of Formula (VI), 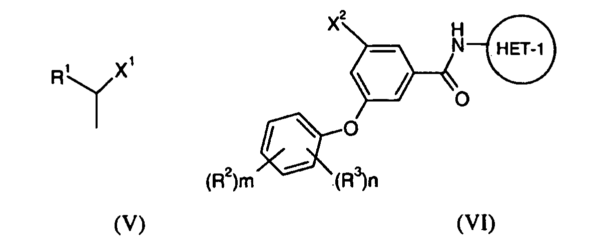 wherein X¹ is a leaving group and X² is a hydroxyl group or X¹ is a hydroxyl group and X² is a leaving group;
[or by reaction with the intermediate ester Formula (VII), wherein P¹ is a protecting group followed by ester hydrolysis and amide formation]; 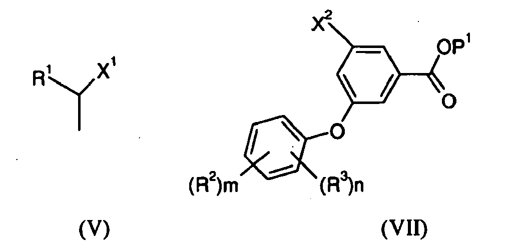 or
(c) reaction of a compound of Formula (VIII) with a compound of Formula (IX) 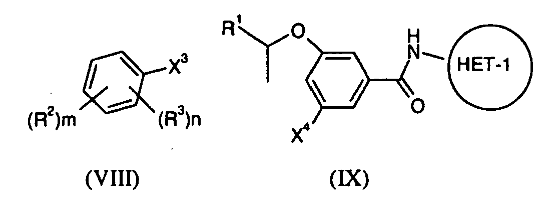 wherein X³ is a leaving, group or an organometallic reagent and X⁴ is a hydroxyl group or X³ is a hydroxyl group and X⁴ is a leaving group or an organometallic reagent;
[or by reaction or (VIII) with the intermediate ester Formula (X), followed by ester hydrolysis and amide formation]; 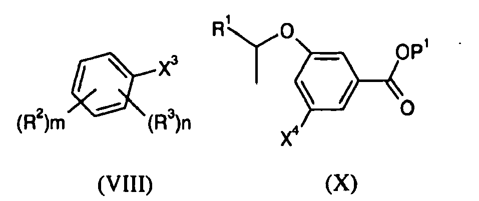 or
(d) reaction of a compound of Formula (XI) with a compound of Formula (XII), 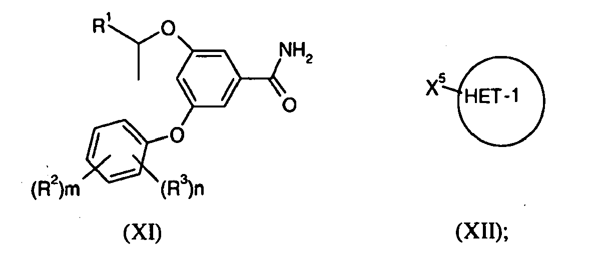
wherein X⁵ is a leaving group;
and thereafter, if necessary:
i) converting a compound of Formula (I) into another compound of Formula (I);
ii) removing any protecting groups; and/or
iii) forming a salt or solvate.

17. A compound of Formula (I), which is:
3-(4-{[(2-methoxyethyl)amino]carbonyl}phenoxy)-5-[(1S)-2-methoxy-(1-methylethyl)oxy]-N-1,3-thiazol-2-ylbenzamide;
3-(4-{[(1H-imidazol-2-ylmethyl)amino]carbonyl}phenoxy)-5-[(1S)-2-methoxy-(1-methylethyl)oxy]-N-1,3-thiazol-2-ylbenzamide;
3-[4-(azetidin-1-ylcarbonyl)phenoxy]-5-[(1S)-2-methoxy-(1-methylethyl)oxy]-N-1,3-thiazol-2-ylbenzamide;
3-[(1S)-2-methoxy-(1-methylethyl)oxy]-5-{4-[(4-methylpiperazin-1-yl)carbonyl]phenoxy}-N-1,3-thiazol-2-ylbenzamide;
3-(3-{[(2-methoxyethyl)amino]carbonyl}phenoxy)-5-[(1S)-2-methoxy-(1-methylethyl)oxy]-N-1,3-thiazol-2-ylbenzamide;
3-(3-{[(1H-imidazol-2-ylmethyl)amino]carbonyl}phenoxy)-5-[(1S)-2-methoxy-(1-methylethyl)oxy]-N-1,3-thiazol-2-ylbenzamide;
3-[(1S)-2-methoxy-(1-methylethyl)oxy]-5-{4-[(4-methylpiperazin-1-yl)carbonyl]phenoxy}-N-(1-methyl-1H-pyrazol-3-yl)benzamide;
3-[(1S)-2-methoxy-(1-methylethyl)oxy]-N-(1-methyl-1H-pyrazol-3-yl)-5-[4-(morpholin-4-ylcarbonyl)phenoxy]benzamide;
3-[4-(azetidin-1-ylcarbonyl)phenoxy]-5-[(1S)-2-methoxy-(1-methylethyl)oxy]-N-(1-methyl-1H-pyrazol-3-yl)benzamide;
3-[(1S)-2-methoxy-(1-methylethyl)oxy]-N-(1-methyl-1H-pyrazol-3-yl)-5-[4-(pyrrolidin-1-ylcarbonyl)phenoxy]benzamide;
3-[4-(7-azabicyclo[2.2.1]hept-7-ylcarbonyl)phenoxy]-5-[(1S)-2-methoxy-(1-methylethyl)oxy]-N-(1-methyl-1H-pyrazol-3-yl)benzamide;
3-{2-chloro-4-[(dimethylamino)sulfonyl]phenoxy}-5-[(1S)-2-methoxy-(1-methylethyl)oxy]-N-(1-methyl-1H-pyrazol-3-yl)benzamide;
3-[(2-chloro-4-{[(1-methylethyl)amino]sulfonyl}phenyl)oxy]-5-[(1S)-2-methoxy-(1-methylethyl)oxy]-N-(1-methyl-1H-pyrazol-3-yl)benzamide;
3-{[2-chloro-4-({[2-(methyloxy)ethyl]amino}sulfonyl)phenyl]oxy}-5-[(1S)-2-methoxy-(1-methylethyl)oxy]-N-(1-methyl-1H-pyrazol-3-yl)benzamide;
3-({2-chloro-4-[(4-methylpiperazin-1-yl)sulfonyl]phenyl}oxy)-5-[(1S)-2-methoxy-(1-methylethyl)oxy]-N-(1-methyl-1H-pyrazol-3-yl)benzamide;
3-{4-[(dimethylamino)sulfonyl]phenoxy}-5-[(1S)-2-methoxy-(1-methylethyl)oxy]-N-(1-methyl-1H-pyrazol-3-yl)benzamide;
3-[(1S)-2-methoxy-(1-methylethyl)oxy]-5-({4-[(4-methylpiperazin-1-yl)sulfonyl]phenyl}oxy)-N-(1-methyl-1H-pyrazol-3-yl)benzamide;
3-{4-[((1-methylethyl)amino)sulfonyl]phenoxy}-5-[(1S)-2-methoxy-(1-methylethyl)oxy]-N-(1-methyl-1H-pyrazol-3-yl)benzamide;
3-(4-{([(2-methoxyethyl)amino]sulfonyl}phenoxy)-5-[(1S)-2-methoxy-(1-methylethyl)oxy]-N-(1-methyl-1H-pyrazol-3-yl)benzamide;
3-(4-cyanophenoxy)-5-[(1S)-2-methoxy-(1-methylethyl)oxy]-N-(1-methyl-1H-pyrazol-3-yl)benzamide;
3-{[4-(aminocarbonyl)phenyl]oxy}-5-[(1S)-2-methoxy-(1-methylethyl)oxy]-N-(1-methyl-1H-pyrazol-3-yl)benzamide;
3-[4-(ethylsulfonyl)phenoxy]-5-[(1S)-2-methoxy-(1-methylethyl)oxy]-N-1,3-thiazol-2-ylbenzamide;
3-[(1S)-2-methoxy-(1-methylethyl)oxy]-N-(1-methyl-1H-pyrazol-3-yl)-5-{[3-(methylthio)phenyl]oxy benzamide;
3-({4-[(1-methylethyl)thio]phenyl}oxy)-5-[(1S)-2-methoxy-(1-methylethyl)oxy]-N-(1-methyl-1H-pyrazol-3-yl)benzamide;
3-[(1S)-2-methoxy-(1-methylethyl)oxy]-N-(1-methyl-1H-pyrazol-3-yl)-5-[3-(methylsulfonyl)phenoxy]benzamide;
3-[(1S)-2-methoxy-(1-methylethyl)oxy]-N-(1-methyl-1H-pyrazol-3-yl)-5-[3-(methylsulfinyl)phenoxy]benzamide;
3-({4-[(1-methylethyl)sulfonyl]phenyl}oxy)-5-[(1S)-2-methoxy-(1-methylethyl)oxy]-N-(1-methyl-1H-pyrazol-3-yl)benzamide;
3-[(1S)-2-methoxy-(1-methylethyl)oxy]-N-(1-methyl-1H-pyrazol-3-yl)-5-[4-(methylsulfonyl)phenoxy]benzamide;
3-[(1S)-2-methoxy-(1-methylethyl)oxy]-5-[4-(methylsulfonyl)phenoxy]-N-(4-methyl-1,3-thiazol-2-yl)benzamide;
3-[(1S)-2-methoxy-(1-methylethyl)oxy]-5-[4-(methylsulfonyl)phenoxy]-N-(5-methyl-1,3-thiazol-2-yl)benzamide;
3-[(1S)-2-methoxy-(1-methylethyl)oxy]-5-[4-(methylsulfonyl)phenoxy]-N-(5-methyl-1,3,4-thiadiazol-2-yl)benzamide;
3-[(1S)-2-methoxy-(1-methylethyl)oxy]-5-[4-(methylsulfonyl)phenoxy]-N-(3-methyl-1,2,4-thiadiazol-5-yl)benzamide;
N-(1-ethyl-1H-pyrazol-3-yl)-3-[(1S)-2-methoxy-(1-methylethyl)oxy]-5-[4-(methylsulfonyl)phenoxy]benzamide;
3-(3,5-difluorophenoxy)-5-[(1S)-2-methoxy-(1-methylethyl)oxy]-N-(1-methyl-1H-pyrazol-3-yl)benzamide;
N-(5-bromopyridin-2-yl)-3-(3,5-difluorophenoxy)-5-[(1S)-2-methoxy-(1-methylethyl)oxy]benzamide;
3-(3,5-difluorophenoxy)-N-[4-(hydroxymethyl)-1,3-thiazol-2-yl]-5-[(1S)-2-methoxy-(1-methylethyl)oxy]benzamide;
3-[(1S)-2-methoxy-(1-methylethyl)oxy]-5-[4-(methylsulfonyl)phenoxy]-N-1H-pyrazol-3-ylbenzamide;
3-[(1S)-2-methoxy-(1-methylethyl)oxy]-N-(5-methyl-1H-pyrazol-3-yl)-5-[4-(methylsulfonyl)phenoxy]benzamide;
3-[(1S)-2-methoxy-(1-methylethyl)oxy]-N-[4-(methoxymethyl)-1,3-thiazol-2-yl]-5-[4-(methylsulfonyl)phenoxy]benzamide;
3-[4-(azetidin-1-ylcarbonyl)phenoxy]-5-[(1S)-2-methoxy-(1-methylethyl)oxy]-N-(3-methyl-1,2,4-thiadiazol-5-yl)benzamide;
3-[(1S)-2-methoxy-(1-methylethyl)oxy]-5-(4-{[(1-methylpiperidin-4-yl)amino]carbonyl}phenoxy)-N-(3-methyl-1,2,4-thiadiazol-5-yl)benzamide;
3-[4-(azetidin-1-ylcarbonyl)-2-chlorophenoxy]-5-[(1S)-2-methoxy-(1-methylethyl)oxy]-N-(1-methyl-1H-pyrazol-3-yl)benzamide;
3-[4-(azetidin-1-ylcarbonyl)-2-fluorophenoxy]-5-[(1S)-2-methoxy-(1-methylethyl)oxy]-N-(1-methyl-1H-pyrazol-3-yl)benzamide;
3-[4-(azetidin-1-ylcarbonyl)-2-(trifluoromethyl)phenoxy]-5-[(1S)-2-methoxy-(1-methylethyl)oxy]-N-(1-methyl-1H-pyrazol-3-yl)benzamide;
3-{4-[(dimethylamino)carbonyl]phenoxy}-5-[(1S)-2-methoxy-(1-methylethyl)oxy]-N-(1-methyl-1H-pyrazol-3-yl)benzamide;
or a salt or solvate thereof.

18. A compound of Formula (I), which is:
3-[4-(azetidin-1-ylcarbonyl)phenoxy]-5-[(1S)-2-methoxy-1-methylethoxy]-N-1H-pyrazol-3-ylbenzamide;
3-[4-(azetidin-1-ylcarbonyl)-2-chlorophenoxy]-5-[(1S)-2-methoxy-1-methylethoxy]-N-(5-methyl-1H-pyrazol-3-yl)benzamide;
3-[4-(azetidin-1-ylcarbonyl)-2-fluorophenoxy]-5-[(1S)-2-methoxy-1-methylethoxy]-N-(5-methyl-1H-pyrazol-3-yl)benzamide;
3-[4-(azetidin-1-ylcarbonyl)-2-fluorophenoxy]-5-[(1S)-2-methoxy-1-methylethoxy]-N-1H-pyrazol-3-ylbenzamide;
3-[4-(azetidin-1-ylcarbonyl)phenoxy]-5-[(1S)-2-methoxy-1-methylethoxy]-N-(5-methyl-1H-pyrazol-3-yl)benzamide; or
3-[(1S)-2-methoxy-1-methylethoxy]-N-(1-methyl-1H-pyrazol-3-yl)-5-[4-(1,2,4-oxadiazol-3-yl)phenoxy]benzamide;
or a salt or solvate thereof.

19. A compound of formula (I) as claimed in Claim 1 which is 3-[4-(azetidin-1-ylcarbonyl)phenoxy]-5-[(1S)-2-methoxy-(1-methylethyl)oxy]-N-(1-methyl-1H-pyrazol-3-yl)benzamide; or a salt or solvate thereof.

20. A compound of formula (I) as claimed in Claim 1 which is 3-[(1S)-2-methoxy-(1-methylethyl)oxy]-N-(1-methyl-1H-pyrazol-3-yl)-5-[4-(methylsulfonyl)phenoxy]benzamide; or a salt or solvate thereof.

21. A compound of formula (1) as claimed in Claim 1 which is 3-[(1S)-2-methoxy-(1-methylethyl)oxy]-5-[4-(methylsulfonyl)phenoxy]-N-(3-methyl-1,2,4-thiadiazol-5-yl)benzamide; or a salt or solvate thereof.

22. A combination of a compound according to any one of Claims 1 to 9 or Claims 17 to 21 with one or more other substances and/or treatments.

## Patentansprüche

1. Verbindungen der Formel (I) und deren Salze und Solvate: 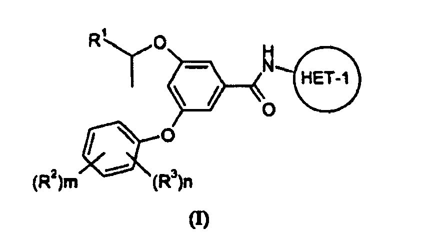 wobei:
R¹ für Methoxymethyl steht;
R² ausgewählt ist aus -C(O) NR⁴R⁵, -SO₂NR⁴R⁵, -S(O)ₚR⁴ und HET-2;
HET-1 für einen 5- oder 6gliedrigen, über Kohlenstoff gebundenen Heteroarylring mit einem Stickstoffatom in der 2-Stellung und gegebenenfalls 1 oder 2 weiteren Ringheteroatomen unabhängig voneinander ausgewählt aus O, N und S steht; wobei dieser Ring gegebenenfalls an einem verfügbaren Kohlenstoffatom oder an einem Ringstickstoffatom, vorausgesetzt, daß es **dadurch** nicht quaternisiert wird, durch 1 oder 2 Substituenten unabhängig voneinander ausgewählt aus R⁶ substituiert ist;
HET-2 für einen 4-, 5- oder 6gliedrigen, über Kohlenstoff oder Stickstoff gebundenen Heteroarylring mit 1, 2, 3 oder 4 Heteroatomen unabhängig voneinander ausgewählt aus O, N und S steht, wobei eine -CH₂-Gruppe gegebenenfalls durch -C(O)- ersetzt sein kann, und wobei ein Schwefelatom in dem heterocyclischen Ring gegebenenfalls zu einer S(O)- oder S(O)₂-Gruppe oxidiert sein kann, wobei dieser Ring gegebenenfalls an einem verfügbaren Kohlenstoff- oder Stickstoffatom durch 1 oder 2 Substituenten unabhängig voneinander ausgewählt aus R⁷ substituiert ist;
R³ ausgewählt ist aus Halogen, Fluormethyl, Difluormethyl, Trifluormethyl, Methyl, Methoxy und Cyano;
R³ ausgewählt ist aus Wasserstoff, (1-4C)-Alkyl [gegebenenfalls substituiert durch 1 oder 2 Substituenten unabhängig voneinander ausgewählt aus HET-2 , -OR⁵, -SO₂R⁵, (3-6C)-Cycloalkyl (gegebenenfalls substituiert durch 1 Gruppe ausgewählt aus R⁷) und -C(O)NR⁵R⁵] , (3-6C)-Cycloalkyl (gegebenenfalls substituiert durch 1 Gruppe ausgewählt aus R⁷) und HET-2;
R⁵ für Wasserstoff oder (1-4C)-Alkyl steht;
oder
R⁴ und R⁵ zusammen mit dem Stickstoffatom, an das sie gebunden sind, ein durch HET-3 definiertes Heterocyclylringsystem bilden können;
R⁶ unabhängig ausgewählt ist aus (1-4C)-Alkyl, Halogen, Hydroxy-(1-4C)-alkyl, (1-4C)-Alkoxy-(1-4C)-alkyl, (1-4C)-Alkyl-S(O)p-(1-4C)-alkyl, Amino-(1-4C)-alkyl, (1-4C)-Alkylamino-(1-4C)-alkyl, Di-(1-4C)-alkylamino-(1-4C)-alkyl und HET-4;
R⁷ ausgewählt ist aus -OR⁵, (1-4C)-Alkyl, -C(O)-(1-4C)-Alkyl, -C(O)NR⁴R⁵, (1-4C)-Alkoxy-(1-4C)-alkyl, Hydroxy-(1-4C)-alkyl und -S(O)pR⁵;
HET-3 für einen über Stickstoff gebundenen, 4-, 5- oder 6gliedrigen gesättigten oder teilweise ungesättigten Heterocyclylring steht, der gegebenenfalls 1 oder 2 weitere Heteroatome (zusätzlich zu dem verbindenden Stickstoffatom) unabhängig voneinander ausgewählt aus O, N und S enthält, wobei eine -CH₂-Gruppe gegebenenfalls durch -C(O)- ersetzt sein kann und wobei ein Schwefelatom in dem Ring gegebenenfalls zu einer S(O)- oder S(O)₂-Gruppe oxidiert sein kann; wobei dieser Ring gegebenenfalls an einem verfügbaren Kohlenstoff- oder Stickstoffatom durch 1 oder 2 Substituenten unabhängig voneinander ausgewählt aus R⁸ substituiert ist; oder
HET-3 für einen über Stickstoff gebundenen, 7gliedrigen gesättigten oder teilweise ungesättigten Heterocyclylring steht, der gegebenenfalls ein weiteres Heteroatom (zusätzlich zu dem verbindenden Stickstoffatom) unabhängig ausgewählt aus O, S und N enthält, wobei eine -CH₂-Gruppe gegebenenfalls durch eine -C(O)-Gruppe ersetzt sein kann und wobei ein Schwefelatom in dem Ring gegebenenfalls zu einer S(O)- oder S(O)₂-Gruppe oxidiert sein kann; wobei der Ring gegebenenfalls an einem verfügbaren Kohlenstoff- oder Stickstoffatom durch 1 oder 2 Substituenten unabhängig ausgewählt aus R⁸ substituiert ist; oder
HET-3 für einen 6-10gliedrigen bicyclischen gesättigten oder teilweise ungesättigten Heterocyclylring steht, der gegebenenfalls 1 weiteres Stickstoffatom (zusätzlich zu dem verbindenden Stickstoffatom) enthält, wobei eine -CH₂-Gruppe gegebenenfalls durch -C(O)-ersetzt sein kann; wobei dieser Ring gegebenenfalls an einem verfügbaren Kohlenstoff- oder Stickstoffatom durch 1 Substituenten ausgewählt aus Hydroxy und R³ substituiert ist;
R⁸ ausgewählt ist aus -OR⁵, (1-4C)-Alkyl, -C(O)(1-4C)-Alkyl, -C(O)NR⁴R⁵, (1-4C)-Alkylamino, Di-(1-4C)-alkylamino, HET-3 (wobei dieser Ring unsubstituiert ist), (1-4C)-Alkoxy-(1-4C)-alkyl, Hydroxy-(1-4C)-alkyl und -S(O)pR⁵;
HET-4 für einen 5- oder 6gliedrigen, über Kohlenstoff oder Stickstoff gebundenen unsubstituierten Heteroarylring mit 1, 2 oder 3 Ringheteroatomen unabhängig voneinander ausgewählt aus O, N und S steht;
p (unabhängig bei jedem Vorkommen) für 0, 1 oder 2 steht;
m für 0 oder 1 steht;
n für 0, 1 oder 2 steht;
mit der Maßgabe, daß, wenn m für 0 steht, n für 1 oder 2 steht;
und daß es sich bei der Verbindung der Formel (I) nicht um:
3-(4-Methansulfonylphenoxy)-5-(2-methoxy-1-methylethoxy)-N-thiazol-2-ylbenzamid;
N-(4-Hydroxymethylthiazol-2-yl)-3-(4-methansulfonylphenoxy)-5-(2-methoxy-1-methylethoxy)benzamid;
N-[4-(1-Hydroxyethyl)thiazol-2-yl]-3-(4-methansulfonylphenoxy)-5-(2-methoxy-1-methylethoxy)benzamid;
N-[3-Hydroxymethyl)-1,2,4-thiadiazol-5-yl]-3-(4-methansulfonylphenoxy)-5-(2-methoxy-1-methylethoxy)benzamid; oder
3-(4-Methansulfonylphenoxy)-5-(2-methoxy-1-methylethoxy)(pyrazol-3-yl)benzamid
handelt.

2. Verbindungen der Formel (I) nach Anspruch 1 und deren Salze und Solvate, wobei R¹ (S)-Konfiguration aufweist.

3. Verbindungen der Formel (I) nach Anspruch 1 und deren Salze und Solvate, wobei m für 1 steht, n für 0 steht und R² sich in der Para-Stellung zur Etherbindung befindet.

4. Verbindungen der Formel (I) nach einem der Ansprüche 1 bis 3 und deren Salze und Solvate, wobei HET-1 für einen 5gliedrigen Ring steht.

5. Verbindungen der Formel (I) nach einem der Ansprüche 1 bis 4 und deren Salze und Solvate, wobei R² ausgewählt ist aus -C(O)NR⁴R⁵ und -SO₂NR⁴R⁵ und R⁴ und R⁵ zusammen mit dem Stickstoffatom, an das sie gebunden sind, ein durch HET-3 definiertes Heterocyclylringsystem bilden können.

6. Verbindungen der Formel (I) nach einem der Ansprüche 1 bis 5 und deren Salze und Solvate, wobei HET-3 für einen 4- bis 6gliedrigen Ring steht.

7. Verbindungen der Formel (I) nach einem der Ansprüche 1 bis 3 und deren Salze und Solvate, wobei R² ausgewählt ist aus -C(O)NR⁴R⁵ und -SO₂NR⁴R⁵ und R⁴ ausgewählt ist aus (1-4C)-Alkyl [substituiert durch 1 oder 2 Substituenten unabhängig voneinander ausgewählt aus HET-2, -OR⁵, -SO₂R⁵, (3-6C)-Cycloalkyl (gegebenenfalls substituiert durch 1 Gruppe ausgewählt aus R⁷) und -C(O)NR⁵R⁵], (3-6C) -Cycloalkyl (gegebenenfalls substituiert durch 1 Gruppe ausgewählt aus R⁷) und HET-2.

8. Verbindungen der Formel (I) nach einem der Ansprüche 1 bis 3 und deren Salze und Solvate, wobei R² für -SO₂R⁴ steht und R⁴ ausgewählt ist aus (1-4C)-Alkyl [substituiert durch 1 oder 2 Substituenten unabhängig voneinander ausgewählt aus HET-2, -OR⁵, -SO₂R⁵, (3-6C)-Cycloalkyl (gegebenenfalls substituiert durch 1 Gruppe ausgewählt aus R⁷) und -C(O)NR⁵R⁵], (3-6C)-Cycloalkyl (gegebenenfalls substituiert durch 1 Gruppe ausgewählt aus R⁷) und HET-2.

9. Verbindungen der Formel (I) nach einem der Ansprüche 1 bis 3 und deren Salze und Solvate, wobei R² für HET-2 steht.

10. Pharmazeutische Zusammensetzung, enthaltend eine Verbindung nach einem der Ansprüche 1 bis 9 oder ein Salz oder Solvat davon zusammen mit einem pharmazeutisch annehmbaren Verdünnungsmittel oder Träger.

11. Verbindungen nach einem der Ansprüche 1 bis 9 und deren pharmazeutisch annehmbare Salze und Solvate zur Verwendung als Medikament.

12. Verbindungen nach einem der Ansprüche 1 bis 9 zur Verwendung bei der Herstellung eines Medikaments zur Behandlung einer durch GLK vermittelten Krankheit.

13. Verbindungen nach einem der Ansprüche 1 bis 9 zur Verwendung bei der Herstellung eines Medikaments zur Behandlung von Typ-2-Diabetes.

14. Verwendung einer Verbindung der Formel (I) nach einem der Ansprüche 1 bis 9 oder eines Salzes oder Solvates davon bei der Herstellung eines Medikaments zur Behandlung einer durch GLK vermittelten Krankheit in einem Säugetier.

15. Verwendung nach Anspruch 14, wobei es sich bei der durch GLK vermittelten Krankheit um Typ-2-Diabetes handelt.

16. Verfahren zur Herstellung einer Verbindung der Formel (I) nach einem der Ansprüche 1 bis 9 oder einem Salz oder Solvat davon, bei dem man (wobei Variablen, wenn nicht anders angegeben, wie in Anspruch 1 definiert sind):
(a) eine Säure der Formel (III) oder ein aktiviertes Derivat davon mit einer Verbindung der Formel (IV) umsetzt 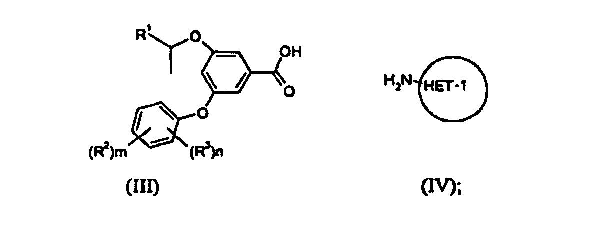 oder
(b) eine Verbindung der Formel (V) mit einer Verbindung der Formel (VI) umsetzt 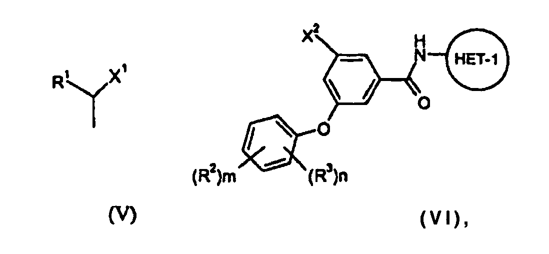 wobei X¹ für eine Abgangsgruppe steht und X² für eine Hydroxylgruppe steht oder X¹ für eine Hydroxylgruppe steht und X² für eine Abgangsgruppe steht;
[oder durch Umsetzung mit dem Ester-Zwischenprodukt der Formel (VII), wobei P¹ für eine Schutzgruppe steht, und anschließender Esterhydrolyse und Amidbildung]; 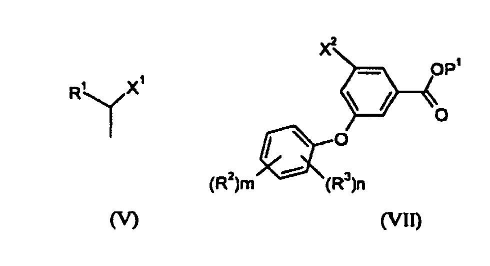 oder
(c) eine Verbindung der Formel (VIII) mit einer Verbindung der Formel (IX) umsetzt 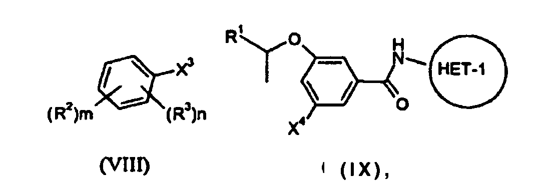 wobei X³ für eine Abgangsgruppe oder ein metallorganisches Reagens steht und X⁴ für eine Hydroxylgruppe steht oder X³ für eine Hydroxylgruppe steht und X⁴ für eine Abgangsgruppe oder ein metallorganisches Reagens steht; [oder durch Umsetzung von (VIII) mit dem Ester-Zwischenprodukt der Formel (X) und anschließender Esterhydrolyse und Amidbildung]; 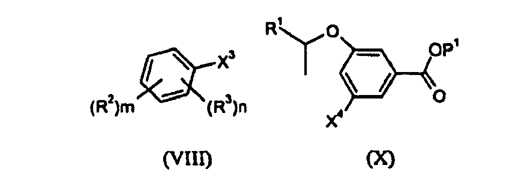 oder
(d) eine Verbindung der Formel (XI) mit einer Verbindung der Formel (XII) umsetzt 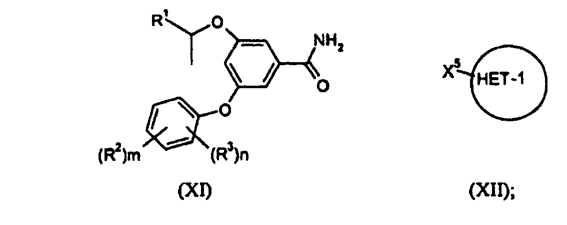
wobei X⁵ für eine Abgangsgruppe steht; und anschließend, falls erforderlich:
i) eine Verbindung der Formel (I) in eine andere Verbindung der Formel (I) umwandelt;
ii) gegebenenfalls vorhandene Schutzgruppen entfernt; und/oder
iii) ein Salz oder Solvat bildet.

17. Verbindungen der Formel (I), bei denen es sich um:
3-(4-{[(2-Methoxyethyl)amino]carbonyl}phenoxy)-5-[(1S)-2-methoxy-(1-methylethyl)oxy]-N-1,3-thiazol-2-ylbenzamid;
3-(4-{[(1H-Imidazol-2-ylmethyl)amino]carbonyl}phenoxy)-5-[(1S)-2-methoxy-(1-methylethyl)oxy]-N-1,3-thiazol-2-ylbenzamid;
3-[4-(Azetidin-1-ylcarbonyl)phenoxy]-5-[(1S)-2-methoxy-(1-methylethyl)oxy]-N-1,3-thiazol-2-ylbenzamid;
3-[(1S)-2-Methoxy-(1-methylethyl)oxy]-5-{4-[(4-methylpiperazin-1-yl)carbonyl]phenoxy}-N-1,3-thiazol-2-ylbenzamid;
3-(3-{[(2-Methoxyethyl)amino]carbonyl}phenoxy)-5-[(1S)-2-methoxy-(1-methylethyl)oxy]-N-1,3-thiazol-2-ylbenzamid;
3-(3-{[(1H-Imidazol-2-ylmethyl)amino]carbonyl}phenoxy)-5-[(1S)-2-methoxy-(1-methylethyl)oxy]-N-1,3-thiazol-2-ylbenzamid;
3-[(1S)-2-Methoxy-(1-methylethyl)oxy]-5-{4-[(4-methylpiperazin-1-yl)carbonyl]phenoxy}-N-(1-methyl-1H-pyrazol-3-yl)benzamid;
3-[(1S)-2-Methoxy-(1-methylethyl)oxy]-N-(1-methyl-1H-pyrazol-3-yl)-5-[4-(morpholin-4-ylcarbonyl)phenoxy]benzamid;
3-[4-(Azetidin-1-ylcarbonyl)phenoxy]-5-[(1S)-2-methoxy-(1-methylethyl)oxy]-N-(1-methyl-1H-pyrazol-3-yl)benzamid;
3-[(1S)-2-Methoxy-(1-methylethyl)oxy]-N-(1-methyl-1H-pyrazol-3-yl)-5-[4-pyrrolidin-1-ylcarbonyl)phenoxy]benzamid;
3-[4-(7-Azabicyclo[2.2.1]hept-7-ylcarbonyl)phenoxy]-5-[(1S)-2-methoxy-(1-methylethyl)oxy]-N-(1-methyl-1H-pyrazol-3-yl)benzamid;
3-{2-Chlor-4-[(dimethylamino)sulfonyl]phenoxy}-5-[(1S)-2-methoxy-(1-methylethyl)oxy]-N-(1-methyl-1H-pyrazol-3-yl)benzamid;
3-[(2-Chlor-4-{[(1-methylethyl)amino]sulfonyl}phenyl)oxy]-5-[(1S)-2-methoxy-(1-methylethyl)oxy]-N-(1-methyl-1H-pyrazol-3-yl)benzamid;
3-{[2-Chlor-4-({[2-(methyloxy)ethyl]amino}sulfonyl)phenyl]oxy}-5-[(1S)-2-methoxy-(1-methylethyl)oxy]-N-(1-methyl-1H-pyrazol-3-yl)benzamid;
3-({2-Chlor-4-[(4-methylpiperazin-1-yl)sulfonyl]phenyl}oxy)-5-[(1S)-2-methoxy-(1-methylethyl)oxy]-N-(1-methyl-1H-pyrazol-3-yl)benzamid;
3-{4-[(Dimethylamino)sulfonyl]phenoxy}-5-[(1S)-2-methoxy-(1-methylethyl)oxy]-N-(1-methyl-1H-pyrazol-3-yl)benzamid;
3-[(1S)-2-Methoxy-(1-methylethyl)oxy]-5-({4-[(4-methylpiperazin-1-yl)sulfonyl]phenyl}oxy)-N-(1-methyl-1H-pyrazol-3-yl)benzamid;
3-{4-[((1-Methylethyl)amino)sulfonyl]phenoxy}-5-[(1S)-2-methoxy-(1-methylethyl)oxy]-N-(1-methyl-1H-pyrazol-3-yl)benzamid;
3-(4-{[(2-Methoxyethyl)amino]sulfonyl}phenoxy)-5-[(1S)-2-methoxy-(1-methylethyl)oxy]-N-(1-methyl-1H-pyrazol-3-yl)benzamid;
3-(4-Cyanophenoxy)-5-[(1S)-2-methoxy-(1-methylethyl)oxy]-N-(1-methyl-1H-pyrazol-3-yl)benzamid;
3-{[4-(Aminocarbonyl)phenyl]oxy}-5-[(1S)-2-methoxy-(1-methylethyl)oxy]-N-(1-methyl-1H-pyrazol-3-yl)benzamid;
3-[4-(Ethylsulfonyl)phenoxy]-5-[(1S)-2-methoxy-(1-methylethyl)oxy]-N-1,3-thiazol-2-ylbenzamid;
3-[(1S)-2-Methoxy-(1-methylethyl)oxy]-N-(1-methyl-1H-pyrazol-3-yl)-5-{[3-(methylthio)phenyl]oxy}benzamid;
3-({4-[(1-Methylethyl)thio]phenyl}oxy)-5-[(1S)-2-methoxy-(1-methylethyl)oxy]-N-(1-methyl-1H-pyrazol-3-yl)benzamid;
3-[(1S)-2-Methoxy-(1-methylethyl)oxy]-N-(1-methyl-1H-pyrazol-3-yl)-5-[3-(methylsulfonyl)phenoxy]benzamid;
3-[(1S)-2-Methoxy-(1-methylethyl)oxy]-N-(1-methyl-1H-pyrazol-3-yl)-5-[3-(methylsulfinyl)phenoxy]benzamid;
3-({4-[(1-Methylethyl)sulfonyl]phenyl}oxy)-5-[(1S)-2-methoxy-(1-methylethyl)oxy]-N-(1-methyl-1H-pyrazol-3-yl)benzamid;
3-[(1S)-2-Methoxy-(1-methylethyl)oxy]-N-(1-methyl-1H-pyrazol-3-yl)-5-[4-(methylsulfonyl)phenoxybenzamid;
3-[(1S)-2-Methoxy-(1-methylethyl)oxy]-5-[4-(methylsulfonyl)phenoxy]-N-(4-methyl-1,3-thiazol-2-yl)benzamid;
3-[(1S)-2-Methoxy-(1-methylethyl)oxy]-5-[4-(methylsulfonyl)phenoxy]-N-(5-methyl-1,3-thiazol-2-yl)benzamid;
3-[(1S)-2-Methoxy-(1-methylethyl)oxy]-5-[4-(methylsulfonyl)phenoxy]-N-(5-methyl-1,3,4-thiadiazol-2-yl)benzamid;
3-[(1S)-2-Methoxy-(1-methylethyl)oxy]-5-[4-(methylsulfonyl)phenoxy]-N-(3-methyl-1,2,4-thiadiazol-5-yl)benzamid;
N-(1-Ethyl-1H-pyrazol-3-yl)-3-[(1S)-2-methoxy-(1-methylethyl)oxy]-5-[4-(methylsulfonyl)phenoxy]benzamid;
3-(3,5-Difluorphenoxy)-5-[(1S)-2-methoxy-(1-methylethyl)oxy]-N-(1-methyl-1H-pyrazol-3-yl)benzamid;
N-(5-Brompyridin-2-yl)-3-(3,5-difluorphenoxy)-5-[(1S)-2-methoxy-(1-methylethyl)oxy]benzamid;
3-(3,5-Difluorphenoxy)-N-[4-(hydroxymethyl)-1,3-thiazol-2-yl]-5-[(1S)-2-methoxy-(1-methylethyl)oxy]benzamid;
3-[(1S)-2-Methoxy-(1-methylethyl)oxy]-5-[4-(methylsulfonyl)phenoxy]-N-1H-pyrazol-3-ylbenzamid;
3-[(1S)-2-Methoxy-(1-methylethyl)oxy]-N-(5-methyl-1H-pyrazol-3-yl)-5-[4-(methylsulfonyl)phenoxy]benzamid;
3-[(1S)-2-Methoxy-(1-methylethyl)oxy]-N-[4-(methoxymethyl)-1,3-thiazol-2-yl]-5-[4-(methylsulfonyl)phenoxy]benzamid;
3-[4-(Azetidin-1-ylcarbonyl)phenoxy]-5-[(1S)-2-methoxy-(1-methylethyl)oxy]-N-(3-methyl-1,2,4-thiadiazol-5-yl)benzamid;
3-[(1S)-2-Methoxy-(1-methylethyl)oxy]-5-(4-{[(1-methylpiperidin-4-yl)amino]carbonyl}phenoxy)-N-(3-methyl-1,2,4-thiadiazol-5-yl)benzamid;
3-[4-(Azetidin-1-ylcarbonyl)-2-chlorphenoxy]-5-[(1S)-2-methoxy-(1-methylethyl)oxy]-N-(1-methyl-1H-pyrazol-3-yl)benzamid;
3-[4-(Azetidin-1-ylcarbonyl)-2-fluorphenoxy]-5-[(1S)-2-methoxy-(1-methylethyl)oxy]-N-(1-methyl-1H-pyrazol-3-yl)benzamid;
3-[4-(Azetidin-1-ylcarbonyl)-2-(trifluormethyl)-phenoxy]-5-[(1S)-2-methoxy-(1-methylethyl)oxy]-N-(1-methyl-1H-pyrazol-3-yl)benzamid;
3-{4-[(Dimethylamino)carbonyl]phenoxy}-5-[(1S)-2-methoxy-(1-methylethyl)oxy]-N-(1-methyl-1H-pyrazol-3-yl)benzamid;
und deren Salze und Solvate handelt.

18. Verbindungen der Formel (I) bei denen es sich um:
3-[4-(Azetidin-1-ylcarbonyl)phenoxy]-5-[(1S)-2-methoxy-1-methylethoxyl-N-1H-pyrazol-3-ylbenzamid;
3-[4-(Azetidin-1-ylcarbonyl)-2-chlorphenoxy]-5-[(1S)-2-methoxy-1-methylethoxy]-N-(5-methyl-1H-pyrazol-3-yl)benzamid;
3-[4-(Azetidin-1-ylcarbonyl)-2-fluorphenoxy]-5-[(1S)-2-methoxy-1-methylethoxy]-N-(5-methyl-1H-pyrazol-3-yl)benzamid;
3-[4-(Azetidin-1-ylcarbonyl)-2-fluorphenoxy]-5-[(1S)-2-methoxy-1-methylethoxy]-N-1H-pyrazol-3-ylbenzamid;
3-[4-(Azetidin-1-ylcarbonyl)phenoxy]-5-[(1S)-2-methoxy-1-methylethoxy]-N-(5-methyl-1H-pyrazol-3-yl)benzamid; oder
3-[(1S)-2-Methoxy-1-methylethoxy]-N-(1-methyl-1H-pyrazol-3-yl)-5-[4-(1,2,4-oxadiazol-3-yl)phenoxy]benzamid;
und deren Salze und Solvate handelt.

19. Verbindung der Formel (I) nach Anspruch 1, bei der es sich um 3-[4-(Azetidin-1-ylcarbonyl)phenoxy]5-[(1S)-2-methoxy-(1-methylethyl)oxy]-N-(1-methyl-1H-pyrazol-3-yl)benzamid handelt; und deren Salze und Solvate.

20. Verbindung der Formel (I) nach Anspruch 1, bei der es sich um 3-[(S1)-2-Methoxy-(1-methylethyl)oxy]-N-(1-methyl-1H-pyrazol-3-yl)-5-[4-(methylsulfonyl)phenoxy]benzamid handelt; und deren Salze und Solvate.

21. Verbindung der Formel (I) nach Anspruch 1, bei der es sich um 3-[(S1)-2-Methoxy-(1-methylethyl)oxy]-5-[4(methylsulfonyl)phenoxy]-N-(3-methyl-1,2,4-thiadiazol-5-yl)benzamid handelt; und deren Salze und Solvate.

22. Kombination einer Verbindung nach einem der Ansprüche 1 bis 9 oder Anspruch 17 bis 21 mit einer oder mehreren anderen Substanzen und/oder Behandlung.

## Revendications

1. Composé de formule (I), ou un sel ou un solvate de celui-ci : 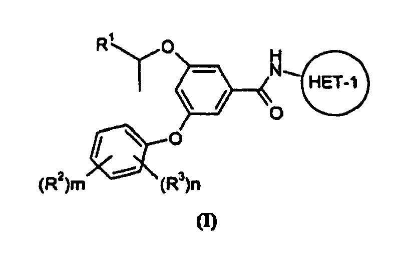 dans laquelle :
R¹ est méthoxyméthyle ;
R² est choisi parmi -C(O)NR⁴R⁵, -SO₂NR⁴R⁵, -S(O)pR⁴ et HET-2 ;
HET-1 est un cycle hétéroaryle à 5 ou 6 chaînons lié par C, contenant un atome d'azote en position 2 et éventuellement 1 ou 2 autres hétéroatomes du cycle choisis indépendamment parmi O, N et S ; lequel cycle est éventuellement substitué sur un atome de carbone disponible, ou sur un atome d'azote du cycle à condition qu'il ne soit pas ainsi quaternisé, par 1 ou 2 substituants choisis indépendamment parmi R⁶ ;
HET-2 est un cycle hétérocyclyle à 4, 5 ou 6 chaînons lié par C ou N, contenant 1, 2, 3 ou 4 hétéroatomes choisis indépendamment parmi 0, N et S, où un groupement -CH₂- peut éventuellement être remplacé par un -C(O)-, et où un atome de soufre dans le cycle hétérocyclique peut éventuellement être oxydé en un groupement S(O) ou S(O)₂, lequel cycle est éventuellement substitué sur un atome de carbone ou d'azote disponible par 1 ou 2 substituants choisis indépendamment parmi R⁷ ;
R³ est choisi parmi halogéno, fluorométhyle, difluorométhyle, trifluorométhyle, méthyle, méthoxy et cyano ;
R⁴ est choisi parmi hydrogène, (1-4C)alkyle [éventuellement substitué par 1 ou 2 substituants choisis indépendamment parmi HET-2, -OR⁵, -SO₂R⁵, (3-6C) cycloalkyle (éventuellement substitué par 1 groupement choisi parmi R⁷) et -C(O)NR⁵R⁵] , (3-6C)cycloalkyle (éventuellement substitué par 1 groupement choisi parmi R⁷) et HET-2 ;
R⁵ est hydrogène ou (1-4C)alkyle ;
ou R⁴ et R⁵, conjointement avec l'atome d'azote auquel ils sont liés, peuvent former un système de cycle hétérocyclyle tel que défini par HET-3 ;
R⁶ est choisi indépendamment parmi (1-4C)alkyle, halogéno, hydroxy(1-4C)alkyle, (1-4C)alcoxy(1-4C)alkyle, (1-4C)alkylS(O)p(1-4C)alkyle, amino(1-4C)alkyle, (1-4C)alkylamino(1-4C)alkyle, di(1-4C)alkylamino(1-4C)alkyle et HET-4 ;
R⁷ est choisi parmi -OR⁵, (1-4C)alkyle, -C(O) (1-4C)alkyle, -C(O)NR⁴R⁵, (1-4C)alcoxy(1-4C)alkyle, hydroxy(1-4C)alkyle et -S(O)pR⁵ ;
HET-3 est un cycle hétérocyclyle saturé ou partiellement insaturé à 4, 5 ou 6 chaînons lié par N, contenant éventuellement 1 ou 2 autres hétéroatomes (outre l'atome N de liaison) choisis indépendamment parmi 0, N et S, où un groupement -CH₂- peut éventuellement être remplacé par un -C(O)- et où un atome de soufre dans le cycle peut éventuellement être oxydé en un groupement S(O) ou S(O)₂ ; lequel cycle est éventuellement substitué sur un atome de carbone ou d'azote disponible par 1 ou 2 substituants choisis indépendamment parmi R⁸ ; ou
HET-3 est un cycle hétérocyclyle saturé ou partiellement insaturé à 7 chaînons lié par N, contenant éventuellement 1 autre hétéroatome (outre l'atome N de liaison) choisi indépendamment parmi O, S et N, où un groupement -CH₂- peut éventuellement être remplacé par un groupement -C(O)- et où un atome de soufre dans le cycle peut éventuellement être oxydé en un groupement S(O) ou S(O)₂ ; lequel cycle est éventuellement substitué sur un atome de carbone ou d'azote disponible par 1 ou 2 substituants choisis indépendamment parmi R⁸ ; ou
HET-3 est un cycle hétérocyclyle bicyclique saturé ou partiellement insaturé à 6-10 chaînons, contenant éventuellement 1 autre atome d'azote (outre l'atome N de liaison), où un groupement -CH₂- peut éventuellement être remplacé par un -C(O)- ; lequel cycle est éventuellement substitué sur un atome de carbone ou d'azote disponible par 1 substituant choisi parmi hydroxy et R³ ;
R⁸ est choisi parmi -OR⁵, (1-4C)alkyle, -C(O) (1-4C)alkyle, -C(O)NR⁴R⁵, (1-4C) alkylamino, di(1-4C)alkylamino, HET-3 (où ledit cycle est non substitué), (1-4C)alcoxy(1-4C)alkyle, hydroxy(1-4C)alkyle et -S(O)pR⁵ ;
HET-4 est un cycle hétéroaryle à 5 ou 6 chaînons non substitué lié par C ou N, contenant 1, 2 ou 3 hétéroatomes du cycle choisis indépendemment parmi O, N et S ;
p est (indépendamment à chaque apparition) 0, 1 ou 2 ;
m est 0 ou 1 ;
n est 0, 1 ou 2 ;
à condition que lorsque m est 0, alors n soit 1 ou 2 ;
et que le composé de formule (I) soit autre que :
le 3-(4-méthanesulfonylphénoxy)-5-(2-méthoxy-1-méthyl-éthoxy)-N-thiazol-2-yl-benzamide ;
le N-(4-hydroxyméthyl-thiazol-2-yl)-3-(4-méthanesulfonylphénoxy)-5-(2-méthoxy-1-méthyléthoxy)-benzamide ;
le N-[4-(1-hydroxy-éthyl)-thiazol-2-yl]-3-(4-méthanesulfonylphénoxy)-5-(2-méthoxy-1-méthyléthoxy)-benzamide ;
le N-[3-hydroxyméthyl-1,2,4-thiadiazol-5-yl]-3-(4-méthanesulfonylphénoxy)-5-(2-méthoxy-1-méthyléthoxy)-benzamide ; ou
le 3-(4-méthanesulfonylphénoxy)-5-(2-méthoxy-1-méthyl-éthoxy)-(pyrazol-3-yl)benzamide.

2. Composé de formule (I) selon la revendication 1, ou un sel ou un solvate de celui-ci, **caractérisé en ce que** R¹ a la configuration (S).

3. Composé de formule (I) selon la revendication 1, ou un sel ou un solvate de celui-ci, **caractérisé en ce que** m vaut 1, n vaut 0 et R² est en position para par rapport à la liaison éther.

4. Composé de formule (I) selon la revendication 1, la revendication 2 ou la revendication 3, ou un sel ou un solvate de celui-ci, **caractérisé en ce que** HET-1 est un cycle à 5 chaînons.

5. Composé de formule (I) selon l'une quelconque des revendications 1 à 4, ou un sel ou un solvate de celui-ci, **caractérisé en ce que** R² est choisi parmi -C(O)NR⁴R⁵ et -SO₂NR⁴R⁵ et R⁴ et R⁵, conjointement avec l'atome d'azote auquel ils sont liés, peuvent former un système de cycle hétérocyclyle tel que défini par HET-3.

6. Composé de formule (I) selon l'une quelconque des revendications 1 à 5, ou un sel ou un solvate de celui-ci, **caractérisé en ce que** HET-3 est un cycle à 4 à 6 chaînons.

7. Composé de formule (I) selon la revendication 1, la revendication 2 ou la revendication 3, ou un sel ou un solvate de celui-ci, **caractérisé en ce que** R² est choisi parmi -C(O)NR⁴R⁵ et -SO₂NR⁴R⁵ et R⁴ est choisi parmi (1-4C)alkyle [substitué par 1 ou 2 substituants choisis indépendamment parmi HET-2, -OR⁵, -SO₂R⁵, (3-6C)cycloalkyle (éventuellement substitué par 1 groupement choisi parmi R⁷) et -C(O)NR⁵R⁵], (3-6C) cycloalkyle (éventuellement substitué par 1 groupement choisi parmi R⁷) et HET-2.

8. Composé de formule (I) selon la revendication 1, la revendication 2 ou la revendication 3, ou un sel ou un solvate de celui-ci, **caractérisé en ce que** R² est -SO₂R⁴ et R⁴ est choisi parmi (1-4C)alkyle [substitué par 1 ou 2 substituants choisis indépendamment parmi HET-2, -OR⁵, -SO₂R⁵, (3-6C)cycloalkyle (éventuellement substitué par 1 groupement choisi parmi R⁷) et -C(O)NR⁵R⁵], (3 - 6C)cycloalkyle (éventuellement substitué par 1 groupement choisi parmi R⁷) et HET-2.

9. Composé de formule (I) selon la revendication 1, la revendication 2 ou la revendication 3, ou un sel ou un solvate de celui-ci, **caractérisé en ce que** R² est HET-2.

10. Composition pharmaceutique comprenant un composé selon l'une quelconque des revendications 1 à 9, ou un sel ou un solvate de celui-ci, conjointement avec un diluant ou un support pharmaceutiquement acceptable.

11. Composé selon l'une quelconque des revendications 1 à 9, ou un sel ou un solvate pharmaceutiquement acceptable de celui-ci, destiné à être utilisé comme médicament.

12. Composé selon l'une quelconque des revendications 1 à 9, destiné à être utilisé dans la préparation d'un médicament destiné au traitement d'une maladie médiée par la GLK.

13. Composé selon l'une quelconque des revendications 1 à 9, destiné à être utilisé dans la préparation d'un médicament destiné au traitement du diabète de type 2.

14. Utilisation d'un composé de formule (I) selon l'une quelconque des revendications 1 à 9, ou d'un sel ou d'un solvate de celui-ci, pour la fabrication d'un médicament destiné au traitement d'une maladie des mammifères médiée par la GLK.

15. Utilisation selon la revendication 14, **caractérisée en ce que** la maladie médiée par la GLK est le diabète de type 2.

16. Procédé de préparation d'un composé de formule (I) selon l'une quelconque des revendications 1 à 9, ou un sel ou un solvate de celui-ci, **caractérisé en ce qu'**il comprend (les variables étant telles que définies dans la revendication 1 sauf indication contraire) :
(a) la réaction d'un acide de formule (III) ou d'un dérivé activé de celui-ci avec un composé de formule (IV), 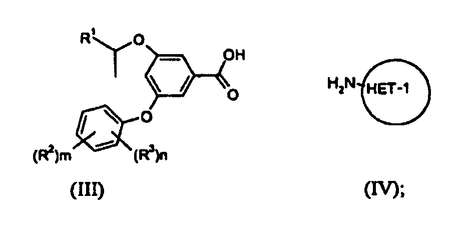 ou
(b) la réaction d'un composé de formule (V) avec un composé de formule (VI), 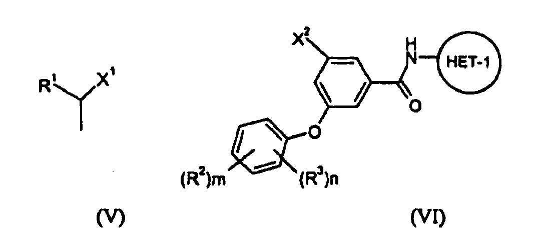 dans lesquelles X¹ est un groupement partant et X² est un groupement hydroxy ou X¹ est un groupement hydroxy et X² est un groupement partant ;
[ou par la réaction avec l'ester intermédiaire de formule (VII), où P¹ est un groupement protecteur, puis par l'hydrolyse de l'ester et la formation d'un amide] ; 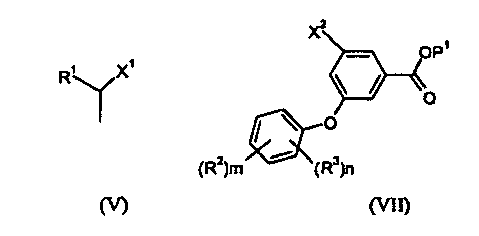 ou
(c) la réaction d'un composé de formule (VIII) avec un composé de formule (IX) 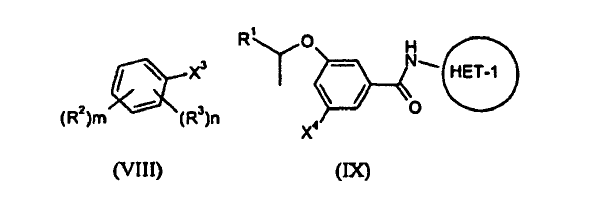 dans lesquelles X³ est un groupement partant ou un réactif organométallique et X⁴ est un groupement hydroxy ou X³ est un groupement hydroxy et X⁴ est un groupement partant ou un réactif organométallique ;
[ou par la réaction de (VIII) avec l'ester intermédiaire de formule (X), puis par l'hydrolyse de l'ester et la formation d'un amide] ; 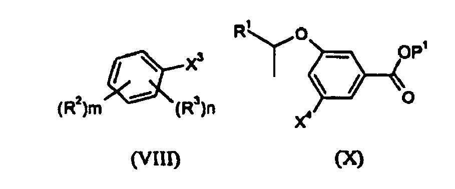 ou
(d) la réaction d'un composé de formule (XI) avec un composé de formule (XII), 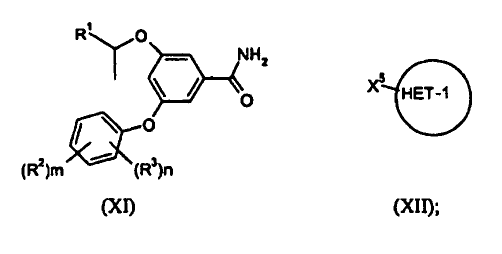
dans laquelle X⁵ est un groupement partant ; et ensuite, le cas échéant :
i) la transformation d'un composé de formule (I) en un autre composé de formule (I) ;
ii) l'élimination de tous groupements protecteurs ; et/ou
iii) la formation d'un sel ou d'un solvate.

17. Composé de formule (I), **caractérisé en ce qu'**il s'agit de :
3-(4-{[(2-méthoxyéthyl)amino]carbonyl}phénoxy)-5-[(1S)-2-méthoxy-(1-méthyléthyl)oxy]-N-1,3-thiazol-2-ylbenzamide ;
3-(4-{[(1H-imidazol-2-ylméthyl)amino]carbonyl}phénoxy)-5-[(1S)-2-méthoxy-(1-méthyléthyl)oxy]-N-1,3-thiazol-2-ylbenzamide ;
3-[4-(azétidin-1-ylcarbonyl)phénoxy]-5-[(1S)-2-méthoxy-(1-méthyléthyl)oxy]-N-1,3-thiazol-2-ylbenzamide ;
3-[(1S)-2-méthoxy-(1-méthyléthyl)oxy]-5-{4-[(4-méthylpipérazin-1-yl)carbonyl]phénoxy}-N-1,3-thiazol-2-ylbenzamide ;
3-(3-{[(2-méthoxyéthyl)amino]carbonyl}phénoxy)-5-[(1S)-2-méthoxy-(1-méthyléthyl)oxy]-N-1,3-thiazol-2-ylbenzamide ;
3-(3-{[(1H-imidazol-2-ylméthyl)amino]carbonyl}phénoxy)-5-[(1S)-2-méthoxy-(1-méthyléthyl)oxy]-N-1,3-thiazol-2-ylbenzamide ;
3-[(1S)-2-méthoxy-(1-méthyléthyl)oxy]-5-{4-[(4-méthylpipérazin-1-yl)carbonyl]phénoxy}-N-(1-méthyl-1H-pyrazol-3-yl)benzamide ;
3-[(1S)-2-méthoxy-(1-méthyléthyl)oxy]-N-(1-méthyl-1H-pyrazol-3-yl)-5-[4-(morpholin-4-ylcarbonyl)phénoxy]benzamide ;
3-[4-(azétidin-1-ylcarbonyl)phénoxy]-5-[(1S)-2-méthoxy-(1-méthyléthyl)oxy]-N-(1-méthyl-1H-pyrazol-3-yl)benzamide ;
3-[(1S)-2-méthoxy-(1-méthyléthyl)oxy]-N-(1-méthyl-1H-pyrazol-3-yl)-5-[4-(pyrrolidin-1-ylcarbonyl)phénoxy]benzamide ;
3-[4-(7-azabicyclo[2,2,1]hept-7-ylcarbonyl)phénoxy]-5-[(1S)-2-méthoxy-(1-méthyléthyl)oxy]-N-(1-méthyl-1H-pyrazol-3-yl)benzamide ;
3-{2-chloro-4-[(diméthylamino)sulfonyl]phénoxy}-5-[(1S)-2-méthoxy-(1-méthyléthyl)oxy]-N-(1-méthyl-1H-pyrazol-3-yl)benzamide ;
3-[(2-chloro-4-{[(1-méthyléthyl)amino]sulfonyl}phényl)oxy]-5-[(1S)-2-méthoxy-(1-méthyléthyl)oxy]-N-(1-méthyl-1H-pyrazol-3-yl)benzamide ;
3-{[2-chloro-4-({[2-(méthyloxy)éthyl]amino}sulfonyl)phényl]oxy}-5-[(1S)-2-méthoxy-(1-méthyléthyl)oxy]-N-(1-méthyl-1H-pyrazol-3-yl)benzamide ;
3-({2-chloro-4-[(4-méthylpipérazin-1-yl)sulfonyl]phényl}oxy)-5-[(1S)-2-méthoxy-(1-méthyléthyl)oxy]-N-(1-méthyl-1H-pyrazol-3-yl)benzamide ;
3-{4-[(diméthylamino)sulfonyl]phénoxy}-5-[(1S)-2-méthoxy-(1-méthyléthyl)oxy]-N-(1-méthyl-1H-pyrazol-3-yl)benzamide ;
3-[(1S)-2-méthoxy-(1-méthyléthyl)oxy]-5-({4-[(4-méthylpipérazin-1-yl)sulfonyl]phényl}oxy)-N-(1-méthyl-1H-pyrazol-3-yl)benzamide ;
3-{4-[((1-méthyléthyl)amino)sulfonyl]phénoxy}-5-[(1S)-2-méthoxy-(1-méthyléthyl)oxy]-N-(1-méthyl-1H-pyrazol-3-yl)benzamide ;
3-(4-{[(2-méthoxyéthyl)amino]sulfonyl}phénoxy)-5-[(1S)-2-méthoxy-(1-méthyléthyl)oxy]-N-(1-méthyl-1H-pyrazol-3-yl)benzamide ;
3-(4-cyanophénoxy)-5-[(1S)-2-méthoxy-(1-méthyléthyl)oxy]-N-(1-méthyl-1H-pyrazol-3-yl)benzamide ;
3-{[4-(aminocarbonyl)phényl]oxy}-5-[(1S)-2-méthoxy-(1-méthyléthyl)oxy]-N-(1-méthyl-1H-pyrazol-3-yl)benzamide ;
3-[4-(éthylsulfonyl)phénoxy]-5-[(1S)-2-méthoxy-(1-méthyléthyl)oxy]-N-1,3-thiazol-2-ylbenzamide ;
3-[(1S)-2-méthoxy-(1-méthyléthyl)oxy]-N-(1-méthyl-1H-pyrazol-3-yl)-5-{[3-(méthylthio)phényl]oxy}benzamide ;
3-({4-[(1-méthyléthyl)thio]phényl}oxy)-5-[(1S)-2-méthoxy-(1-méthyléthyl)oxy]-N-(1-méthyl-1H-pyrazol-3-yl)benzamide ;
3-[(1S)-2-méthoxy-(1-méthyléthyl)oxy]-N-(1-méthyl-1H-pyrazol-3-yl)-5-[3-(méthylsulfonyl)phénoxy]benzamide ;
3-[(1S)-2-méthoxy-(1-méthyléthyl)oxy]-N-(1-méthyl-1H-pyrazol-3-yl)-5-[3-(méthylsulfinyl)phénoxy]benzamide ;
3-({4-[(1-méthyléthyl)sulfonyl]phényl}oxy)-5-[(1S)-2-méthoxy-(1-méthyléthyl)oxy]-N-(1-méthyl-1H-pyrazol-3-yl)benzamide ;
3-[(1S)-2-méthoxy-(1-méthyléthyl)oxy]-N-(1-méthyl-1H-pyrazol-3-yl)-5-[4-(méthylsulfonyl)phénoxy]benzamide ;
3-[(1S)-2-méthoxy-(1-méthyléthyl)oxy]-5-[4-(méthylsulfonyl)phénoxy]-N-(4-méthyl-1,3-thiazol-2-yl)benzamide ;
3-[(1S)-2-méthoxy-(1-méthyléthyl)oxy]-5-[4-(méthylsulfonyl)phénoxy]-N-(5-méthyl-1,3-thiazol-2-yl)benzamide ;
3-[(1S)-2-méthoxy-(1-méthyléthyl)oxy]-5-[4-(méthylsulfonyl)phénoxy]-N-(5-méthyl-1,3,4-thiadiazol-2-yl)benzamide ;
3-[(1S)-2-méthoxy-(1-méthyléthyl)oxy]-5-[4-(méthylsulfonyl)phénoxy]-N-(3-méthyl-1,2,4-thiadiazol-5-yl)benzamide ;
N-(1-éthyl-1H-pyrazol-3-yl)-3-[(1S)-2-méthoxy-(1-méthyléthyl)oxy]-5-[4-(méthylsulfonyl)phénoxy]benzamide ;
3-(3,5-difluorophénoxy)-5-[(1S)-2-méthoxy-(1-méthyléthyl)oxy]-N-(1-méthyl-1H-pyrazol-3-yl)benzamide ;
N-(5-bromopyridin-2-yl)-3-(3,5-difluorophénoxy)-5-[(1S)-2-méthoxy-(1-méthyléthyl)oxy]benzamide ;
3-(3,5-difluorophénoxy)-N-[4-(hydroxyméthyl)-1,3-thiazol-2-yl]-5-[(1S)-2-méthoxy-(1-méthyléthyl)oxy]benzamide ;
3-[(1S)-2-méthoxy-(1-méthyléthyl)oxy]-5-[4-(méthylsulfonyl)phénoxy]-N-1H-pyrazol-3-ylbenzamide ;
3-[(1S)-2-méthoxy-(1-méthyléthyl)oxy]-N-(5-méthyl-1H-pyrazol-3-yl)-5-[4-(méthylsulfonyl)phénoxy]benzamide ;
3-[(1S)-2-méthoxy-(1-méthyléthyl)oxy]-N-[4-(méthoxyméthyl)-1,3-thiazol-2-yl]-5-[4-(méthylsulfonyl)phénoxy]benzamide ;
3-[4-(azétidin-1-ylcarbonyl)phénoxy]-5-[(1S)-2-méthoxy-(1-méthyléthyl)oxy]-N-(3-méthyl-1,2,4-thiadiazol-5-yl)benzamide ;
3-[(1S)-2-méthoxy-(1-méthyléthyl)oxy]-5-(4-{[(1-méthylpipéridin-4-yl)amino]carbonyl}phénoxy)-N-(3-méthyl-1,2,4-thiadiazol-5-yl)benzamide ;
3-[4-(azétidin-1-ylcarbonyl)-2-chlorophénoxy]-5-[(1S)-2-méthoxy-(1-méthyléthyl)oxy]-N-(1-méthyl-1H-pyrazol-3-yl)benzamide ;
3-[4-(azétidin-1-ylcarbonyl)-2-fluorophénoxy]-5-[(1S)-2-méthoxy-(1-méthyléthyl)oxy]-N-(1-méthyl-1H-pyrazol-3-yl)benzamide ;
3-[4-(azétidin-1-ylcarbonyl)-2-(trifluorométhyl)phénoxy]-5-[(1S)-2-méthoxy-(1-méthyléthyl)oxy]-N-(1-méthyl-1H-pyrazol-3-yl)benzamide ;
3-{4-[(diméthylamino)carbonyl]phénoxy}-5-[(1S)-2-méthoxy-(1-méthyléthyl)oxy]-N-(1-méthyl-1H-pyrazol-3-yl)benzamide ;
ou un sel ou un solvate de celui-ci.

18. Composé de formule (I), **caractérisé en ce qu'**il s'agit de :
3-[4-(azétidin-1-ylcarbonyl)phénoxy]-5-[(1S)-2-méthoxy-1-méthyléthoxy]-N-1H-pyrazol-3-ylbenzamide ;
3-[4-(azétidin-1-ylcarbonyl)-2-chlorophénoxy]-5-[(1S)-2-méthoxy-1-méthyléthoxy]-N-(5-méthyl-1H-pyrazol-3-yl)benzamide ;
3-[4-(azétidin-1-ylcarbonyl)-2-fluorophénoxy]-5-[(1S)-2-méthoxy-1-méthyléthoxy]-N-(5-méthyl-1H-pyrazol-3-yl)benzamide ;
3-[4-(azétidin-1-ylcarbonyl)-2-fluorophénoxy]-5-[(1S)-2-méthoxy-1-méthyléthoxy]-N-1H-pyrazol-3-ylbenzamide ;
3-[4-(azétidin-1-ylcarbonyl)phénoxy]-5-[(1S)-2-méthoxy-1-méthyléthoxy]-N-(5-méthyl-1H-pyrazol-3-yl)benzamide ; ou
3-[(1S)-2-méthoxy-1-méthyléthoxy]-N-(1-méthyl-1H-pyrazol-3-yl)-5-[4-(1,2,4-oxadiazol-3-yl)phénoxy]benzamide ;
ou un sel ou un solvate de celui-ci.

19. Composé de formule (I) selon la revendication 1, ce composé étant le 3-[4-(azétidin-1-ylcarbonyl)phénoxy]-5-[(1S)-2-méthoxy-(1-méthyléthyl)oxy]-N-(1-méthyl-1H-pyrazol-3-yl)benzamide; ou un sel ou un solvate de celui-ci.

20. Composé de formule (I) selon la revendication 1, ce composé étant le 3-[(1S)-2-méthoxy-(1-méthyléthyl)oxy]-N-(1-méthyl-1H-pyrazol-3-yl-5-[4-(méthylsulfonyl)phénoxy]benzamide; ou un sel ou un solvate de celui-ci.

21. Composé de formule (I) selon la revendication 1, ce composé étant le 3-[(1S)-2-méthoxy-(1-méthyléthyl)oxy]-5-[4-(mehtylsulfonyl)phénoxy-N-(3-méthyl-1,2,4-thiadiazol-5-yl)benzamide; ou un sel ou un solvate de celui-ci.

22. Combinaison d'un composé selon l'une quelconque des revendications 1 à 9 ou des revendications 17 à 21 et d'un ou plusieurs autres traitements et/ou substances.
